(19) Europäisches Patentamt / European Patent Office / Office européen des brevets

(11) **EP 3 844 782 B1**

(12) **EUROPEAN PATENT SPECIFICATION**

(45) Date of publication and mention of the grant of the patent:
**13.07.2022 Bulletin 2022/28**

(21) Application number: **19765427.0**

(22) Date of filing: **02.09.2019**

(51) International Patent Classification (IPC):
**G16H 50/70** (2018.01)  **G16H 20/17** (2018.01)
**G16H 50/20** (2018.01)  **G16H 10/60** (2018.01)

(52) Cooperative Patent Classification (CPC):
**G16H 20/17; G16H 50/20; G16H 50/70;**
G16H 10/60

(86) International application number:
**PCT/EP2019/073338**

(87) International publication number:
**WO 2020/043922 (05.03.2020 Gazette 2020/10)**

(54) **RETROSPECTIVE HORIZON BASED INSULIN DOSE PREDICTION**

REAKTIONSHORIZONTBASIERTE INSULINDOSISVORHERSAGE

PRÉDICTION DE LA DOSE D'INSULINE BASÉE SUR UN HORIZON DE RÉPONSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **31.08.2018  US 201862725413 P**
**20.09.2018  EP 18195592**

(43) Date of publication of application:
**07.07.2021 Bulletin 2021/27**

(73) Proprietor: **Novo Nordisk A/S**
**2880 Bagsværd (DK)**

(72) Inventor: **IMANBAYEV, Anuar**
**Seattle, Washington 98109 (US)**

(56) References cited:
WO-A2-2011/051922      US-A1- 2010 262 434
US-A1- 2017 053 101

• Cooper Midroni ET AL: "Predicting Glycemia in Type 1 Diabetes Patients: Experiments with XGBoost", International Conference on Brain Inspired Cognitive Systems, 13 November 2016 (2016-11-13), pages 274-283, XP055568039, DOI: 10.2337/dc11-0471 Retrieved from the Internet: URL:http://ceur-ws.org/Vol-2148/paper13.pd f [retrieved on 2019-03-12]
• CLAUDIO COBELLI ET AL: "Artificial Pancreas: Past, Present, Future", DIABETES, vol. 60, no. 11, 24 October 2011 (2011-10-24), pages 2672-2682, XP055570616, US ISSN: 0012-1797, DOI: 10.2337/db11-0654
• COBELLI C ET AL: "Diabetes: Models, Signals, and Control", IEEE REVIEWS IN BIOMEDICAL ENGINEERING, IEEE, USA, vol. 2, 1 January 2009 (2009-01-01), pages 54-96, XP011285638, ISSN: 1937-3333

**Description**

The present disclosure relates generally to systems and methods for assisting patients and health care practitioners in managing insulin treatment to diabetics, in which the quality of insulin dose amount data is evaluated based on datasets of blood glucose measurements.

## BACKGROUND OF THE INVENTION

Diabetes mellitus (DM) is impaired insulin secretion and variable degrees of peripheral insulin resistance leading to hyperglycemia. Type 2 diabetes mellitus is characterized by progressive disruption of normal physiologic insulin secretion. In healthy individuals, basal insulin secretion by pancreatic β cells occurs continuously to maintain steady glucose levels for extended periods between meals. Also in healthy individuals, there is prandial secretion in which insulin is rapidly released in an initial first-phase spike in response to a meal, followed by prolonged insulin secretion that returns to basal levels after 2-3 hours. Years of poorly controlled hyperglycemia can lead to multiple health complications. Diabetes mellitus is one of the major causes of premature morbidity and mortality throughout the world.

Effective control of blood/plasma glucose can prevent or delay many of these complications but may not reverse them once established. Hence, achieving good glycemic control in efforts to prevent diabetes complications is the primary goal in the treatment of type 1 and type 2 diabetes. In particular, frequent changes in insulin dosage titration are key to helping stabilize blood glucose levels in patients (Bergenstal et al., "Can a Tool that Automates Insulin Titration be a Key to Diabetes Management?" Diabetes Tech. and Thera. 2012; 14(8) 675-682). Smart titrators with adjustable step size and physiological parameter estimation and pre-defined fasting blood glucose target values have been developed to administer insulin medicament treatment regimens. Optimal initiation and titration methods for the long-acting basal insulins are still being determined. However, evidence suggests that many patients often do not receive insulin doses titrated sufficiently to achieve target levels of glucose control (remaining on suboptimal doses and failing to reach treatment targets) (Holman et al., "10-year follow-up of intensive glucose control in type 2 diabetes," N. Engl. J. Med. 2008; 359: 1577-1589).

One of the major problems with insulin regimens is the lack of patient autonomy and empowerment. Patients often must visit clinics to have new titrations calculated. When a clinic has to titrate the insulin dosages for the patient, there is a natural limitation on the possible frequency of changing the titration dose. Self-titration regimens facilitate empowerment of patients, allowing them to become more involved in their treatment, which can result in improved glycemic control (Khunti et al., "Self-titration of insulin in the management of people with type 2 diabetes: a practical solution to improve management in primary care," Diabetes, Obes., and Metabol. 2012; 15(8) 690-700). Patients who take an active role in the management of their diabetes and titration of their insulin may feel more empowered to take charge of their self-care and have a stronger belief that their actions can influence their disease, thus leading to better treatment outcomes (Norris et al., "Self-management education for adults with type 2 diabetes: a meta-analysis on the effect of glycemic control." Diabetes Care. 2002; 25:1159-71; Kulzer et al., "Effects of self-management training in type 2 diabetes: a randomized, prospective trial," Diabet. Med. 2007; 24:415-23; Anderson et al., "Patient empowerment: results of a randomized controlled trial." Diabetes Care. 1995; 18:943-9). Further, when patients have control of their own titration, the frequency of titrations increases, which increases the likelihood that patients will achieve desired blood glucose levels.

However, autonomous patient titrations depend on trustworthy data. If a patient is to autonomously titrate a dosage, it is essential for the patient to be confident that the previously recommended titration level was adhered to. Insulin doses may be captured or measured incorrectly (e.g. Friedrichs et al., "Dosing Accuracy and Injection Force of Different Insulin Glargine Pens." J Diabetes Sci Technol. 2013; 7(5): 1346-1353; Abdel-Twab et al., "Dosing Accuracy of Two Disposable Insulin Pens According to New Iso 11680-1: 2012 Requirements." J Diabetes Sci Technol. 2015; 10(1): 157-161). For autonomous titration, insulin dose data is transmitted via Bluetooth or other connectivity solution, stored in a database, and then analysed. Thus, errors may be propagated throughout an entire algorithmic chain to produce a wrong and/or even a harmful prediction or result. This is an issue that plagues not only engineers, researchers, data scientists, marketers, and employees but also HCPs and type II diabetes mellitus (T2DM) patients for whom these new connected digital health apps, products, and services are being designed and crafted for.

The main data quality challenge is successful capture of the bodily injected insulin dose from medical devices into medical software. There are a number of promising venues to resolve this data quality challenge, both hardware improvements (e.g. the addition of sensors such as sound, speed, temperature, pressure, or optics) and software methods. Software solutions either attempt to directly filter out the actual bodily injected insulin dose or they attempt to indirectly filter out all other possible insulin dosing events or dispenses, such as air shots, wet shots, priming events, partial doses, and so on (Daskalaki et al., "Real-Time Adaptive Models for the personalized prediction of Glycemic Profile in Type 1 Diabetes Patients." Diabetes Technol. Thera. 2012; 14(2): 168-174; Pappada et al. "Neural Network-Based Real-Time Prediction of Glucose in Patients with Insulin-Dependence Diabetes." Diabetes Technol. Thera. 2011; 13(2):

135-141). Software solutions that range in complexity, from simple to advanced heuristics filtering rules all the way to complex and preferably automated machine learning and artificial intelligence systems. However, these current software solutions have failed to consistently achieve high accuracy scores.

US 2017/053101 discloses a method for determining a treatment dose for a treated patient, the method comprising training a model using blood glucose history data and treatment dose data of insulin administered by patients of a patient population, as well as outcome attributes associated with each treatment dose. Based on this a next recommended treatment dose of insulin for the treated patient based on one or more of the identified optimum treatment doses can be made.

Given the above background, what is needed in the art are devices, systems and methods for providing accurate quality control information for insulin dose amount data, thus enabling more accurate titration regimens, and thereby treating type 2 diabetes.

## DISCLOSURE OF THE INVENTION

In the disclosure of the present invention, embodiments and aspects will be described which will address one or more of the above objects or which will address objects apparent from the below disclosure as well as from the description of exemplary embodiments.

The present disclosure addresses the need in the art for devices, systems and methods for providing data quality control and reconstruction of captured insulin dose amounts.

According to a first aspect of the invention a computing system for providing medication dose guidance recommendations for a query subject to treat diabetes mellitus is provided, wherein the system comprises one or more processors and a memory. The memory comprises instructions that, when executed by the one or more processors, perform a method responsive to receiving a dose guidance request. The instructions comprising the steps of (a) obtaining a first data structure comprising query subject parameters, (b) obtaining a first data set, comprising a plurality of glucose measurements of the query subject taken over a time course and thereby establish a blood glucose history, each respective glucose measurement in the plurality of glucose measurements comprising (i) a blood glucose (BG) level, and (ii) a corresponding blood glucose timestamp representing when in the time course the respective glucose measurement was made, (c) obtaining a second data set, comprising an insulin injection history of the query subject, wherein the injection history comprises a plurality of injections during all or a portion of the time course and, for each respective injection in the plurality of injections comprising (i) a corresponding injection amount, and (ii) an injection timestamp representing when in the time course the respective injection occurred, (d) obtaining a trained model for retrospective insulin dose prediction based on blood glucose response data sets having a given retrospective horizon (RH), (e) performing data optimization for the dataset of the query subject, the data optimization including inputting query subject data to the trained model and obtaining at least one reconstructed insulin dose amount for the query subject, and (f) providing a medication dose guidance recommendation, the recommendation being calculated based on data from the first data structure, the first data set, the second data set, and at least one reconstructed insulin dose amount for the query subject.

In this way historic dose data can be reconstructed based on patient blood glucose values, this allowing more accurate dose recommendations to be calculated.

The step of performing data optimization may comprise (i) obtaining from the dataset of the query subject a plurality of query subject blood glucose measurements in a respective time course and at least one query subject insulin dose amount in a respective subset of time points, and (ii) inputting the plurality of query subject blood glucose measurements into the trained model, thereby obtaining one or more query model output values for the query subject, wherein the one or more query output values for the query subject comprise at least one or more reconstructed insulin dose amounts for the query subject.

The method may further comprise performing data preprocessing and data processing for the dataset of the query subject, wherein (i) the data preprocessing includes accessing the dataset of the query subject, selecting a predetermined type of insulin dose, selecting a predetermined type of blood glucose measurement, and reorganizing the plurality of query subject blood glucose measurements and the at least one subject insulin dose amounts into a plurality of preprocessed query subject blood glucose measurements and/or an at least one preprocessed query subject insulin dose amount, (ii) the data processing includes combining the preprocessed query subject blood glucose histories and the preprocessed query subject insulin dose histories into a query subject data table, and designating independent variables and dependent variables in the query subject data table, and (iii) the data optimization includes applying the query subject data table to the trained model and obtaining at least one reconstructed insulin dose amount for the query subject.

In an exemplary embodiment a computing system as disclosed above is provided, wherein the step of obtaining a trained model comprises (a) obtaining a training set that comprises a plurality of reference entities, wherein the plurality of reference entities comprises a plurality of reference insulin dose histories and a plurality of reference blood glucose histories, wherein each reference insulin dose history is paired with a reference blood glucose history to form a reference entity in the plurality of reference entities, (b) for each respective reference entity in the plurality of reference entities, obtaining (i) a blood glucose measurement for each time point in a respective time course from the corresponding blood glucose history and (ii) an insulin dose amount for each time point in a respective subset of time points in the respective time course from the corresponding reference insulin dose history, wherein each respective reference entity in the plurality of reference entities provides a reference plurality of blood glucose measurements for the respective time course, wherein the reference plurality of blood glucose measurements is of a predetermined type of blood glucose measurements, and each respective reference entity in the plurality of reference entities provides one or more reference insulin dose amounts in the respective subset of time points, wherein the one or more reference insulin dose amounts are of a predetermined type of insulin dose, (c) obtaining a test set that comprises a plurality of test entities, wherein the plurality of test entities comprises a plurality of test insulin dose histories and a plurality of test blood glucose histories, wherein each test insulin dose history is paired with a test blood glucose history to form a test entity in the plurality of test entities, wherein each respective test blood glucose history provides a plurality of blood glucose measurements, and wherein each respective test insulin dose history provides one or more test insulin dose amounts, wherein the one or more test insulin dose amounts are of a predetermined type of insulin dose, (d) training a model using the reference plurality of blood glucose measurements for the respective time course and the at least one reference insulin dose amount across the training set, thereby obtaining a trained model, (e) inputting the test plurality of blood glucose histories into the trained model thereby obtaining one or more trained model output values for the test set, wherein the one or more trained output values comprises one or more reconstructed insulin dose amounts for the test set, and (f) evaluating the trained model based on the trained model output values for the test set. The training and test set data may be resampled for injecting into a machine learning engine.

In exemplary embodiments the plurality of reference insulin dose histories and the plurality of reference blood glucose histories are from a plurality of training subjects, or the plurality of reference insulin dose histories and the plurality of reference blood glucose histories are from a single training subject.

In further exemplary embodiments each time point in the subset of time points immediately precedes a predefined time period in the time course, wherein the predefined period in the time course is selected from the set of 20-40 minutes, 30-60 minutes, 40-60 minutes, 50-70 minutes, 60-80 minutes, 80-100 minutes, 100-120 minutes and 120-180 minutes.

The respective reference insulin dose history may comprise a plurality of insulin doses administered to the corresponding reference entity during all or a portion of the time course and, for each respective insulin dose in the plurality of insulin doses, a corresponding insulin dose amount and an insulin dose timestamp representing when in the time course the respective insulin dose occurred, and wherein the insulin dose timestamp is in the subset of time points.

The method may further comprise the step of providing one or more model performance metrics, wherein the one or more model performance metrics includes a root mean square error or an accuracy score.

In an exemplary embodiment the data optimization further includes handling missing data, wherein one or more temporal gaps in the subject data table are interpolated by resampling the subject data table by a predefined time interval.

The predetermined type of blood glucose measurements may be (i) continuous glucose monitoring data, or (ii) self-monitoring of blood glucose data. The predetermined type of insulin dose may consist of (i) one or more bolus insulin dose events, or (ii) one or more basal insulin dose events.

The computing system may be adapted for providing a long-acting or ultra-long-acting insulin adjustment day dose recommendation for the query subject, wherein the data structure comprises (i) a glucose upper target range level of the subject, (ii) a glucose lower target range level of the subject, and (iii) a current dose guidance baseline.

The computing system may be adapted for providing a bolus dose recommendation for the query subject, wherein the data structure comprises meal information.

In a **further aspect** of the invention a computing system is provided that serves the technical purpose of the determination of a dose recommendation or dose titration with improved and maximally optimized insulin dosage data quality defined in the context of unacceptable or unusable insulin dosage values which may be inaccurately logged and delivered, missing (e.g. by titration regimen, a user is supposed to take same dose for all three days, but only one day's dosage is logged due user error or sometimes clerical error not recognizing user input correctly), or corrupt (e.g. insulin doses such as 4000, -200, or timestamp swapped, 15th minute occurring before the 10th minute instead of the 5th minute), which are reconstructed, fixed, expanded, and aligned with the bodily-responsive and thus bodily-injected insulin dosage value based on the retrospective horizon (RH) prediction (i.e. back in time to the dosing event) from the proposed machine learning approach that is specifically tuned, adjusted, and primed to the representative and generalizable sample-population-level general physiological response that is aligned with the particular input subject's dataset. Tightly captured in this same embodiment is the flexibility and scalability of this computing system to select and choose the appropriate machine learning approach model to be trained on new population-level data to be aligned with and representative of other or future real-world subjects to achieve generalizable predictions. In turn, the computer system comprises one or more processors and a memory, the memory comprising instructions that, when executed by the one or more processors, perform a method comprising: (a) obtaining a training set that is randomly shuffled and allocated fraction (e.g. 70%) of all available dataset that comprises training reference entities, particularly training insulin dose histories and training blood glucose histories, that are paired and aligned with each other, (b) obtaining a testing set that is randomly shuffled and allocated fraction (e.g. 30%) of all available dataset that comprises reference entities, particularly testing insulin dose histories and testing blood glucose histories, that are paired and aligned with each other, (c) for each training or testing reference entity, comprising of (i) a blood glucose measurement for each time point in a time course from the corresponding training blood glucose history and (ii) an insulin dose amount for each time point in a subset of time points in the same time course from the corresponding reference of insulin dose history, wherein blood glucose measurements that are usually temporally linear and forward-facing are processed and converted into retrospective horizon (RH) temporal segments of varying length, e.g. 60 minutes backwards, back to the timestamp of the dosing event. Additionally, these RH temporal segments of e.g. 60 are affected by the type of blood glucose measurements, thus by the types of devices that capture such blood glucose measurements. More specifically, blood glucose measurements are of predetermined type of blood glucose measurements, usually determined by the type of device that captures such blood glucose measurements. For Continuous Glucose Monitor (CGM) type for example at data resolution of 5 minute intervals, then a retrospective horizon (RH) of 60 minutes back would allow 12 CGM blood glucose measurements, for Flash Glucose Monitor (FGM) type for example at data resolution of 15 minute intervals, then the RH of 60 minutes back would allow instead only 4 FGM blood glucose measurements, other embodiments may take this RH setting anywhere from 5 minutes to 15 minutes, to 30 minutes, to 75 minutes, to 90 minutes or more back, depending on the blood glucose device's data resolution. The insulin dose measurements are of predetermined type of insulin dose, usually determined by the type of device that captures such insulin doses; insulin pens, pumps, and other such devices.

Insulin dose measurements occur at timestamped events with RH of e.g. 60 minutes backwards in time, not forwards in time. Thus, while this is a predictor, it's a retrospective predictor, meant for data quality cleaning purposes, instead of forecasting ahead purposes.

The method comprises the further step (d) that all available data is split (e.g. into 70-30% training and testing datasets) with application of various resampling techniques (e.g. K-Fold Cross Validation, jack-knife) applicable to machine learning and other data-driven learning that is not applicable to traditional hypothesis-based, statistical learning. These resampling techniques both estimate and evaluate the skill of the data-driven machine learning approach model on new, unseen data, better capturing the general physiological response in the studied sample population.

When modeling just a single individual patient's data and trying to get at the general physiological response, the modeler is limited to the power sample N of the subjects in the study, for example 1925. So, it's very cross-sectional, snapshot view of that particular sample population at that time and space. In short, only one single instance of a train subset, test subset, (and validation) subset is possible within the confines of traditional, statistical, hypothesis-driven modeling.

However when modeling for a population of patient's data and trying to get at the general physiological response, the modeler may elevate one's rigor beyond the power sample N of the subjects in the study. By splitting the available subjects into train, test and sometimes even validation sets (e.g. 60% train, 30% test, 10% validation) and able to resample into even groups of 5, 10, 100 or as much as warranted, one is effectively accounting for the variation in 1925 * 5 = 9625 instances of the same 1925 original subjects, 1925 * 10 = 19250 instances, and 1925 * 100 = 192500 instances across all groups. In short, with data-driven machine learning modelling, there are at least 5 and 10 (recommended but can and does get pushed higher to 50, 100, and 200) instances of different train, test, and validation subsets possible.

The present invention is attempting to fully optimize this resampling property of data-driven learning approach as opposed to hypothesis-driven learning approach.

The method comprises the further data quality check steps (e) in which beyond the train-test splitting, a series of tuned, adjusted, and primed re-sampling techniques may be performed to fully realize point (d)'s last point of fully optimizing this resampling property of data-driven learning approach as opposed to hypothesis-driven learning approach, and (f) in which beyond both train-test splitting and resampling, a series of pre-processing and timestamp growing techniques may be performed that further sift and filter through the dataset, in order to produce a train-test split, resampled, and pre-processed dataset for the training step up next. Most of these steps are dealing with the RH temporal segments, for which the concept of rolling binning may be used.

Further advantages of these resampling and pre-processing techniques are the retaining of any and all pertinent coefficients and markers of the machine learning model to be trained in the next step, thus unpacking an otherwise black-box training model from the reference training data.

When using resampling and pre-processing techniques for the defined Retrospective Horizon in the context of the addressed problem, the model's coefficients can be unpacked from its inception foundation.

Thus, not just any "trained model" falls into scope of the disclosed concept which uses glucose measurement data and insulin data. Instead, a trained model using resampling and pre-processing techniques which uses glucose measurement data that is defined within a retrospective horizon segment backwards in time and insulin data that is event-based back in the same RH segment back in time will satisfy the main requirements and constraints of the disclosed concept.

The method comprises the further steps of (g) training a model using training dataset's blood glucose measurements for the respective time course and the at least one reference insulin dose amount across the training set, thereby obtaining a trained model, and (h) for model evaluation purpose on the population-level, applying the trained model from (g) with the testing blood glucose histories (e.g. 30% of the dataset), obtaining one or more trained model output predicted values for one or more reconstructed insulin dose amounts in response to the test set's input of blood glucose histories, comparing the trained model's (g) predicted, reconstructed insulin dose amounts that may or may not align directly with the known, labelled, true, actual values, and evaluating the model by comparing the trained model-generated values with the real values from the test set.

For example, for a sample population of CGM users, the following 60-minute retrospective horizon (RH) of the following 12 CGM values as X inputs [300, 265, 250, 225, 200, 175, 150, 135, 125, 100, 90, 80] is paired up with the labelled, known, real value Y of say Y=34 IUs (insulin units), that is the Y_test because the model hasn't ever seen this particular X input of 12 CGM values. The model then recruits the trained model from step (g) in order to forecast retrospectively a predicted value, Y_test_predicted that is e.g. Y_pred = 32 IU. So then for evaluation, the predicted value of 32 IUs is 2 IUs below the actual, known or labelled value of 34 IU. A simple evaluation metric is the percent error: For example, Percent Error = (absolute value of $(V_{observed} - V_{true}) / V_{true}$)*100, which in this example is: $((|32 - 34|)/ 34)*100 = (2/34)*100 = 5.8824\%$. This is a competitive result where up to 15% error is usually acceptable, in most fields.

The method comprises the further steps of (i) applying the trained model from (g) on the individual subject's blood glucose history in order to retrospectively clean the insulin dosage data for the technical purpose of medical dose guidance request and recommendation, applying the trained model from (for example: for CGM user with RH60 with the following 12 CGM values as X inputs: [150, 145, 140, 135, 130, 125, 120, 100, 90, 80, 70, 65]), obtaining one or more trained model output predicted values for one or more reconstructed insulin dose amounts (for example, 42 IUs) in response to this particular individual subject's input of blood glucose histories, and (j) handshaking with the Medication Dose Guidance Request and Subject Parameters, verifying that the retrospectively reconstructed insulin dose is accepted by the Medication dose guidance request. Upon the Medication dose guidance request's confirmation, then the retrospectively reconstructed insulin dose overrides the previous insulin dose (for example, 49 IUs, thus correctly identifying e.g. a new pen with 7 units of air-shot), and completing this Medication Dose Guidance Request to send into the Recommendation engine providing a dose recommendation.

In a **yet further aspect,** disclosed herein is a computing system for performing data quality control of a dataset from a subject. As disclosed herein, the computer system comprises one or more processors and a memory. The memory comprises instructions that, when executed by the one or more processors, perform a method. The method comprises (a) obtaining a training set that comprises a plurality of reference entities, where the plurality of reference entities comprises a plurality of reference insulin dose histories and a plurality of reference blood glucose histories, where each reference insulin dose history is paired with a reference blood glucose history to form a reference entity in the plurality of reference entities. The method further comprises (b) for each respective reference entity in the plurality of reference entities, obtaining (i) a blood glucose measurement for each time point in a respective time course from the corresponding blood glucose history and (ii) an insulin dose amount for each time point in a respective subset of time points in the respective time course from the corresponding reference insulin dose history. Each respective reference entity in the plurality of reference entities provides a reference plurality of blood glucose measurements for the respective time course. The reference plurality of blood glucose measurements is of a predetermined type of blood glucose measurements, and each respective reference entity in the plurality of reference entities provides one or more reference insulin dose amounts in the respective subset of time points.

The one or more reference insulin dose amounts are of a predetermined type of insulin dose. The method further comprises (c) obtaining a test set that comprises a plurality of test entities. The plurality of test entities comprises a plurality of test insulin dose histories and a plurality of test blood glucose histories. Each test insulin dose history is paired with a test blood glucose history to form a test entity in the plurality of test entities. Each respective test blood glucose history provides a plurality of blood glucose measurements. Each respective test insulin dose history provides one or more test insulin dose amounts. Moreover, the one or more test insulin dose amounts are of a predetermined type of insulin dose. The method further comprises (d) training a model using the reference plurality of blood glucose measurements for the respective time course and the at least one reference insulin dose amount across the training set, thereby obtaining a trained model. The method further comprises (e) inputting the test plurality of blood glucose histories into the trained model thereby obtaining one or more trained model output values for the test set. The one or more trained output values comprise one or more reconstructed insulin dose amounts for the test set.

In some embodiments, the method further comprises: (f) obtaining from the dataset of a query subject a plurality of query subject blood glucose measurements in a respective time course and at least one query subject insulin dose amount in a respective subset of time points; and (g) inputting the plurality of query subject blood glucose measurements into the trained model, thereby obtaining one or more query model output values for the query subject. In such embodiments, the one or more query output values for the query subject comprise at least one or more reconstructed insulin dose amounts for the query subject.

In other embodiments, the one or more query output values for the query subject comprise at least one or more projected insulin dose amounts for the query subject.

In some embodiments, the plurality of reference insulin dose histories and the plurality of reference blood glucose histories are from a plurality of training subjects.

In some embodiments, the plurality of reference insulin dose histories and the plurality of reference blood glucose histories are from a single training subject.

In some embodiments, the method further comprises: (h) repeating the obtaining (f) and the inputting (g) for each query subject in a plurality of query subjects.

In some embodiments, each time point in the subset of time points immediately precedes a predefined time period in the time course. However, the step of performing preprocessing data as defined below may be able to produce the same subset of time points.

In some embodiments, the predefined period in the time course is selected from the set of 20-40 minutes, 30-60 minutes, 40-60 minutes, 50-70 minutes, 60-80 minutes, 80-100 minutes, 100-120 minutes or 120-180 minutes.

In some embodiments, the respective reference insulin dose history comprises a plurality of insulin doses administered to the corresponding reference entity during all or a portion of the time course and, for each respective insulin dose in the plurality of insulin doses, a corresponding insulin dose amount and an insulin dose timestamp representing when in the time course the respective insulin dose occurred. In such embodiments, the insulin dose timestamp is in the subset of time points.

In some embodiments, the plurality of reference entities comprises a plurality of glucose histories and a plurality of insulin dose histories from at least 10,000 reference subjects.

In some embodiments, the method further comprises providing one or more model performance metrics, including a root mean square error or an accuracy score.

In some embodiments, the one or more model performance metrics includes the root mean square error calculated as:

$$\text{RMSE} = \sqrt{\frac{1}{n}\sum_{j=1}^{n}\left(y_j - \hat{y}_j\right)^2}$$

where n is a positive integer of two or greater, j is an integer index that runs between 1 and $n$, each $y_j$ is a reconstructed insulin dose amount for the j$^{th}$ test entity in the plurality of test entities at a respective time point in a set of points, and each $\hat{y}_j$ is a corresponding test insulin dose amount for the j$^{th}$ test entity at the respective time point.

In some embodiments, the one or more model performance metrics includes the accuracy score (r$^2$) calculated as:

$$\text{r}^2 = 1 - \frac{\sum_{j=1}^{n}\left(y_j - y'_j\right)^2}{\sum_{j=1}^{n}\left(y_j - \overline{y'}\right)^2}$$

where n is a positive integer of two or greater, j is an integer index that runs between 1 and $n$, each $y_j$ is a test insulin dose amount for a j$^{th}$ test entity in the plurality of test entities at a respective time point in the set of points, each $y_j'$ is the reconstructed dose amount for the j$^{th}$ entity at the respective time point, and y' is the mean test insulin dose amount across n.

In some embodiments, the obtaining one or more trained model output values for the query subject further comprises obtaining a reconstructed blood glucose history.

In some embodiments, the method further comprises performing data preprocessing, data processing, and data optimization for the dataset of the query subject. In some such embodiments, the data preprocessing includes accessing the dataset of the query subject, selecting a predetermined type of insulin dose, selecting a predetermined type of blood glucose measurement, and reorganizing the plurality of query subject blood glucose measurements and the at least one subject insulin dose amounts into a plurality of preprocessed query subject blood glucose measurements and/or an at least one preprocessed query subject insulin dose amount. In some such embodiments, the data processing includes combining the preprocessed query subject blood glucose histories and the preprocessed query subject insulin dose histories into a query subject data table, and designating independent variables and dependent variables in the query subject data table. In some such embodiments, the data optimization includes applying the query subject data table to the trained model and obtaining at least one reconstructed or projected insulin dose amount for the query subject.

In some embodiments, the predetermined type of insulin dose consists of one or more bolus insulin dose events.

In some embodiments, the predetermined type of insulin dose consists of one or more basal insulin dose events.

In some embodiments, the data optimization further includes handling missing data, where one or more temporal gaps in the subject data table are interpolated by resampling the subject data table by a predefined time interval.

In some embodiments, the predefined time interval is 1 minute, 2 minutes, 5 minutes, 10 minutes, 15 minutes, 20 minutes, or 30 minutes.

In some embodiments, the predefined time interval is between 1 and 30 minutes.

In some embodiments, the model is a regression model selected from the group consisting of a linear regression model, a nonlinear regression model, a support vector machine, a random forest, a Keras artificial neural network, and a gradient tree boosting model.

In some embodiments, the predetermined type of blood glucose measurements is continuous glucose monitoring data. In some such embodiments, the continuous blood glucose monitoring data includes measurements taken at intervals of 30 seconds, 1 minute, 2 minutes 5 minutes, or 10 minutes.

In some embodiments, the predetermined type of blood glucose measurements is self-monitoring of blood glucose data. In some such embodiments, the self-monitoring of blood glucose data includes measurements taken at intervals of at least $\geq 1$ per hour, $\geq 1$ per day, $\geq 2$ per day, $\geq 3$ per day, $\geq 4$ per day, $\geq 1$ per 7 days, $\geq 1$ per 14 days, $\geq 4$ per 7 days, $\geq 4$ per 14 days, $\geq 1$ per 30 days, $\geq 4$ per 30 days.

In some embodiments, the predetermined type of insulin dose consists of one or more bolus insulin dose events and the one or more bolus insulin dose events occur over a predefined period in the time course selected from the set of 80-100 minutes, 100-120 minutes, 110-130 minutes, 120-140 minutes, 130-150 minutes, 140-160 minutes, 150-170 minutes or 160-180 minutes.

In some embodiments, the predetermined type of insulin dose consists of one or more basal insulin dose events and the one or more basal insulin dose events occur over a predefined period in the time course selected from the set of 20-40 minutes, 30-60 minutes, 40-60 minutes, 50-70 minutes, 60-80 minutes, 80-100 minutes, 100-120 minutes, 36 hours, or 24-48 hours.

## BRIEF DESCRIPTION OF THE DRAWINGS

In the following embodiments of the invention will be described with reference to the drawings, wherein

fig. 1 illustrates an example integrated computing system for providing medication dose guidance recommendations for a query subject to treat diabetes mellitus using reconstruction of insulin doses,

figs. 2A and 2B show a flowchart of processes and features for a first embodiment of a system providing a dose guidance recommendation,

figs. 2C and 2D illustrates how a TGL value is determined based on CGM data,

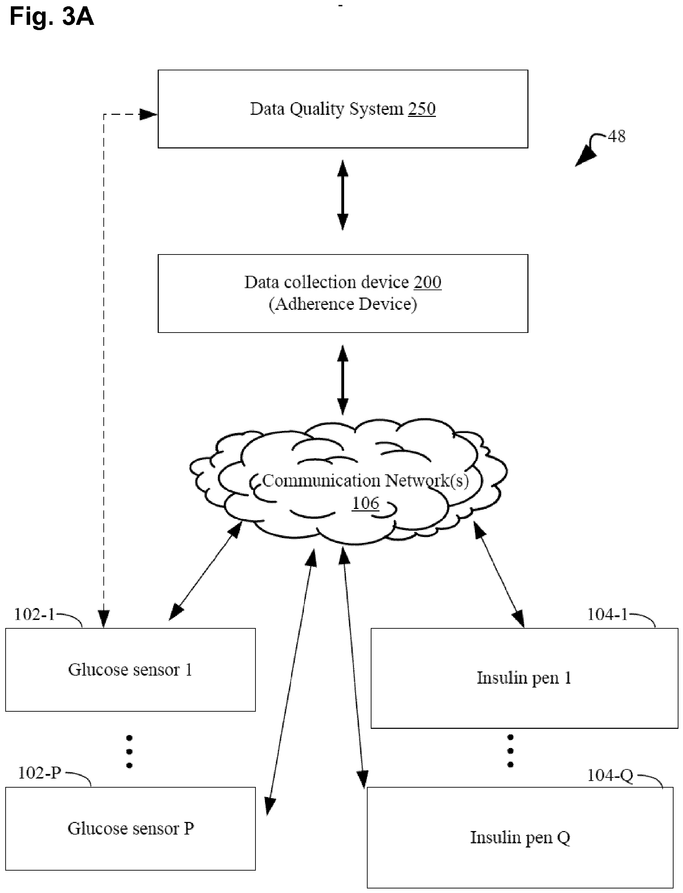

fig. 3A illustrates an exemplary system topology that includes a data quality checking system to verify and correct reported data from a subject, a regimen monitor device for autonomously adjusting a long acting insulin medicament dosage in a prescribed insulin regimen for the subject, a data collection device for collecting patient data, one or more glucose sensors that measure glucose data from the subject, and one or more insulin pens that are used by the subject to inject insulin medicaments in accordance with the prescribed insulin medicament regimen, where the above-identified components are interconnected, optionally through a communications network, in accordance with an embodiment of the present disclosure,

figs. 3B and 3C collectively illustrate a system for performing data quality checks in a prescribed insulin regimen in accordance with an embodiment of the present disclosure,

figs. 4A and 4B collectively provide a flow chart of processes and features of a device for autonomously performing data quality checks in a prescribed insulin regimen in accordance with various embodiments of the present disclosure,

fig. 5 illustrates an example of data input into the data quality check system in accordance with an embodiment of the present disclosure,

figs. 6A, 6B, 6C, 6D, 6E, 6F, 6G, 6H, 61, 6J, 6K, and 6L collectively illustrate an example data preprocessing module in accordance with an embodiment of the present disclosure,

figs. 7A, 7B, 7C, 7D, 7E, 7F, 7G, 7H, 7I and 7J collectively illustrate an example data processing module in accordance with an embodiment of the present disclosure,

figs. 8A and 8B collectively illustrate an example data optimization module in accordance with an embodiment of the present disclosure,

figs. 9A, 9B, 9C, 9D, 9E, 9F, and 9G collectively illustrate an example of training a random forest regressor model in accordance with an embodiment of the present disclosure,

figs. 10A, 10B, 10C, 10D, 10E, 10F, and 10G collectively illustrate an example of training a Keras artificial neural network regressor model in accordance with an embodiment of the present disclosure,

figs. 11A, 11B, 11C, 11D, 11E and 11F collectively illustrate an example of training a support vector machine regressor model in accordance with an embodiment of the present disclosure, and

figs. 12A, 12B, 12C, 12D, 12E and 12F collectively illustrate an example of training a gradient tree boosting regressor model in accordance with an embodiment of the present disclosure.

In the figures like structures are mainly identified by like reference numerals.

## DESCRIPTION OF EXEMPLARY EMBODIMENTS

Diabetes is a growing world health epidemic. Although diabetes can be effectively managed with established titration treatment regimens and pharmaceuticals, the data that is necessary for providing automatic titration recommendations requires quality checks to ensure accuracy. The present disclosure provides a data quality system to process patient-gathered data and thus enable automatic titration algorithms and self-titration. This enhances patient empowerment as well as substantially reducing treatment costs by reducing the frequency of required physician consultations for dose adjustments, all without reducing therapeutic outcomes.

The present disclosure relies upon the acquisition of sets of training and test data that include information relating to at least one subject. The dataset(s) include at least a plurality of glucose measurements of the subject taken over a time course to establish a blood glucose history, and for each respective glucose measurement in the plurality of glucose measurements a corresponding glucose timestamp representing when in the time course the respective glucose measurement was made, and one or more basal insulin injection histories, where the injection history includes a plurality of injections during all or a portion of the time course and, for each respective injection in the plurality of injections, a corresponding dose event amount and a dose event timestamp representing when in the time course the respective injection event occurred.

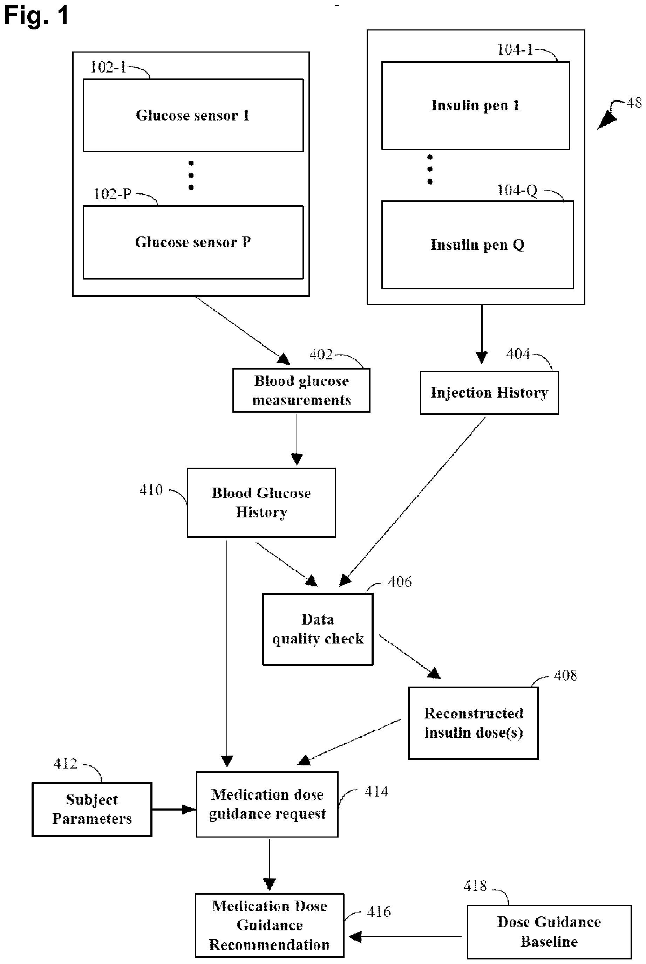

Referring to fig. 1 a computing system for providing medication dose guidance recommendations for a given patient to treat diabetes mellitus will be described. The system comprises one or more processors and a memory with instructions that, when executed by the one or more processors, perform a method responsive to receiving a dose guidance request (DGR).

The instructions comprising the steps of (a) obtaining a first data structure comprising patient (query subject) parameters, (b) obtaining a first data set comprising a plurality of glucose measurements of the patient taken over a time course and thereby establish a blood glucose history, each respective glucose measurement in the plurality of glucose measurements comprising (i) a blood glucose (BG) level, and (ii) a corresponding blood glucose timestamp representing when in the time course the respective glucose measurement was made, and (c) obtaining a second data set comprising an insulin injection history of the patient, wherein the injection history comprises a plurality of injections during all or a portion of the time course and, for each respective injection in the plurality of injections comprising (i) a corresponding injection amount, and (ii) an injection timestamp representing when in the time course the respective injection occurred.

The instructions comprise the further steps of (d) obtaining a trained model for retrospective insulin dose prediction based on blood glucose response data sets having a given retrospective horizon, (e) performing data optimization for the dataset of the query subject, the data optimization including inputting query subject data to the trained model and obtaining at least one reconstructed insulin dose amount for the query subject, and (f) providing a medication dose guidance recommendation, the recommendation being calculated based on data from the first data structure, the first data set, the second data set, and at least one reconstructed insulin dose amount for the query subject.

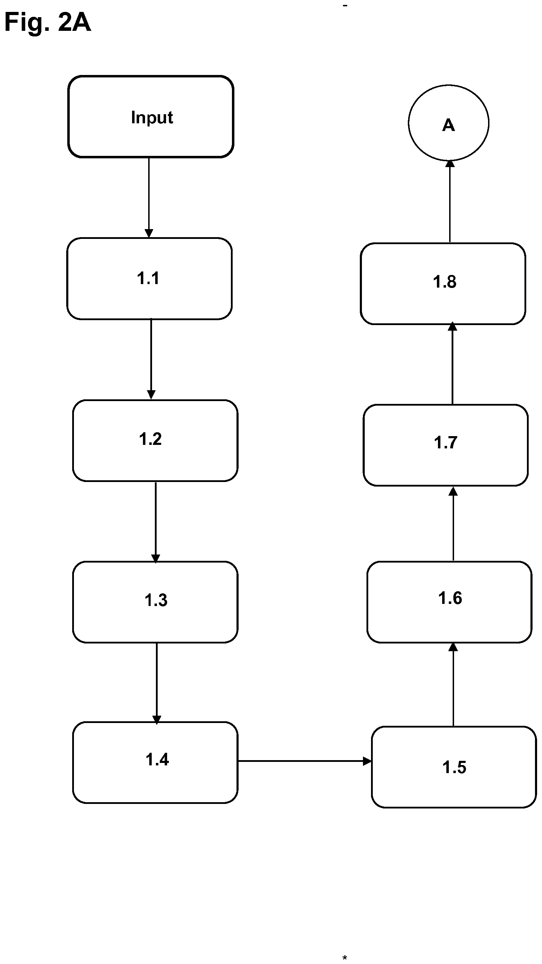

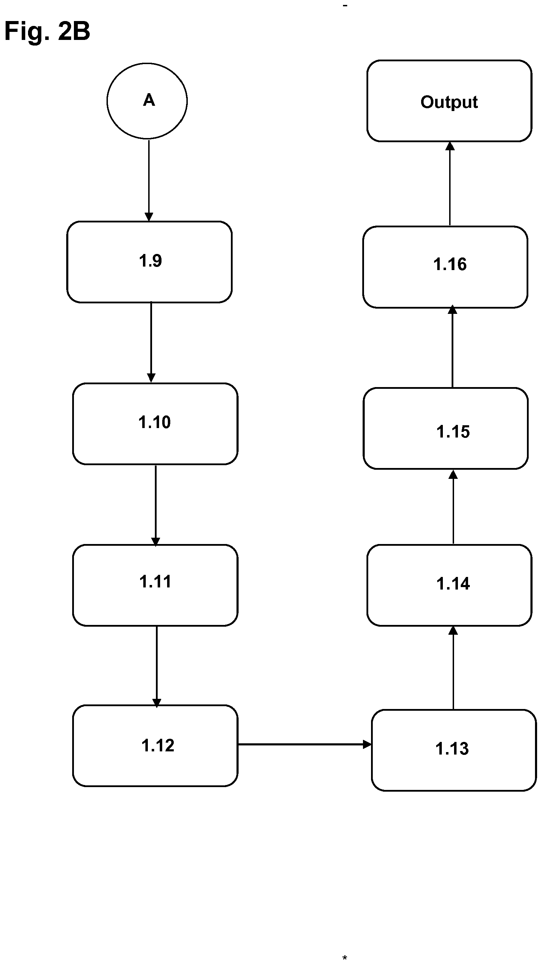

Having described the basic components of an exemplary embodiment of the invention, a specific embodiment will be described with reference to figs. 2A and 2B in which a flow-chart illustrates how a dose guidance request (DGR) is received and processed when the stored instructions are executed. The number of functions, the order of the functions and the specific functionality, e.g. rules, embedded in each give function are all exemplary. In the exemplary embodiment the described rules correspond to the titration label recommendations for Tresiba® from Novo Nordisk A/S.

### 1.1 Valid Request Check

This function is described above and checks that all the data types necessary in a dose guidance request (i.e. client input data) are present and within a specified range. In the event that a request lacks the appropriate data the function will returns an error message. Otherwise the check passes the data along.

For a given set-up additional input data may be required for a request to pass the check, e.g. a "hypo history" including any reported hypoglycemic events with a timestamp, a max basal limit for a given period of time, e.g. per day or per week, the time of request (TOR), a unique identifier of an individual dose guidance request, and a unique identifier of a client.

**1.2 Last Injection Data Refresh Check**

This function checks that the injection history data has been refreshed within a predetermined time limit, e.g. the last 30 seconds, this is to ensure that it has the most up to date record of the injection history data.

**1.3 Injection History Check**

This function checks whether there was already a dose event within the past 8 hours. If so, recommending another injection would violate the Tresiba labelling (i.e. take an injection once daily and at least 8 hours apart) and put the user at risk for a hypoglycemic event. If there are no dose events within the previous 8 hours, the check will pass the data along to the next function.

**1.4 Make-up injection check**

The Tresiba® labelling states that the user should take an injection once daily and at least 8 hours apart. But in the event that the patient forgets to take their daily dose, they can take the forgotten dose the next day in addition to their regularly scheduled dose (spaced out by at least 8 hours). Correspondingly, this function first checks to see if there's already been a dose event within the calendar day. If so, it then checks to see if the previous calendar day is without a dose event, and if this is true, it will pass the check. If there is a dose event recorded from the previous calendar day, the check will return an error message and not give a dose recommendation.

**1.5 Recorded hypoglycemic event check**

This function checks whether the data structure received with the request comprises an indicator for one or more recorded hypoglycemic events. If one or more hypoglycemic events have been recorded within a preset time limit no new ADDR will be calculated but the current DGB will be adjusted down with 2 UI of insulin. Hypoglycemic events may be recorded manually by the patient and/or may be registered automatically when a CGM is used for BG data capture. In the latter case the patient may be requested to confirm the event.

**1.6 BG data quality check**

In case BG data is supplied based on individual BG measurements, e.g. fasting BG, the function will check that a valid BG value is recorded for a specified number of days out of a specified number of recent days, e.g. at least 3 valid BG values for the last 4 days. In case BG data is supplied based on CGM measurements a CGM data quality check may be performed to ensure that there are no unacceptable gaps in the data that may lead to a wrong titration glucose level determination. Exemplary criterions for what an unacceptable level of CGM data quality is are described below. In the event that the data quality is not able to meet the minimum threshold, this function will return an error message and the dose guidance request will not proceed. Alternatively, it may be possible to fill in gaps within the CGM data. This is to increase the probability that despite gaps in the data (e.g. a new sensor is warming up) a dose guidance request will pass the next CGM data quality check. Examples of suitable mathematical approaches will be described in greater detail below.

**1.7 Dose event identification**

In a pen device with integrated dose logging functionality such as NovoPen® 6, according to its instructions for use, the patient should prime the device until they see insulin squirt out. These priming "injections" (or "air shots") are not differentiated from actual body injections by the pen and would be seen from the engine's perspective as individual injections taken into the body. As the number of actual injections taken by the patient is one of the parameters that may be checked by the dose guidance system to determine whether the patient is in compliance with the titration regimen, it is relevant to be able to filter out such priming "injections". This filtering could take place in the dose logging circuitry (whether integrated as in NovoPen® 6 or provided as an add-on logging device to be mounted on a pen device), in the patient's smartphone app which will typically be the device adapted to receive and collect injection data and BG data before they are transmitted as part of a request for an ADDR, or in the cloud engine. The filtering will typically be based on dose size (priming doses are generally (much) smaller than injection doses, and time between injections. A number of algorithms performing this analysis are known, e.g. as disclosed in US 2019/0035500. The filtering may be implemented at more than one level, as a subsequent filtering would just filter out nothing. The same considerations apply to "split doses", i.e. a (typically) large dose which by the patient is split into two separate injections, which should be combined to a "dose event", see below, in order not to count as two individual doses which would jeopardize a titration regimen based on the number of dose events.

As an alternative to filtering and in accordance with an aspect of the present invention, the actual injected dose amounts may be reconstructed using BG data as input to a trained model for the relationship between measured BG values and injected dose amounts of insulin, this as described in greater detail above and below.

For example: Two timestamps very close together (5 seconds apart) came in with two doses, first one with 2 IUs and second one with 20 IUs. This is a rather simple case where the RHIDP (Retrospective Horizon Insulin Dose Predictor) model has no trouble 60 minutes afterwards to correct the insulin dose trace and show correctly that the patient responded to only 20 IUs of e.g. Tresiba basal insulin - and thus not 22 IUs, thereby correctly identifying the first dose of 2 IUs as an air-shot. As a safety check the reconstructed value may be accepted in accordance with rules specifying e.g. the amount of deviation from the recorded dose amounts or the absolute size of the reconstructed value.

A more difficult example: Three timestamps this time, but still very close together (5 seconds apart) came in three doses: first dose is 15 IUs, second dose 7 IUs, third dose 15 IUs. How much was bodily injected? RHIDP correctly predicts that 30 IUs were bodily injected based on the bodily response in the BG trace, or how much the BG values fell or fluctuated, picked up by machine learning. What happened in this case was a split dose usually occurring when one pen device is ended and another is started. For example, the NovoPen® 6 have 7 IUs of air with the first dispense occurring from the pen device. Thus, the RHIDP model correctly identifies that 7 IUs were not bodily injected.

So, from the UI/UX or visibility side. First, 60 minutes back, the patient/user sees in their Insulin Dose Log: 15, 7, 15 and that is difficult for the HCP/provider or Decision Support system to figure out which insulin dose to use for that timestamp event. Here is where RHIDP helps out by identifying that the bodily injected and thus bodily responded value is 30 IUs. So then, the Insulin dose log becomes updated with 30 IUs of e.g. bodily injected Tresiba Basal Insulin doses and 7 IUs of air-shot. This example showcases split doses.

### 1.8 Dose event check

This function is checking to see if the patient has been sufficiently adherent in his/her injection routine. It looks for at least 3 dose events since the most recent ADDR, and that they have occurred within today and up to a previous 4 calendar day window. This extra calendar day that is being checked allows for a day that is without a dose (e.g. he/she forgot) and subsequently takes it the next day - in addition to the normally scheduled dose. If there are less than 3 dose events in this time frame (because the patient forgot multiple days) than this check will fail, and it will lead to a re-recommendation of the most recent ADDR made. Additionally, it will ensure only a single ADDR is dispensed and only re-recommendations follow in a scenario where repeated requests come in for an ADDR without any injections having since taken place.

### 1.9 DGB check

This function checks to see when the most recent ADDR was made - or when the starting basal dose (SBD) value was set. If the most recent ADDR was made within the current and previous 7 calendar days, then the check passes. If the most recent ADDR is older than this, than a new SBD amount must be inputted, and the dose guidance request process must be re-started from this new point. The new SBD would then be considered the current DGB.

### 1.10 Adherence check

This function checks the injection amounts of the three most recent dose events in the dataset to ensure that they are each at least equal to or greater than the amount of the most recent ADDR. If any of the dose events are less than this amount, the check will fail, and it will rerecommend the amount from the most recent ADDR. This ensures that the engine does not titrate up based on elevated glucose levels which are due to under-dosing.

### 1.11 Too many doses check

This function checks whether there are more than two dose events in the last 48 hours which would be against the recommendations of the Tresiba® label.

### 1.12 Daily TGL determination

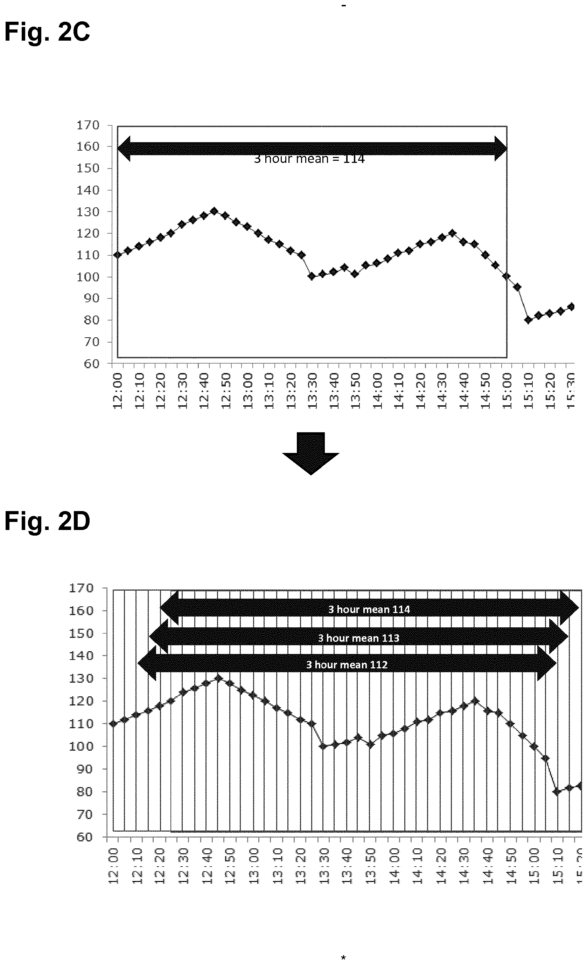

In case BG data is supplied based on individual BG measurements, e.g. fasting BG, the function will check that a valid BG value is recorded within a specified time range. In case BG data is supplied based on CGM measurements, this function looks at the CGM dataset and determines, if possible, a TGL for each day since most recent ADDR or up to a maximum of days, e.g. 4 days from the TOR, this being the dose guidance period. A "day" may be a calendar day (in which case some days would be partial), or it may be a 24 hours period calculated from e.g. the TOR, see below. It does this by applying e.g. a 3 hour "sliding window" across CGM readings one data point at the time, see figs. 2C and 2D, calculating the mean of each 3 hours interval from the current time up until the time of the last dose adjustment. In an exemplary embodiment a daily TGL is determined only for days having passed the BGH data quality test as described below. The lowest 3 hour mean for each day will be recorded as a TGL value and used for titration.

### 1.13 Daily TGL check

This check looks for any daily TGLs that are below the lower limit of the target glucose range parameter. If so, then the next ADDR is reduced with 2 IU from the most recent ADDR given. If not, the check passes onto the next function.

### 1.14 Average TGL determination

If at least a minimum number of valid daily TGL values have been determined, e.g. 2, for the dose guidance period, e.g. last 4 days, an average for the at least 2 TGL values is calculated.

### 1.15 Titration Determination

This function utilizes the overall TGL average, and if it is within the target range, the titration determination will be +0 IU from the most recent ADDR. If the TGL average is above the upper limit of the target range, then the next ADDR is increased by 2 IU from the most recent ADDR. If the TGL average is below the upper limit of the target range, then the next ADDR is lowered by 2 IU from the most recent ADDR.

### 1.16 Maximum limit check

To prevent overdosing this function checks a that a given dose maximum for a given period of time has not been exceeding, e.g. a maximum of 300 IU of Tresiba® for the last week. A patient specific value may also be included in the request data. Alternatively, the function may check whether the next ADDR would exceeded the patient's "overbasalisation" limit (BW (kg) * OBL (IU/kg)). This would require that a body weight (BW) of the subject, and an overbasalisation limit (OBL) of the subject have been provided as part of the data received with the request.

### 1.17 Output

The main output from the engine as a reply to a dose guidance request is indeed the ADDR as received by the client, however, additional information may be of use to the patient, either directly related to the patient's treatment, e.g. TGL values calculated by the engine based on CGM values as well as specific error or warning messages, or validation data to improve reliability and safety. Correspondingly, the output may comprise one or more of the following types of data: User ID, transaction ID, ADDR, day TGLs, overall TGL, dose event history, hypoglycaemia history, warning and error codes.

Turning to fig. 3A an example of an integrated system 48 for performing data quality control in a prescribed insulin regimen for a subject in accordance with an aspect of the present disclosure is described in conjunction with figs. 3A-3C.

for the acquisition of such data is illustrated. The integrated system 48 includes one or more connected insulin pens 104, one or more glucose monitors 102, memory and a processor (not shown) for performing data quality control of autonomous insulin injection data of a subject. In some embodiments, a glucose monitor 102 is a continuous glucose monitoring device. In some embodiments, a glucose monitor 102 is a self-monitoring glucose device.

Reference will now be made in detail to embodiments, examples of which are illustrated in the accompanying drawings. In the following detailed description of implementations, numerous specific details are set forth in order to provide a thorough understanding of the present invention. However, it will be apparent to one of ordinary skill in the art that the present invention may be practiced without these specific details.

Definitions

The terminology used in the present disclosure is for the purpose of describing particular embodiments only and is not intended to be limiting of the invention. It will also be understood that the term "and/or" as used herein refers to and encompasses any and all possible combinations of one or more of the associated listed items. It will be further understood that the terms "includes" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

The term "or" is intended to mean an inclusive "or" rather than an exclusive "or." That is, unless specified otherwise, or clear from the context, the phrase "X employs A or B" is intended to mean any of the natural inclusive permutations. That is, the phrase "X employs A or B" is satisfied by any of the following instances: X employs A; X employs B; or X employs both A and B.

It will also be understood that, although the terms first, second, *etc.* may be used herein to describe various elements, these elements should not be limited by these terms. These terms are only used to distinguish one element from another. For example, a first filter could be termed a second filter, and, similarly, a second filter could be termed a first filter. The first filter and the second filter are both filters, but they are not the same filter.

The terms "subject," "individual," and "user" are used interchangeably herein and refer to humans. Preferably, the individual is an adult individual.

The term "diabetes" or "diabetes mellitus" includes type 1 diabetes mellitus, type 2 diabetes mellitus, gestational diabetes (during pregnancy) and other states that cause hyperglycemia. The term is used for a metabolic disorder in which the pancreas produces insufficient amounts of insulin, or in which the cells of the body fail to respond appropriately to insulin thus preventing cells from absorbing glucose. As a result, glucose builds up in the blood and treatment is required to control blood glucose levels.

According to the present invention, basal insulin comprises or consists of long-acting insulin and ultra-long acting insulin. According to the present invention, bolus insulin comprises or consists of short-acting and rapid-acting insulin. In principle, the longer the half-life of the insulin, the more stable and evenly distributed the glucose-lowering effect over a dosing interval (i.e. time interval between injections). Both basal and bolus insulin can be administered to a subject with an insulin pen.

By the term insulin pen is meant an injection device suitable for applying discrete doses of insulin, and wherein the injection device is adapted for logging and communicating dose related data.

According to the present invention, both the basal insulin and the bolus insulin are administered in an amount to achieve a beneficial glycemic control in said subject. According to the present invention, the beneficial glycemic control in said subject is determined by at least the levels of $HbA_{1c}$ (glycosylated hemoglobin) in said subject after administration of said basal insulin or of said bolus insulin.

As used herein the term "U" refers to a unit of insulin (or an analogue or derivative thereof). The designation "U" with a number following indicates the concentration as measured by the number of units per ml of fluid volume (Joslin's Diabetes Deskbook, 2nd edition, Chapter 9 Using insulin to treat diabetes - general principles, page 268). Further information about the meaning of "U" can be found in a document from the EMA (reference EMEA/CHMP/ BWP/ 124446/2005) entitled "Guideline on potency labelling for insulin analogue containing products with particular reference to the use of "International Units" or "Units"" (see http://www.ema.europa.eu/docs/en_GB/document_library/Scientific_guideline/2009/09/WC500003654.pdf). "IU" refers to an international unit of human insulin as defined according to the WHO Expert Committee on Biological Standardization. IU is a standardized parameter. For commercial insulins, the labels indicate the content of 1 U (unit) of the particular insulin analogue.

As used herein, the term "medicament" refers to a chemical substance used for medical treatment of a subject. The terms "medicament," "medication," and "drug" are used interchangeably herein. The medicament may be prescribed by a physician or other health care provider. Alternatively, the medicament may be an over-the-counter drug or product. In a specific example of the present disclosure, the medicament is insulin, which is used to treat diabetes mellitus.

Description

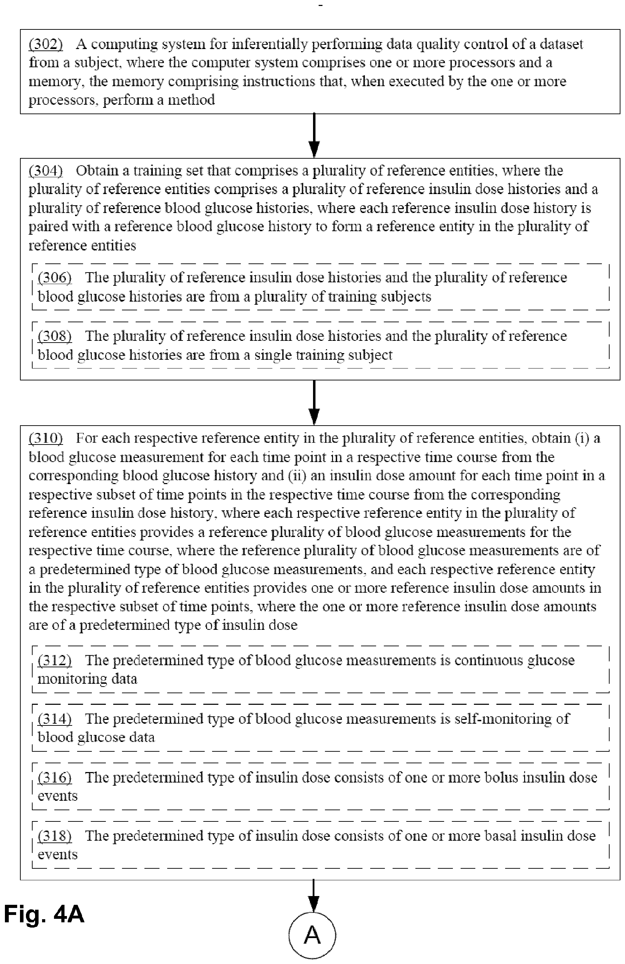

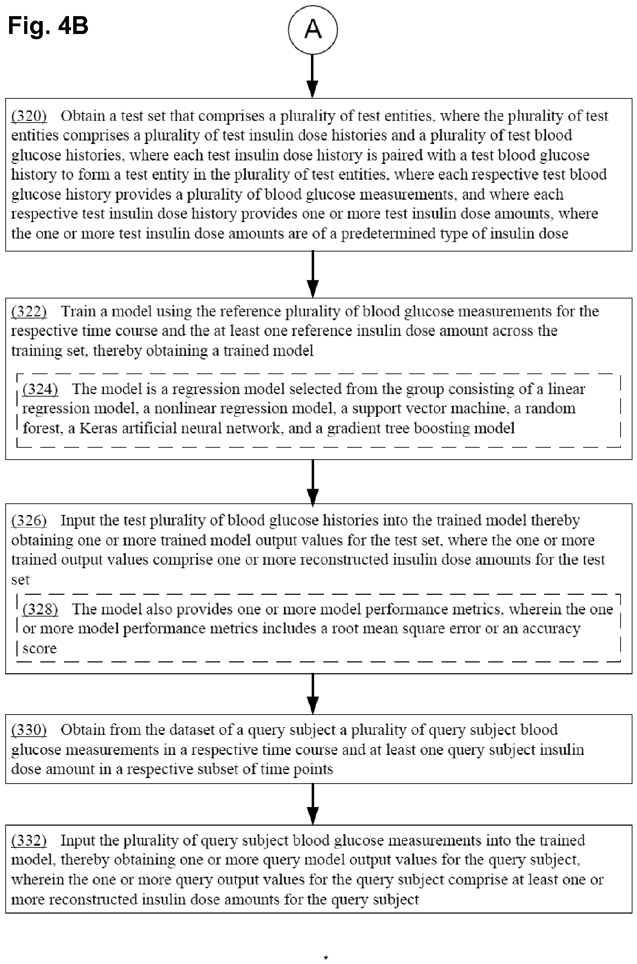

A detailed description of a system 48 for performing data quality control in a prescribed insulin regimen for a subject in accordance with aspects of the present disclosure is described in conjunction with figs. 1 and 2. As such, figs. 1 and 2 collectively illustrate the topology of an exemplary system in accordance with the present disclosure. In the topology, there is a data quality control system for autonomously providing a data quality check of blood glucose data and insulin dose data collected as part of a prescribed insulin regimen for a subject ("Data Quality System 250") (figs. 1 and 2), a device for data collection ("data collection device 200"), one or more glucose sensors 102 associated with the subject (figs. 1 and 4), and one or more insulin pens 104 for injecting insulin medicaments into the subject ( figs. 1 and 4).

Throughout the present disclosure, the data collection device 200 and the data quality system 250 will be referenced as separate devices solely for purposes of clarity. That is, the disclosed functionality of the data collection device 200 and the disclosed functionality of the data quality system 250 are contained in separate devices as illustrated in fig. 3A. However, it will be appreciated that, in fact, in some embodiments, the disclosed functionality of the data collection device 200 and the disclosed functionality of the data quality system 250 are contained in a single device. In some embodiments, the disclosed functionality of the data collection device 200 and/or the disclosed functionality of the data quality system 250 are contained in a single device and this single device is a glucose monitor 102 or the insulin pen 104.

Referring to fig. 3A, the data quality system 250 autonomously performs a data quality control verification of insulin medicament dosage in a prescribed insulin regimen for a subject. To do this, the data collection device 200, which is in electrical communication with the data quality system 250, receives autonomous glucose measurements originating from one or more glucose sensors 102 attached to a subject on an ongoing basis. In some embodiments, the data collection device 200 also receives insulin medicament injection data from one or more insulin pens 104 used by the subject to inject insulin medicaments. In some embodiments, the data collection device 200 receives such data directly from the glucose sensor(s) 102 and insulin pens 104 used by the subject. For instance, in some embodiments the data collection device 200 receives this data wirelessly through radio-frequency signals. In some embodiments such signals are in accordance with an 802.11 (Wi-Fi), Bluetooth, or ZigBee standard. In some embodiments, the data collection device 200 receives such data directly, analyzes the data, and passes the analyzed data to the data quality system 250. In some embodiments, a glucose sensor 102 and/or insulin pen 104 includes an RFID tag and communicates to the data collection device 200 and/or the data quality system 250 using RFID communication. In some embodiments, the data collection device 200 also obtains or receives physiological measurements 210 of the subject (e.g., from wearable physiological measurement devices, from measurement devices within the data collection device 200 such as a magnetometer or thermostat, etc.). For some embodiments of the present invention, said insulin pen device is FlexPen®(s), FlexTouch®(s), NovoPen®(s), or NovoPen Echo®(s). FlexPen®, FlexTouch®, NovoPen®, and NovoPen Echo® are trademarks of Novo Nordisk A/S.

In some embodiments, the data collection device 200 and/or the data quality system 250 is not proximate to the subject and/or does not have wireless capabilities or such wireless capabilities are not used for the purpose of acquiring glucose data, insulin medicament injection data, and/or physiological measurement data. In such embodiments, a communication network 106 may be used to communicate glucose measurements from the glucose sensor 102 to the data collection device 200 and/or the data quality system 250, insulin medicament injection data from the one or more insulin pens 104 to the data collection device 200 and/or the data quality system 250, and/or physiological measurement data from one or more physiological measurement devices (not shown) to the data collection device 200 and/or the data quality system 250.

Examples of networks 106 include, but are not limited to, the World Wide Web (WWW), an intranet and/or a wireless network, such as a cellular telephone network, a wireless local area network (LAN) and/or a metropolitan area network (MAN), and other devices by wireless communication. The wireless communication optionally uses any of a plurality of communications standards, protocols and technologies, including but not limited to Global System for Mobile Communications (GSM), Enhanced Data GSM Environment (EDGE), high-speed downlink packet access (HSDPA), high-speed uplink packet access (HSUPA), Evolution, Data-Only (EV-DO), HSPA, HSPA+, Dual-Cell HSPA (DC-HSPDA), long term evolution (LTE), near field communication (NFC), wideband code division multiple access (W-CDMA), code division multiple access (CDMA), time division multiple access (TDMA), Bluetooth, Wireless Fidelity (Wi-Fi) (e.g., IEEE 802.11a, IEEE 802.11ac, IEEE 802.11ax, IEEE 802.11b, IEEE 802.11g and/or IEEE 802.11n), voice over Internet Protocol (VoIP), Wi-MAX, a protocol for e-mail (e.g., Internet message access protocol (IMAP) and/or post office protocol (POP)), instant messaging (e.g., extensible messaging and presence protocol (XMPP), Session Initiation Protocol for Instant Messaging and Presence Leveraging Extensions (SIMPLE), Instant Messaging and Presence Service (IMPS)), and/or Short Message Service (SMS), or any other suitable communication protocol, including communication protocols not yet developed as of the filing date of the present disclosure.

In some embodiments, there is a single glucose sensor 102 attached to the subject and the data collection device 200 and/or the data quality system 250 is part of the glucose sensor 102. That is, in some embodiments, the data collection device 200 and/or the data quality system 250 and the glucose sensor 102 are a single device.

In some embodiments, the data collection device 200 and/or the data quality system 250 is part of an insulin pen. That is, in some embodiments, the data collection device 200 and/or the data quality system 250 and an insulin pen 104 are a single device.

Of course, other topologies of the system 48 are possible. For instance, rather than relying on a communications network 106, the one or more glucose sensors 102 and the one or more insulin pens 104 may wirelessly transmit information directly to the data collection device 200 and/or regimen monitor device 250. Further, the data collection device 200 and/or the data quality system 250 may constitute a portable electronic device, a server computer, or in fact constitute several computers that are linked together in a network or be a virtual machine in a cloud computing context. As such, the exemplary topology shown in fig. 3A merely serves to describe the features of an embodiment of the present disclosure in a manner that will be readily understood to one of skill in the art.

While the system 48 disclosed in fig. 3A can work standalone, in some embodiments it can also be linked with electronic medical records to exchange information in any way described above.

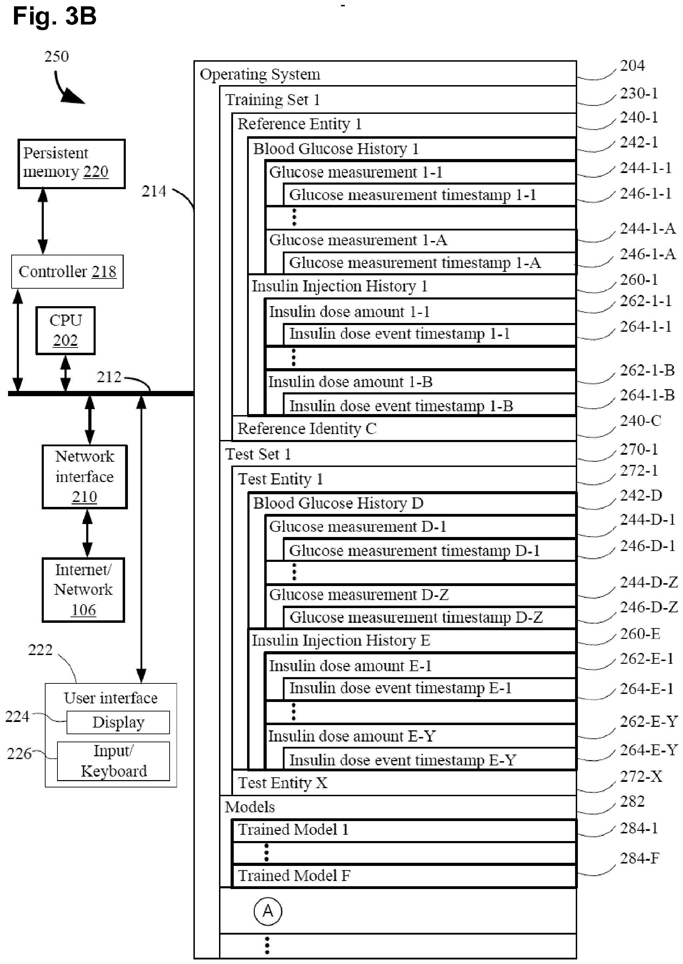

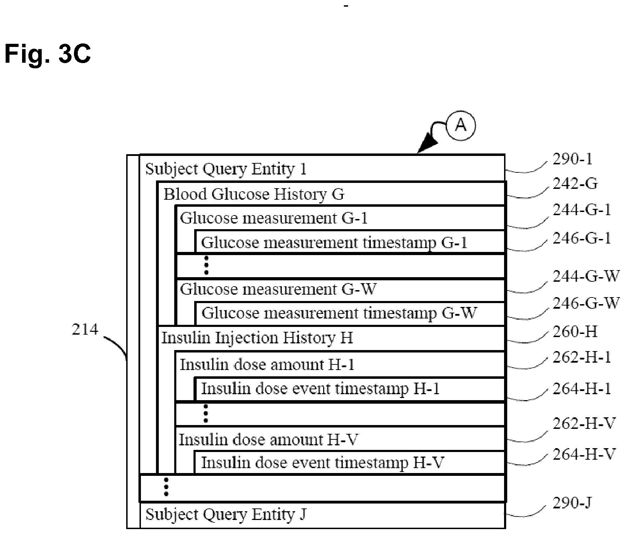

Referring to figs. 3B and 3C, in typical embodiments, the data quality system 250 comprises one or more computers. For purposes of illustration in figs. 3B and 3C, the data quality system 250 is represented as a single computer that includes all of the functionality for performing data quality control of a dataset from a subject. However, the disclosure is not so limited. In some embodiments, the functionality for performing data quality control of a dataset from a subject is spread across any number of networked computers and/or resides on each of several networked computers and/or is hosted on one or more virtual machines at a remote location accessible across the communications network 106. One of skill in the art will appreciate that any of a wide array of different computer topologies are used for the application and all such topologies are within the scope of the present disclosure.

In some embodiments, an exemplary data quality system 250 for performing data quality control of a dataset from a subject comprises one or more processing units (CPU's) 202, a network or other communications interface 210, a memory 214 (e.g., random access memory), one or more magnetic disk storage and/or persistent devices 220 optionally accessed by one or more controllers 218, one or more communication busses 212 for interconnecting the aforementioned components, and a power supply 224 for powering the aforementioned components. In some embodiments, data in memory 214 is seamlessly shared with non-volatile memory 220 using known computing techniques such as caching. In some embodiments, memory 214 and/or memory 220 includes mass storage that is remotely located with respect to the central processing unit(s) 202. In other words, some data stored in memory 214 and/or memory 220 may in fact be hosted on computers that are external to the data quality control computer system 250 but that can be electronically accessed by the data quality control computer system 250 over an Internet, intranet, or other form of network or electronic cable (illustrated as element 106 in fig. 3A) using network interface 210.

In some embodiments, the memory 214 of the data quality system 250 for performing data quality control of a dataset from a subject stores:

- an operating system 204 that includes procedures for handling various basic system services;
- one or more training sets 230, each training set (e.g. 230-1) comprising a plurality of reference entities 240, each reference entity (e.g. 240-1 and 240-C) comprising:

  ◦ a plurality of blood glucose histories 242, each blood glucose history (e.g. 242-1) comprising a plurality of glucose measurements 244 of a subject over the time course, and for each respective glucose measurement in the plurality of glucose measurements (e.g. 244-1-1 and 244-1-A), a timestamp 246 representing when the respective glucose measurement was made (e.g. 246-1-1 and 246-1-A); and
  ◦ a plurality of insulin injection histories 260, each insulin injection history (e.g. 260-1) comprising one or more insulin dose amounts 262, and for each respective insulin dose amount in the one or more insulin dose amounts (e.g. 262-1-1 and 262-1-B), a timestamp 264 representing when the respective insulin injection was made (e.g. 264-1-1, and 264-1-B);

- one or more test sets 270, each test set (e.g. 270-1) comprising a plurality of test entities 272, each test entity (e.g. 272-1 and 272-X) comprising:

  ◦ a plurality of blood glucose histories 242, each blood glucose history (e.g. 242-D) comprising a plurality of glucose measurements 244 of a subject over the time course, and for each respective glucose measurement in the plurality of glucose measurements (e.g. 244-D-1, and 244-D-Z), a timestamp 246 representing when the respective glucose measurement was made (e.g. 246-D-1 and 246-D-Z); and
  ◦ a plurality of insulin injection histories 260, each insulin injection history (e.g. 260-E) comprising one or more insulin dose amounts 262, and for each respective insulin dose amount in the one or more insulin dose amounts (e.g. 262-E-1 and 262-E-Y), a timestamp 264 representing when the respective insulin injection was made (e.g. 264-E-1, and 264-E-Y);

- one or more models 282, where each trained model (e.g. 284-1 and 284-F) may be one of one or more model types (e.g. regression model types such as a linear regression model, a nonlinear regression model, a support vector machine, a random forest, a Keras artificial neural network, and/or a gradient tree boosting model), and where each trained model 284 may be trained using the plurality of reference entities (e.g. 240-1 and 240-C) and the validity of each trained model 284 may be verified using the plurality of test entities (e.g. 272-1 and 272-272-X); and
- one or more subject query entities 290, each subject query entity (e.g. 290-1 and 290-J) comprising:

  ◦ a plurality of blood glucose histories 242, each blood glucose history (e.g. 242-G) comprising a plurality of glucose measurements 244 of a subject over the time course, and for each respective glucose measurement in the plurality of glucose measurements (e.g. 244-G-1, and 244-G-W), a timestamp 246 representing when the respective glucose measurement was made (e.g. 246-G-1 and 246-G-W); and

&#9702; a plurality of insulin injection histories 260, each insulin injection history (e.g. 260-H) comprising one or more insulin dose amounts 262, and for each respective insulin dose amount in the one or more insulin dose amounts (e.g. 262-H-1 and 262-H-V), a timestamp 264 representing when the respective insulin injection was made (e.g. 264-H-1, and 264-H-V).

In some embodiments, the data quality control module 250 is accessible within any browser (phone, tablet, laptop/desktop). In some embodiments the data quality control module 250 runs on native device frameworks, and is available for download onto an operating system 204 such as Android or iOS.

In some implementations, one or more of the above identified data elements or modules of the data quality system 250 for performing data quality control of a dataset from a subject are stored in one or more of the previously described memory devices, and correspond to a set of instructions for performing a function described above. The above-identified data, modules or programs (e.g., sets of instructions) need not be implemented as separate software programs, procedures or modules, and thus various subsets of these modules may be combined or otherwise re-arranged in various implementations. In some implementations, the memory 214 and/or 220 optionally stores a subset of the modules and data structures identified above. Furthermore, in some embodiments the memory 214 and/or 220 stores additional modules and data structures not described above.

In some embodiments, a regimen monitor device 250 for performing data quality control of a dataset from a subject is a smart phone (e.g., an iPhone), laptop, tablet computer, desktop computer, or other form of electronic device (e.g., a gaming console). In some embodiments, the data quality system 250 is not mobile. In some embodiments, the data quality system 250 is mobile. In other embodiments, the data quality system 250 is not a smart phone but rather is a tablet computer, desktop computer, emergency vehicle computer, or other form or wired or wireless networked device. In some embodiments, the data quality system 250 has any or all of the circuitry, hardware components, and software components found in the regimen monitor device 250 depicted in figs. 2A and 2B. In the interest of brevity and clarity, only a few of the possible components of the data quality system 250 are shown in order to better emphasize the additional software modules that are installed on the data quality system 250.

The data quality system 250 accesses and/or stores the one or more training sets 230, the one or more test sets 270, the one or more trained models 284, and/or the one or more subject query entities 290.

In some embodiments, the glucose measurements 244 are autonomously measured. The FREESTYLE LIBRE CGM by ABBOTT ("LIBRE") is an example of a glucose sensor that may be used as a glucose sensor 102 in order to make autonomous glucose measurements of a subject. The LIBRE allows calibration-free glucose measurements with an on-skin coin-sized sensor, which can send up to eight hours of data to a reader device (e.g., the data collection device 200 and/or the data quality system 250) via near field communications, when brought close together. The LIBRE can be worn for fourteen days in all daily life activities.

In some embodiments, the insulin medicament specified by the insulin medicament dosage regimen consists of a single insulin medicament having a duration of action that is between 12 and 24 hours or a mixture of insulin medicaments that collectively have a duration of action that is between 12 and 24 hours. Examples of such long acting insulin medicaments include, but are not limited to Insulin Degludec (developed by NOVO NORDISK under the brand name Tresiba), NPH (Schmid, 2007, "New options in insulin therapy. J Pediatria (Rio J). 83(Suppl 5):S146-S155), Glargine (LANTUS, March 2, 2007, insulin glargine [rDNA origin] injection, [prescribing information], Bridgewater, New Jersey: Sanofi-Aventis), and Determir (Plank et al., 2005, "A double-blind, randomized, dose-response study investigating the pharmacodynamic and pharmacokinetic properties of the long-acting insulin analog detemir," Diabetes Care 28:1107-1112).

In some embodiments, the plurality of glucose measurements 244 (e.g. part of one or more training sets 230, one or more test sets 270, and/or one or more subject query entities 290) is limited to glucose measurements taken from the subject in the past four hours, the past twelve hours, the past 24 hours, the past two days, the past week, or the past two weeks.

Now that details of a system 48 and system 250 for performing data quality control of a dataset from a subject have been disclosed, details regarding a flow chart of processes and features of the system, in accordance with an embodiment of the present disclosure, are disclosed with reference to figs. 4A and 4B.

With reference to block 302 in fig. 4A, a goal of the present disclosure is to perform data quality control of a dataset from a subject. In some embodiments, the data control is performed using a device such as the data collection device 200 and a data quality system 250. As illustrated in figs. 3B and 3C, the device includes one or more processors 202 and a memory 214/220. In some embodiments, the data quality control is performed upon a request from a subject. In some embodiments, the data quality control is performed upon a request from a health care provider. In some embodiments, the data quality control is performed automatically. In some embodiments, the data quality control is performed automatically on a recurring basis.

With reference to block 304 in fig. 4A, the data quality control system continues by obtaining a training set that comprises a plurality of reference entities, where the plurality of reference entities comprises a plurality of reference insulin dose histories and a plurality of reference blood glucose histories, where each reference insulin dose history is paired with a reference blood glucose history to form a reference entity in the plurality of reference entities

In some embodiments, the plurality of reference insulin dose histories and the plurality of reference blood glucose histories are from a plurality of training subjects. In some embodiments, the plurality of subjects comprises at least 100 subjects, at least 1,000 subjects, at least 5,000 subjects, at least 10,000 subjects, at least 50,000 subjects or at least 100,000 subjects. In some embodiments, each training subject provides one reference insulin dose history and one reference blood glucose history. In some embodiments, each training subject provides more than one reference dose history and more than one reference blood glucose history.

In some embodiments, the plurality of reference insulin dose histories and the plurality of reference blood glucose histories are from a single training subject. In some embodiments, the single training subject provides one reference insulin dose history and one reference blood glucose history. In some embodiments, the single training subject provides more than one reference dose history and more than one reference blood glucose history.

With reference to block 310 in fig. 4A, the data quality control system continues by obtaining, for each respective reference entity in the plurality of reference entities, (i) a blood glucose measurement for each time point in a respective time course from the corresponding blood glucose history and (ii) an insulin dose amount for each time point in a respective subset of time points in the respective time course from the corresponding reference insulin dose history, where each respective reference entity in the plurality of reference entities provides a reference plurality of blood glucose measurements for the respective time course, where the reference plurality of blood glucose measurements are of a predetermined type of blood glucose measurements, and each respective reference entity in the plurality of reference entities provides one or more reference insulin dose amounts in the respective subset of time points, where the one or more reference insulin dose amounts are of a predetermined type of insulin dose.

In some embodiments, the predetermined type of blood glucose measurements is continuous glucose monitoring data. In some embodiments, the predetermined type of blood glucose measurements is self-monitoring of blood glucose data. In some embodiments, the predetermined type of insulin dose consists of one or more bolus insulin dose events. In some embodiments, the predetermined type of insulin dose consists of one or more basal insulin dose events. In some embodiments, the respective time course is selected from the set of 20-40 minutes, 30-60 minutes, 40-60 minutes, 50-70 minutes, 60-80 minutes, 80-100 minutes or 100-120 minutes.

With reference to block 320 in fig. 4B, the data quality control system continues by obtaining a test set that comprises a plurality of test entities, where the plurality of test entities comprises a plurality of test insulin dose histories and a plurality of test blood glucose histories, where each test insulin dose history is paired with a test blood glucose history to form a test entity in the plurality of test entities, where each respective test blood glucose history provides a plurality of blood glucose measurements, and where each respective test insulin dose history provides one or more test insulin dose amounts, where the one or more test insulin dose amounts are of a predetermined type of insulin dose

With reference to block 322 in fig. 4B, the data quality control system continues by training a model using the reference plurality of blood glucose measurements for the respective time course and the at least one reference insulin dose amount across the training set, thereby obtaining a trained model.

In some embodiments, the model is a regression model selected from the group consisting of a linear regression model, a nonlinear regression model, a support vector machine, a random forest, a Keras artificial neural network, and a gradient tree boosting model. In some embodiments, the model is a classifier model or an ensemble model. In some embodiments the model is a supervised learning model, an unsupervised learning model, a semi-supervised learning model, or a reinforcement learning model. In some embodiments, the type of model is selected form the set of nearest neighbor, naive Bayes, decision trees, linear regression, support vector machines, neural networks, k-means clustering, q-learning, temporal difference, and deep adversarial networks.

With reference to block 326 in fig. 4B, the data quality control system continues by inputting the test plurality of blood glucose histories into the trained model thereby obtaining one or more trained model output values for the test set, where the one or more trained output values comprise one or more reconstructed insulin dose amounts for the test set.

In some embodiments, the reconstructed insulin dose amount (e.g. the insulin dose amount that is predicted to have been actually bodily injected based on the blood glucose measurements) is a positive integer. In some embodiments, the reconstructed insulin dose amount is represented by a floating-point number. In some embodiments the prediction outcome metric is in the set of between 0 IU and 5 IU, between 0 IU and 10 IU, between 0 IU and 25 IU, between 0 IU and 50 IU, between 0 IU and 100 IU, between 0 IU and 150 IU, between 0 IU and 200 IU, and between 0 IU and 250 IU.

In some embodiments, the data quality system further provides one or more model performance metrics. In some embodiments, these one or more model performance metrics includes a root mean square error, a mean absolute error, an explained variance score, or an accuracy score.

In some embodiments, the first major performance metric is the "Accuracy" score that is actually the coefficient of determination or r-squared ($r^2$) of the prediction. The closer to maximum of "1" this accuracy metric is, the better the model is at forecasting the bodily injected insulin dose amount from the response-based BG profile curve.

Formula: "Accuracy" Score or Coefficient of Determination (r-squared):

$$r^2 = 1 - \frac{\sum_{j=1}^{n} \left( y_j - y'_j \right)^2}{\sum_{j=1}^{n} (y_j - \bar{y'})^2}.$$

The accuracy score is a statistic that provides information about the goodness of fit of a model. In regression, the r-squared coefficient of determination is a statistical measure of how well the regression predictions approximate the real data points. An r-squared value of "1" indicates that the regression predictions perfectly fit the data. The coefficient r-squared is defined as (1 - u/v), where u is the residual sum of squares (e.g. $\Sigma(y - y')^2$) and v is the total sum of squares (e.g. $\Sigma(y - \bar{y'})^2$). The best possible score is 1.0, and the score can be negative (because the model can be arbitrarily worse).

In some embodiments, the second major performance metric is the Root Mean Square Error (RMSE). The smaller this error metric, the better is the model at forecasting the bodily injected insulin dose amount from the give blood glucose measurements.

Formula: Root Mean Square Error (RMSE):

$$\text{RMSE} = \sqrt{\frac{1}{n}\sum_{j=1}^{n}\left(y_j - \hat{y}_j\right)^2}.$$

The root-mean-square error (RMSE) or root-mean-square deviation (RMSD) is a frequently used measure of the differences between values (sample and population values) predicted by a model or an estimator and the values actually observed. The RMSE represents the sample standard deviation of the differences between predicted values and observed values. These individual differences are called residuals when the calculations are performed over the data sample that was used for estimation, and are called prediction errors when computed out-ofsample. The RMSE serves to aggregate the magnitudes of the errors in predictions for various times into a single measure of predictive power. RMSE is a measure of accuracy, to compare forecasting errors of different models for a particular data and not between datasets, as it is scale-dependent. The effect of each error on RMSE is proportional to the size of the squared error. Thus, larger prediction errors (e.g. predicted insulin injection amounts that differ substantially from the expected value) have a disproportionately large effect on RMSE. Consequently, RMSE is very sensitive to outliers. The calculation of both RMSE and r-squared prediction metrics provides clear information on the accuracy of the trained models.

With reference to block 330 in fig. 4B, the data quality control system continues by obtaining from the dataset of a query subject a plurality of query subject blood glucose measurements in a respective time course and at least one query subject insulin dose amount in a respective subset of time points. In some embodiments, the system further comprises obtaining datasets and inputting each dataset into the trained model from a plurality of query subjects.

In some embodiments, the data quality control system concurrently executes a data quality control request for the plurality of subjects. In some embodiments, the plurality of subjects comprises at least 100 subjects, at least 1,000 subjects, at least 5,000 subjects, at least 10,000 subjects, at least 50,000 subjects or at least 100,000 subjects.

With reference to block 332 in fig. 4B, the data quality control system continues by inputting the plurality of query subject blood glucose measurements into the trained model, thereby obtaining one or more query model output values for the query subject, where the one or more query output values for the query subject comprise at least one or more reconstructed insulin dose amounts for the query subject. In some embodiments, each of the subjects has a diabetic condition.

In some embodiments this diabetic condition is type 2 diabetes mellitus. In some embodiments, the medication dose guidance request is for a dose of insulin medicament, where the dose of insulin medicament is to achieve a predetermined blood glucose target range of a subject to treat diabetes mellitus.

According to the present disclosure, in some embodiments basal insulin medicament is administered in an amount to achieve a beneficial glycemic control in the subject. In some embodiments, the predetermined blood glucose target range to achieve a beneficial glycemic control is 50-180mg/dL, 60-180mg/dL, 70-180mg/dL, 80-180mg/dL, 50-200mg/dL, 60-200mg/dL, 70-200mg/dL, 80-200mg/dL, 50-250mg/dL, 60-250mg/dL, 70-250mg/dL, or 80-250mg/dL.

In some embodiments, the present disclosure insulin medicament is used to treat subjects that are at least 5 years old, at least 10 years old, at least 11 years old, at least 12 years old, at least 13 years old, at least 14 years old, at least 15 years old., at least 16 years old, at least 17 years old, at least 18 years old, at least 19 years old, or at least 20 years old.

In some embodiments, the present disclosure insulin medicament is used to treat a subject whose body mass index is no greater than 32 kg/m$^2$. In some embodiments, the present disclosure is used to treat a subject whose body mass index is no greater than 34 kg/m$^2$. In some embodiments, the present disclosure is used to treat a subject whose body mass index is no greater than 35 kg/m$^2$. In some embodiments, the present disclosure is used to treat a subject whose body mass index is no greater than 36 kg/m$^2$. In some embodiments, the present disclosure is used to treat a subject whose body mass index is no greater than 37 kg/m$^2$. In some embodiments, the present disclosure is used to treat a subject whose body mass index is no greater than 38 kg/m$^2$.

In some embodiments, the present disclosure is used to treat subjects whose body mass index is about 25 kg/m$^2$. In some embodiments, the present disclosure is used to treat subjects whose body mass index is between 20 kg/m$^2$ and 30 kg/m$^2$.

In some embodiments, the present disclosure is used to treat subjects that have been suffering from diabetes for at least 1 year, at least 5 years, at least 7 years, at least 9 years, or at least 11 years.

In some embodiments, the present disclosure achieves a baseline HbA$_{1c}$ level for subjects of no more than 7% after a number of weeks of treatment. In some embodiments, the present disclosure achieves a baseline HbA$_{1c}$ level for subjects of no more than 7% after a between 10 and 40 weeks of treatment, between 15 and 30 weeks of treatment, or between 20 and 28 weeks of treatment, or after 26 weeks of treatment. In some embodiments, the present disclosure achieves a baseline HbA$_{1c}$ level for subjects of no more than 6%, no more than 7%, or no more than 8% after a number of weeks of treatment. In some embodiments, the present disclosure achieves a baseline HbA$_{1c}$ level for subjects of no more than 6%, no more than 7%, or no more than 8% after between 10 and 40 weeks of treatment, between 15 and 30 weeks of treatment, or between 20 and 28 weeks of treatment, or after 26 weeks of treatment.

In some embodiments, the medicament comprises an insulin that is delivered by injection, such as by use of an insulin pen device 104. In some embodiments, the medicament is LysB29(Nchexadecandioyl-y-Glu) des(B30) human insulin (insulin degludec, Tresiba®).

In some embodiments, the basal insulin comprises or consists of long-acting insulin or ultra-long acting insulin.

In some embodiments, the medicament comprises a 'long-acting insulin' that also:

(a) induces in a subject a maximum deviation from mean insulin concentration (AUCF%) over a 24 hour period of $\leq \pm 20$, for example $\leq \pm 18$, $\leq \pm 17$, $\leq \pm 16$, $\leq \pm 15$, $\leq \pm 14$, $\leq \pm 13$, $\leq \pm 12$, $\leq \pm 11$, $\leq \pm 10$, $\leq \pm 9$, $\leq \pm 8$, $\leq \pm 7$, $\leq \pm 6$, $\leq \pm 5$, $\leq \pm 4$, $\leq \pm 3$, $\leq \pm 2$, $\leq \pm 1$, $\leq \pm 0.5$, $\leq \pm 0.1$.

In some embodiments, the medicament comprises a 'long-acting insulin' comprising a derivative or analogue of a naturally occurring insulin that:

(a) exhibits in physiological conditions, at least in part, the insulin receptor binding of the naturally occurring insulin, preferably, at least 0.01% of the insulin receptor binding of the naturally occurring insulin, for example, at least 0.1%, at least, 1%, at least 5%, at least 10%, at least 15%, at least 20%, at least 25% at least 50%, at least 65%, at least 75%, at least 85%, at least 95%, at least 100%, at least 110%, at least 120%, at least 130%, at least 140% or at least 150% of the insulin receptor binding of the naturally occurring insulin, and/or, at least in part, the potency of the naturally occurring insulin, preferably, at least 25% of the potency of the naturally occurring insulin, for example, at least 50%, at least 65%, at least 75%, at least 85%, at least 95%, at least 100%, at least 110%, at least 120%, at least 130%, at least 140% or at least 150% of the potency of the naturally occurring insulin; and
(b) exhibits a mean terminal half-life of at least 18 hours in physiological conditions when injected subcutaneously, for example, greater than 18 hours, at least 20 hours, greater than 20 hours, greater than 22 hours, at least 22.5 hours, or greater than 24 hours, at least 25 hours, at least 27.5 hours, at least 30 hours, at least 32.5, at least 35 hours, at least 37.5 hours, or at least 40 hours, or between 18 and 40 hours, between 20 and 40 hours, between 24 and 40 hours.

In some embodiments, the medicament comprises a 'long-acting insulin' that also:
(c) induces in a subject a maximum deviation from mean insulin concentration (AUCF%) over a 24 hour period of $\leq \pm 20$, for example, $\leq \pm 18$, $\leq \pm 17$, $\leq \pm 16$, $\leq \pm 15$, $\leq \pm 14$, $\leq \pm 13$, $\leq \pm 12$, $\leq \pm 11$, $\leq \pm 10$, $\leq \pm 9$, $\leq \pm 8$, $\leq \pm 7$, $\leq \pm 6$, $\leq \pm 5$, $\leq \pm 4$, $\leq \pm 3$, $\leq \pm 2$, $\leq \pm 1$, $\leq \pm 0.5$, $\leq \pm 0.1$.

In principle, the longer the half-life of the insulin, the more stable and evenly distributed the glucose-lowering effect over a dosing interval (i.e. time interval between injections).

In some embodiments, the medicament comprises a derivative or analogue of a naturally occurring insulin that:

(a) exhibits in physiological conditions, at least in part, the insulin receptor binding of the naturally occurring insulin, preferably, at least 0.01% of the insulin receptor binding of the naturally occurring insulin, for example, at least 0.1%, at least, 1%, at least 5%, at least 10%, at least 15%, at least 20%, at least 25% at least 50%, at least 65%, at least 75%, at least 85%, at least 95%, at least 100%, at least 110%, at least 120%, at least 130%, at least 140% or at least 150% of the insulin receptor binding of the naturally occurring insulin, and/or, at least in part, the potency of the naturally occurring insulin, preferably, at least 25% of the potency of the naturally occurring insulin, for example, at least 50%, at least 65%, at least 75%, at least 85%, at least 95%, at least 100%, at least 110%, at least 120%, at least 130%, at least 140% or at least 150% of the potency of the naturally occurring insulin; and

(b) exhibits a mean terminal half-life of at least 5 hours and less than 18 hours in physiological conditions when injected subcutaneously, for example, at least 7 hours, at least 8 hours, at least 10 hours, at least 12.5 hours, greater than 12.5 hours, at least 15 hours or at least 17.5 hours and less than 18 hours, between 5 and 17.5 hours, between 10 and 17.5 hours or between 15 and 17.5 hours.

In some embodiments, the medicament consists of a single insulin medicament having a duration of action that is between 12 and 24 hours or a mixture of insulin medicaments that collectively have a duration of action that is between 12 and 24 hours. Examples of such insulin medicaments include, but are not limited to, Insulin Degludec (developed by NOVO NORDISK under the brand name TRESIBA®), NPH (Schmid, 2007, "New options in insulin therapy," J Pediatria (Rio J). 83(Suppl 5): S146-S155), Glargine (LANTUS, March 2, 2007), Insulin Glargine injection (Dunn et al. 2003, "An Updated Review of its Use in the Management of Diabetes Mellitus" Drugs 63: p. 1743), and Determir (Plank et al., 2005, "A double-blind, randomized, dose-response study investigating the pharmacodynamic and pharmacokinetic properties of the long-acting insulin analog detemir," Diabetes Care 28:1107-1112).

Reverting to fig. 1 an example method 400 (e.g. performed at an electronic device) for providing an adjusted day dose recommendation to the subject is illustrated. In some embodiments, the method of fig. 1 is performed when the subject has not previously been provided with an adjusted day dose recommendation. In some embodiments, the method of fig. 1 is performed to provide a re-recommendation of a previously provided adjusted day dose recommendation. In some embodiments, the method of fig. 1 is performed to provide an updated adjusted day dose recommendation, subsequent to providing one or more previous adjusted day dose recommendations.

Referring to fig. 1, with the integrated system of data collection and analysis 48, autonomous timestamped insulin injections 404 and blood glucose measurements 402 of the subject are obtained. Also, in some embodiments, data from the one or more insulin pens 104 used to apply a prescribed insulin regimen to the subject is obtained 404 as a plurality of records. Each record comprises a timestamped event specifying an amount of injected insulin medicament that the subject received as part of the prescribed insulin medicament dosage regimen. In some embodiment, the glucose measurements 402 (e.g., the plurality of blood glucose measurements that comprise a blood glucose history 410) are quality assessed, and a reconstructed blood glucose history is calculated when necessary. The blood glucose history 410 or the reconstructed blood glucose history and the insulin injection history 404 are stored in non-transitory memory 202. The memory 202 also includes instructions that, when executed by the one or more processors, perform a method responsive to receiving a data quality check request. In this way, the glucose data and the injection history data are analyzed 406. The data quality check 406 reconstructs insulin injection doses based on the blood glucose history 410 and the injection history 404. These reconstructed insulin injection doses are used to produce reconstructed insulin injection does 408, along with further data regarding the subject (e.g., subject parameters 412 and/or dose guidance baseline 418), can be used to provide a medication dose guidance recommendation in some embodiments.

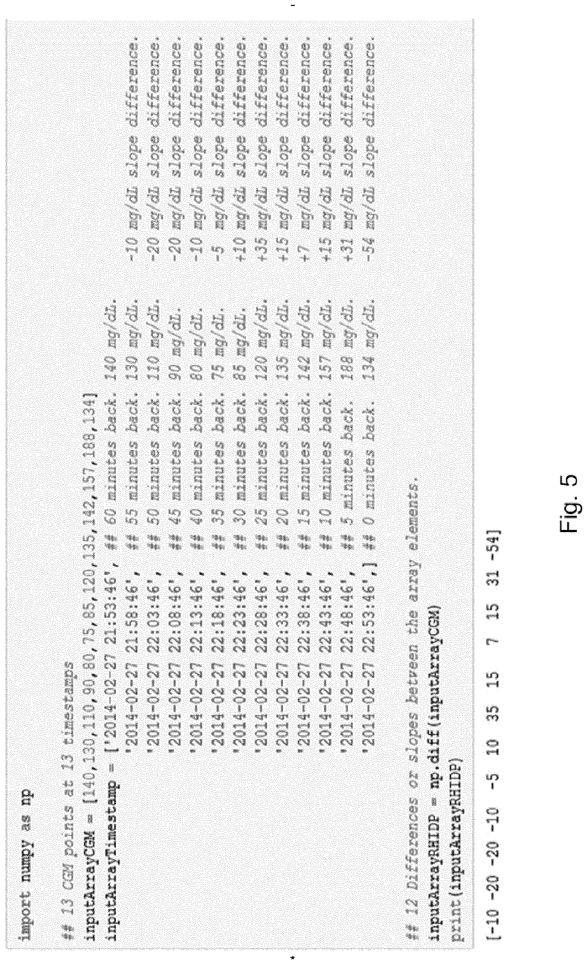

Fig. 5 illustrates an example of blood glucose measurement data being imported. Each blood glucose data point comprises a blood glucose measurement and a timestamp representing when in the time course the respective blood glucose measurement was made. In some embodiments, the blood glucose measurements 402 (here continuous glucose monitoring data) are entered into memory (e.g. "inputarrayCGM") and the differences between the blood glucose measurements are calculated, e.g. "input arrayRHIDP = np.diff (inputArrayCGM)".

In some embodiments, a set of blood glucose data points includes at least 2 blood glucose measurements, at least 4 blood glucose measurements, at least 5 blood glucose measurements, at least 7 blood glucose measurements, at least 10 blood glucose measurements, at least 13 blood glucose measurements, at least 15 blood glucose measurements, at least 20 blood glucose measurements, at least 30 blood glucose measurements, at least 50 blood glucose measurements, at least 100 blood glucose measurements, at least 250 blood glucose measurements, at least 300 blood glucose measurements, or at least 500 blood glucose measurements. In some embodiments, each time point in the respective time course for the set of blood glucose data points occurs up to 30 seconds after the immediately preceding time point, up to 1 minute after the immediately preceding time point, up to 5 minutes after the immediately preceding time point, up to 10 minutes after the immediately preceding time point, up to 30 minutes after the immediately preceding time point, up to 1 hour after the immediately preceding time point, up to 6 hours after the immediately preceding time point, up to 12 hours after the immediately preceding time point, or up to 24 hours after the immediately preceding time point. In some embodiments, the blood glucose history further includes information regarding meal, exercise, or other variables that may affect blood glucose levels.

In some embodiments, insulin dose timestamps (e.g. predefined time points) in the insulin injection history of the subject are used to determine appropriate time courses (e.g. the subsets of time points immediately preceding a predefined time period) for evaluation of blood glucose measurement data.

After accessing the data, multiple processes can be performed by the data quality control system. As shown below in the examples, multiple model types can be trained and multiple performance metrics may be calculated to provide the most accurate predicted insulin injected does.

Examples

*Example 1: Training different model types*

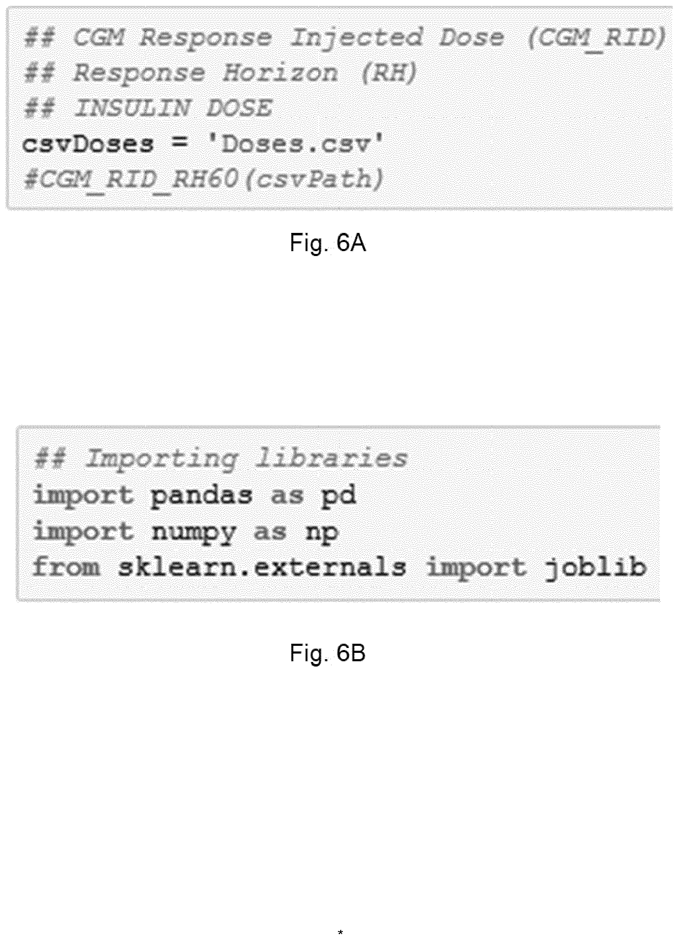

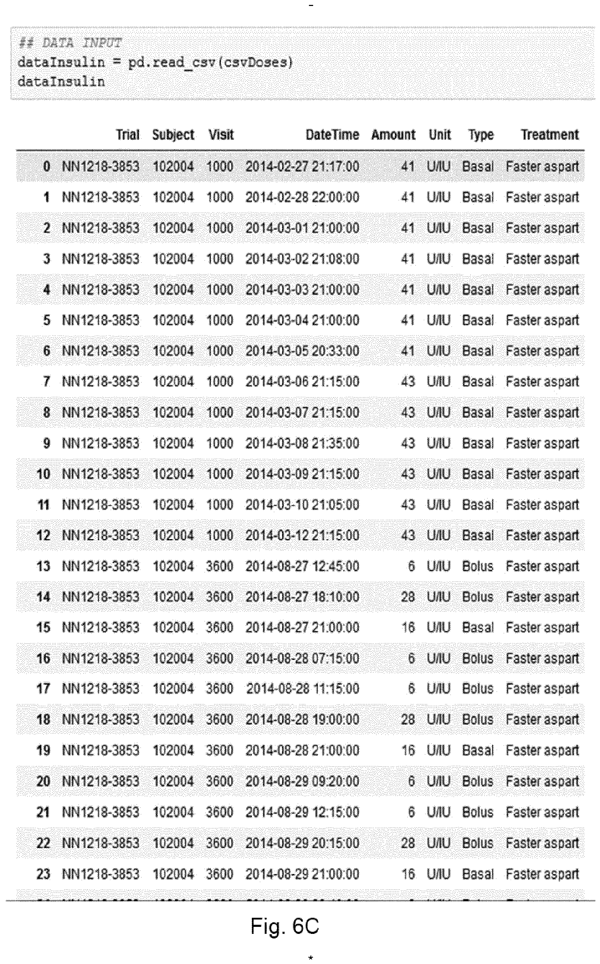

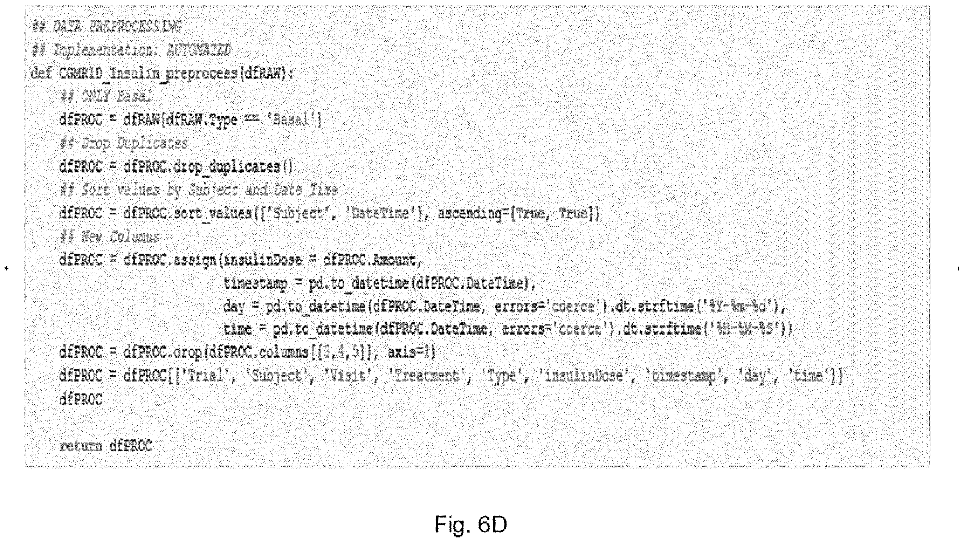

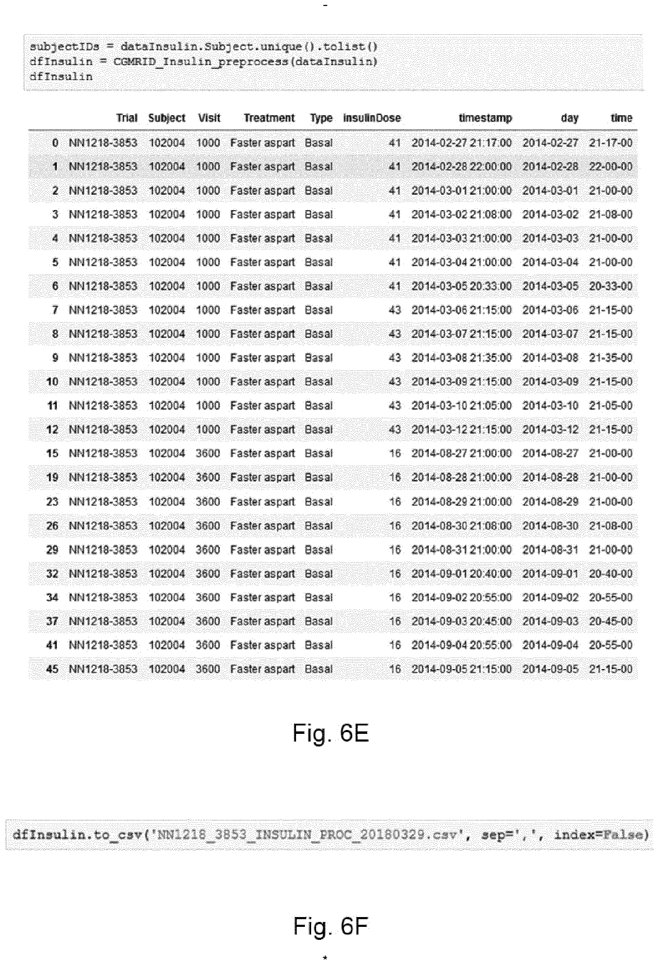

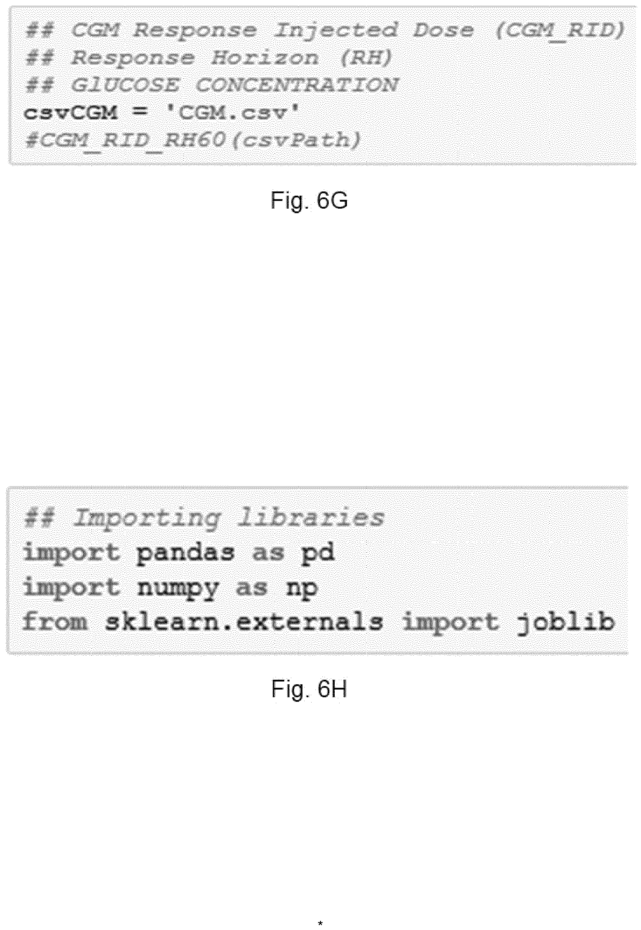

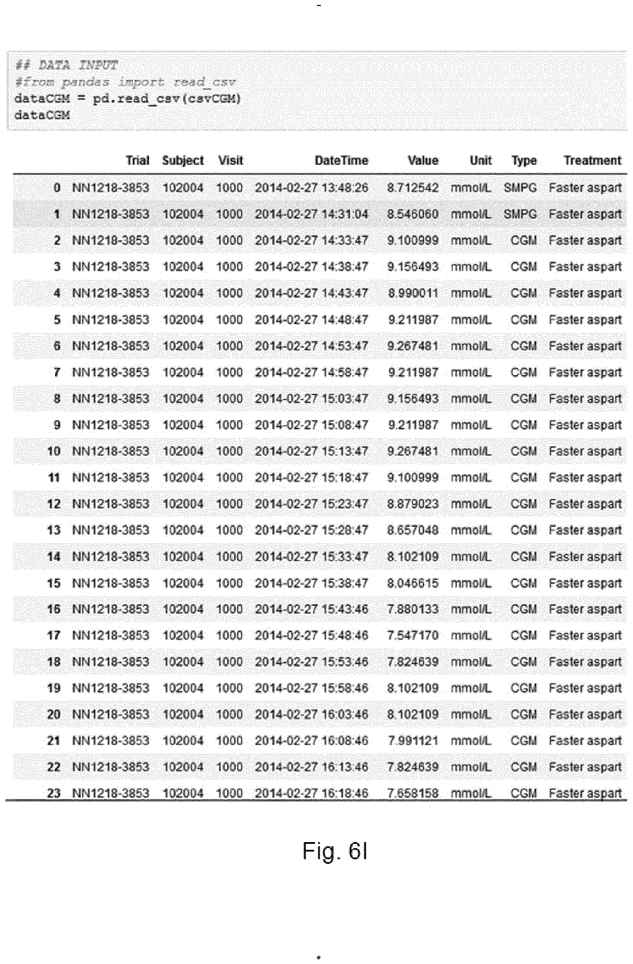

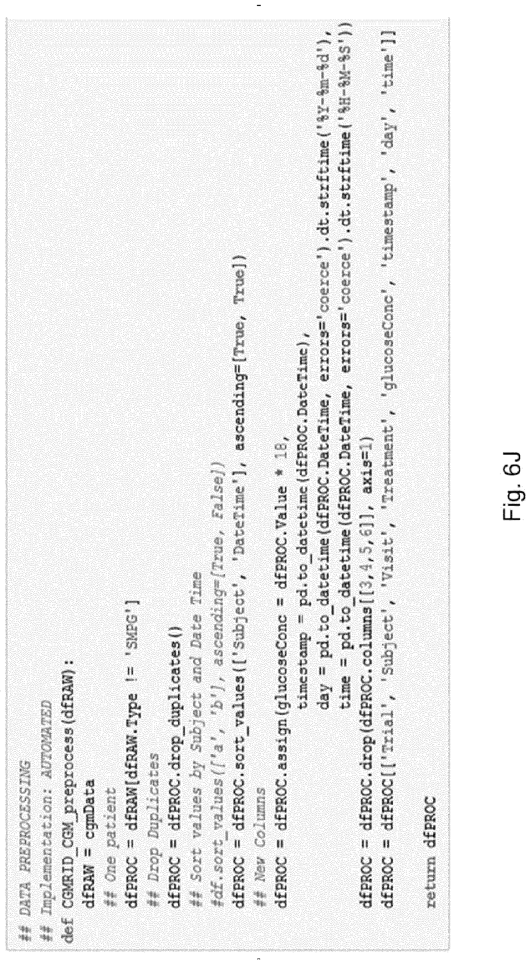

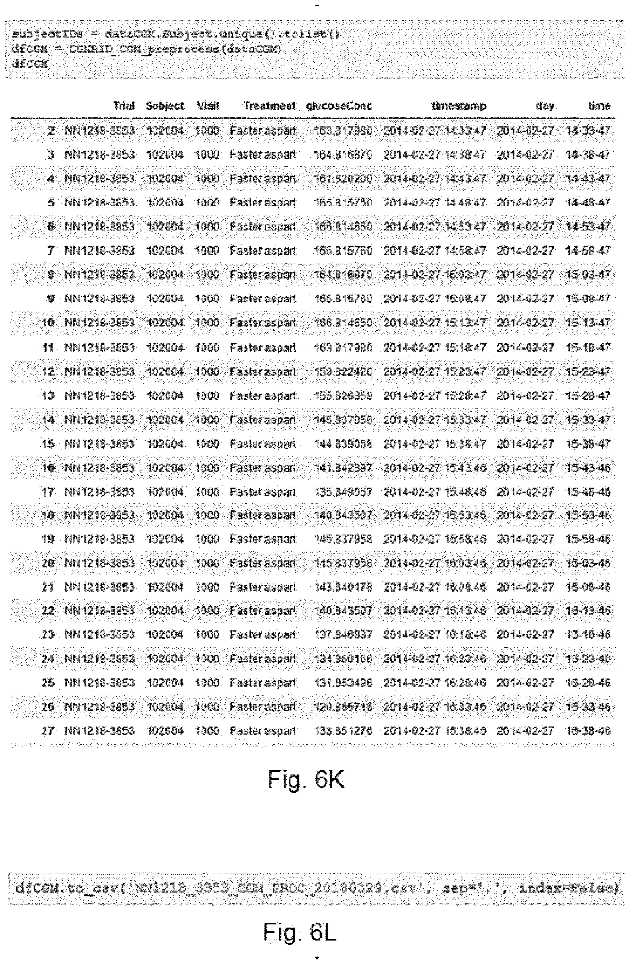

Referring to figs. 6A, 6B, 6C, 6D, 6E, 6F, 6G, 6H, 61, 6J, 6K, and 6L, examples of data preprocessing are shown. The data comprises at least a plurality of glucose measurements (e.g. a glucose history). In some embodiments, the data further comprises one or more insulin injections (e.g. an insulin injection history). In some embodiments, the data comes from one subject. In some embodiments, the data comes from a plurality of subjects, where each the data for each subject comprises at least one glucose history and at least one insulin injection history.

In some embodiments, data preprocessing is required to convert data received from glucose sensors 102 and insulin pens 104 and/or from a data collection device 200. Even before the data quality system 250 performs a data quality control check, patient data collected may be transformed (e.g. to convert the data into a more usable for and/or to remove unwanted data).

In some embodiments, the data quality system 250 itself transforms the raw data. In other embodiments, the data collection device 200 optionally transforms the raw subject data.

Referring to figs. 6A and 6G, in some embodiments, the first step is to access the raw data. In fig. 6A, an example is shown of accessing the raw data file 'Doses.csv' for the insulin dose data of a clinical trial. Referring to figs. 6B and 6H, in some embodiments, the second step is to import required software libraries to perform necessary calculations and transformations. In the examples here, the required software libraries are Python libraries. Referring to figs. 6C and 61, in some embodiments, the optional third step is to view the raw, unprocessed data (e.g. to understand how the data table looks like and what's needed to transform the raw data for further analysis). Referring to figs. 6D and 6J, in some embodiments, the fourth step is to select only the desired data. For example, in fig. 6D, only data for basal insulin events (not bolus insulin events) is selected, and in fig. 6J only data from continuous glucose monitoring (e.g. instead of self-monitoring blood glucose data). Step 4, in some embodiments, also comprises deleting duplicate records, sorting values by subject (e.g. if the data derives from more than one subject) and/or timestamp, adding new columns (e.g. 'Day' and/or 'Time') for easier data cleaning and/or exploratory data analysis, removing unneeded columns, and reorganizing the columns (e.g. into a visually understandable format to display to subjects). Referring to figs. 6E and 6K, these show examples of subject data that has been transformed into cleaned, preprocessed data. Once the data has been cleaned, it is stored for record keeping and in preparation for further data processing and analysis (see figs. 6F and 6L).

figs. 6A, 6B, 6C, 6D, 6E, and 6F collectively show an example of pre-processing subject insulin injection history data. Figs. 6G, 6H, 61, 6J, 6K, and 6L collectively show an example of preprocessing subject blood glucose history data. In some embodiments, data from only one subject is pre-processed. In some embodiments, data from a plurality of subjects is pre-processed simultaneously.

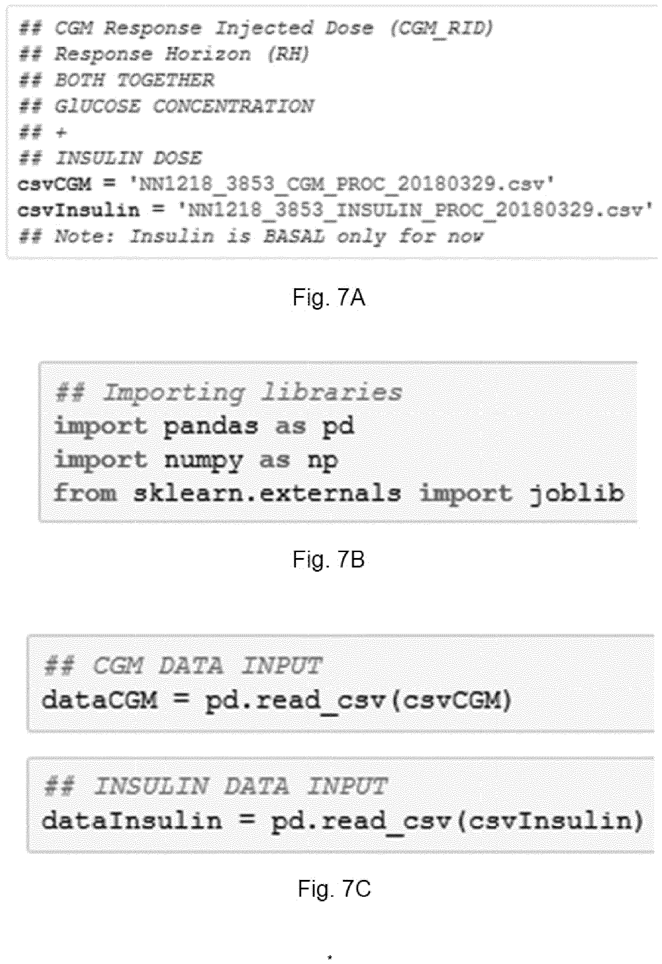

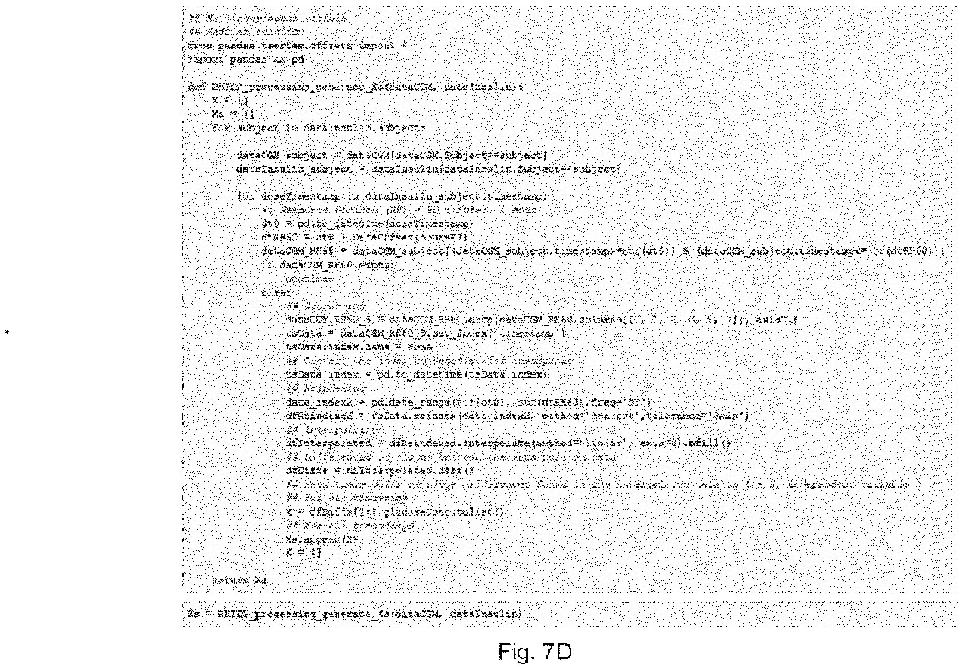

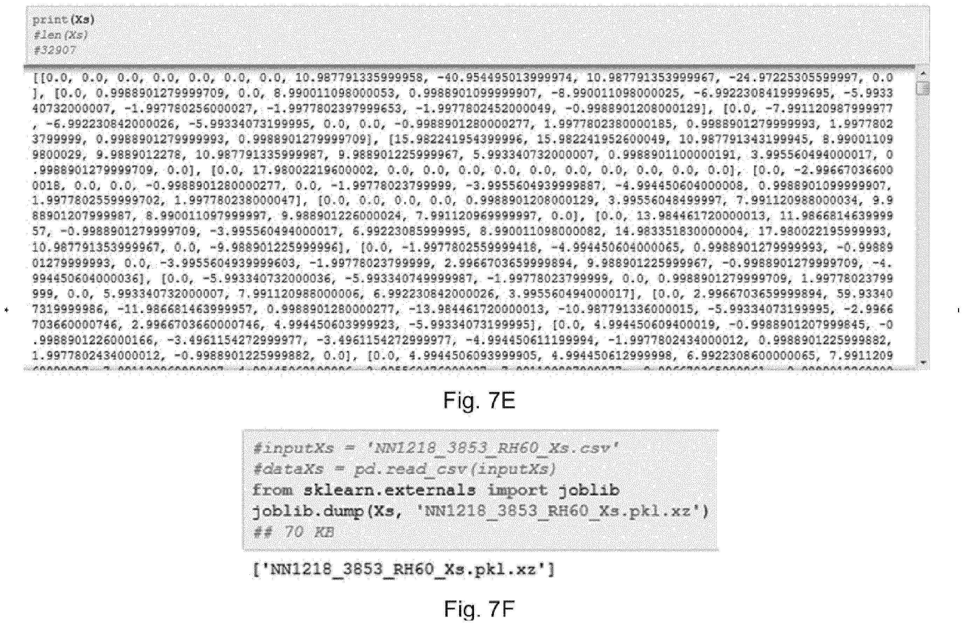

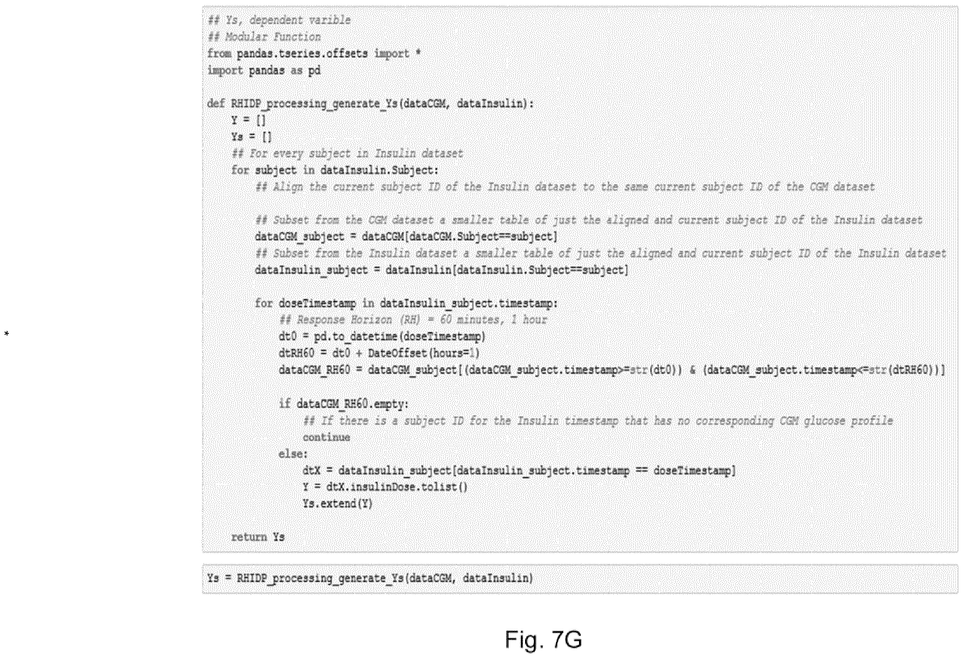

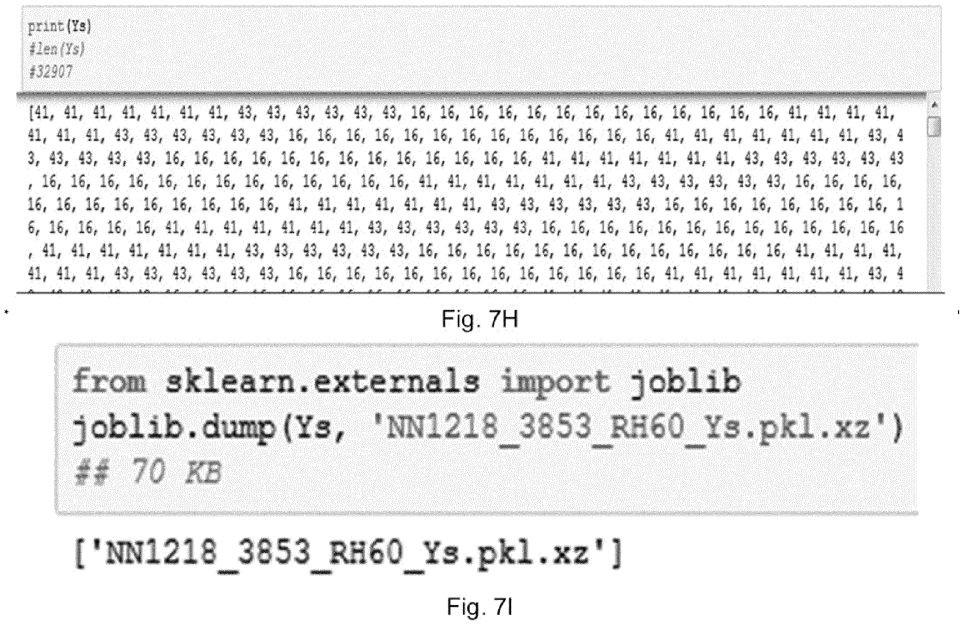

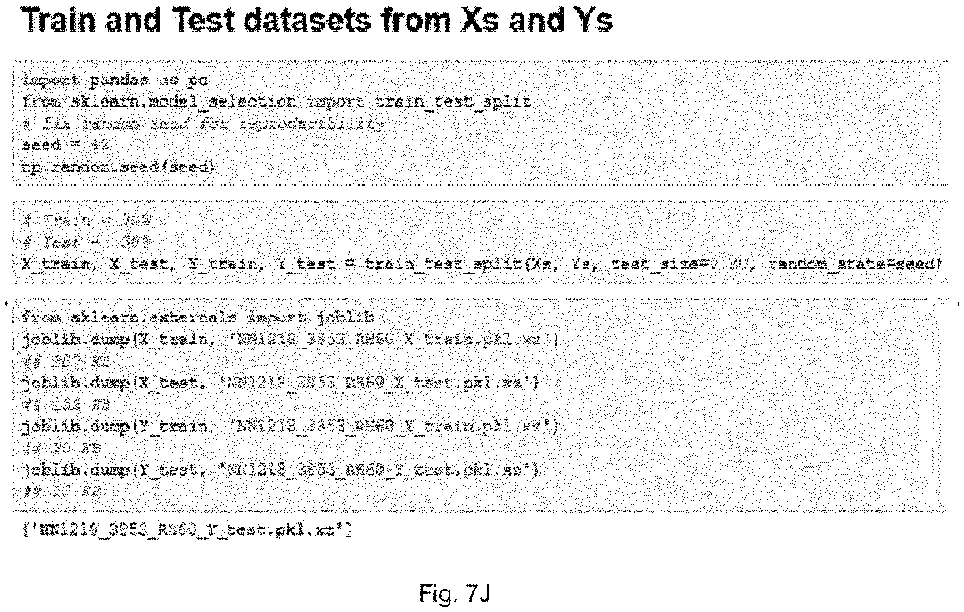

Referring to figs. 7A, 7B, 7C, 7D, 7E, 7F, 7G, 7H, 7I and 7J collectively, the figs. show an example of data processing. In some embodiments, data preprocessing comprises combining the preprocessed insulin injection history data (e.g. the Insulin dataset) and the preprocessed blood glucose history data (e.g. the CGM dataset). In some embodiments, when data from more than one subject has been preprocessed simultaneously, the data processing will organize the data by subject. In some embodiments, the data processing produces four datasets. In some embodiments, these datasets comprise at least a training dataset for the glucose measurements (e.g. X_train, the training independent variable), a training dataset for the insulin dose amounts (e.g. Y_train, the training dependent variable), a test dataset for the glucose measurements (e.g. X_test, the test independent variable), a test dataset for the insulin dose amounts (e.g. Y_test, the test dependent variable).

Referring to fig. 7A, in some embodiments the first step involves accessing the preprocessed data. The second step involves importing the necessary libraries, as shown in fig. 7B. In some embodiments, these libraries are the same as for the data preprocessing, as shown in fig. 6B. The third step of data processing involves importing the preprocessed data (e.g. reading in the preprocessed data). In some embodiments, the processing uses a double nested loop: to 1) process all the subjects of the Insulin dataset and 2) within each subject, to process through all the timestamps of the insulin dose events. During this secondary step, at each timestamp of insulin dose event of each subject, the following data transformative steps are taken:

1. Subset the CGM dataset to select data for one subject.
2. Subset the Insulin dataset to select corresponding insulin data for one subject.

3. Select 60 minutes (e.g. a retrospective horizon) of data from the initial time point of the subsetted Insulin dataset of the first insulin dosing event's timestamp from the CGM dataset.

4. For each subject, insulin dose timestamps that have no corresponding CGM or glucose data for the following 60 minutes are skipped.

5. The data table is converted into a time series table for resampling.

6. The resampling occurs by re-indexing to every 3 minutes and aligning as much as possible with the original dataset's 5 minutes CGM resolution.

7. A linear interpolation is applied to the dataset, with backfilling to fill in any gaps created by the resampling. In some embodiments, these gaps are temporal gaps in the provided data that are intervals of 1 minute, 2 minutes, 5 minutes, 10 minutes, 15 minutes, 20 minutes, or 30 minutes without recorded data.

8. Twelve values are calculated, where each value is the difference between two temporally adjacent time points in the subset CGM dataset (the CGM dataset should contain thirteen blood glucose measurements, corresponding to recordings taken every five minutes during the 60 minutes immediately following an insulin injection). In some embodiments, when the time course immediately following an insulin injection comprises a different time period (e.g. 15 minutes, 30 minutes, 45 minutes, 50 minutes, or 90 minutes) and/or when the blood glucose measurements are recorded at different time intervals (e.g. every 30 seconds, every 1 minute, every 2 minutes, every 10 minutes, every 30 minutes, or every hour), the corresponding number of CGM difference values calculated will differ.

9. These twelve float values (both positive and negatives) of CGM differences themselves are converted into an array for each subject's each insulin dose event's timestamp.

10. All such arrays of calculated CGM differences are compiled for each subject, for each appropriate time course into one array.

Thus, the data processing transforms the input data tables into an array of lists of lists. The values in this array of lists of lists correspond to the independent (e.g. X) values.

In some embodiments, the data processing proceeds to a fourth step to generate the dependent (e.g. Y) variables. This automated function applies a doubly nested for loop to 1) process all the subjects of the Insulin dataset and then 2) within each subject, to process all the timestamps of the insulin dose events. During this secondary step, at each timestamp of insulin dose event of each subject, the following data transformative steps are taken:

1. Subset the CGM dataset to select data for one subject.

2. Subset the Insulin dataset to select corresponding insulin data for one subject.

3. Select 60 minutes (e.g. a retrospective horizon) of data from the initial time point of the subsetted Insulin dataset of the first insulin dosing event's timestamp from the CGM dataset. In some embodiments, this retrospective horizon time course is between 20-40 minutes, 30-60 minutes, 40-60 minutes, 50-70 minutes, 60-80 minutes, 80-100 minutes, 100-120 minutes, 110-130 minutes, 120-140 minutes, 36 hours, or 24-48 hours.

4. For each subject, insulin dose timestamps that have no corresponding CGM or glucose data for the following 60 minutes are skipped.

5. From this finalized subset Insulin dataset, extract just the insulinDose (e.g. the insulin injection dose amount) column's value into a list. A single integer is expected here.

6. All insulin injection dose amounts for each subject, for each appropriate time course are compiled into a list of lists. Since the list from step 5 should always be just a single integer number for the amount of insulin dose units, this essentially becomes just a list of corresponding insulin dose amount units.

Thus, the data processing has transforming the input Insulin datasets into an array of list of lists of the dependent (e.g. Y) values.

At this point all of the independent and dependent values are compiled into respective data tables (with each data point retaining information regarding its respective subject and respective time course). To proceed with training models, the data must be split into training and test data tables (fig. 7J).

In this example a randomly selected 70% of the data table is designated the training set and the remaining 30% of the data table is designated the test set. In other embodiments, the training set may be 60% of the original data table, 50% of the original data table, or 40% of the original data table.

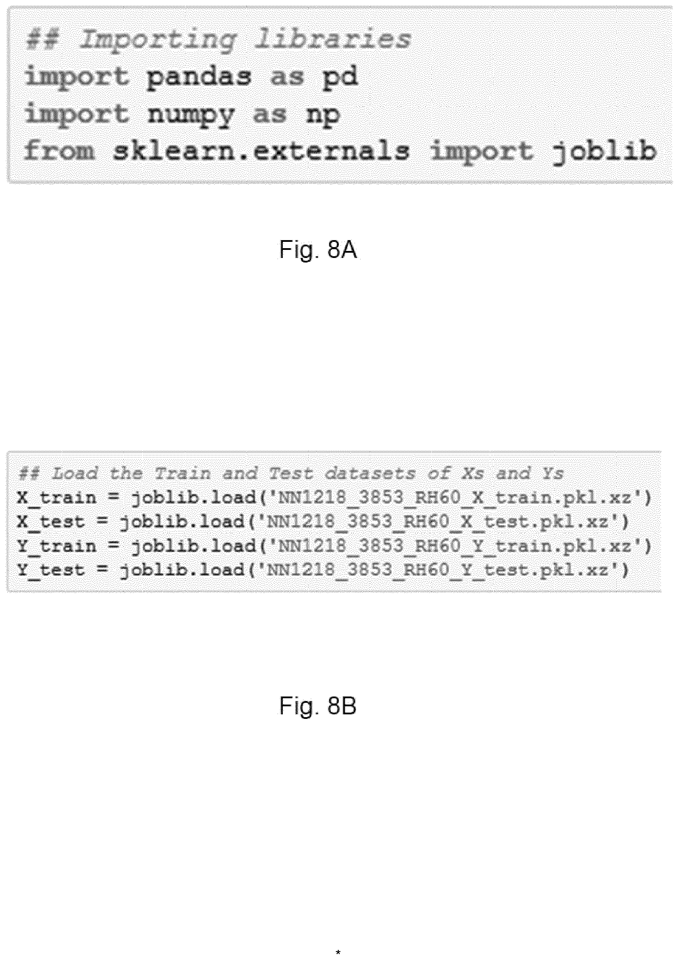

Referring to figs. 8A and 8B, the model training proceeds with data optimization. The training and test dataset described above may now be used to train and validate different machine learning models. These models, in some embodiments, are regressors, classifiers, or ensembles. The data optimization module applies both manual and automated methods of comparing, contrasting, validating to select the best performing model with optimized parameters.

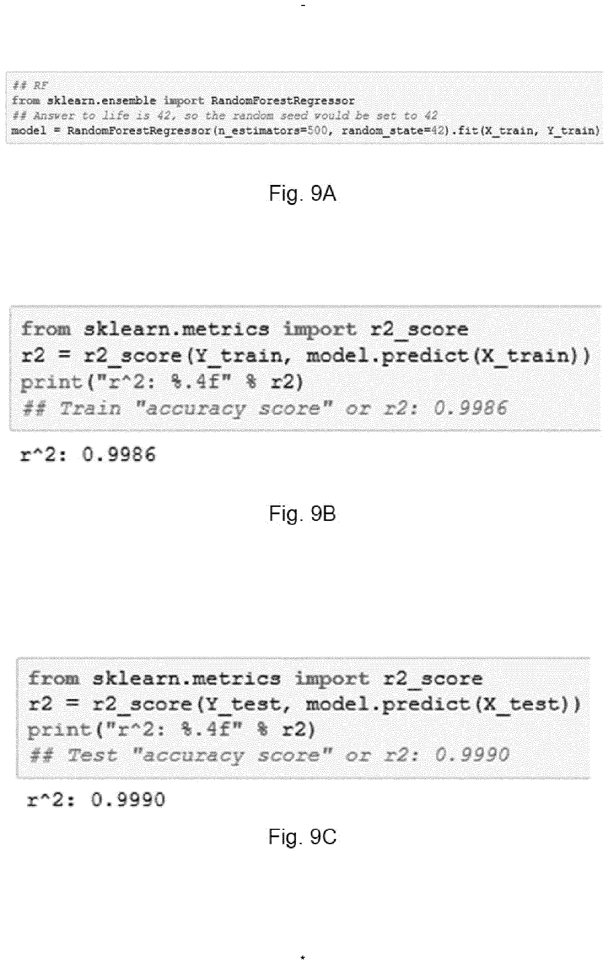

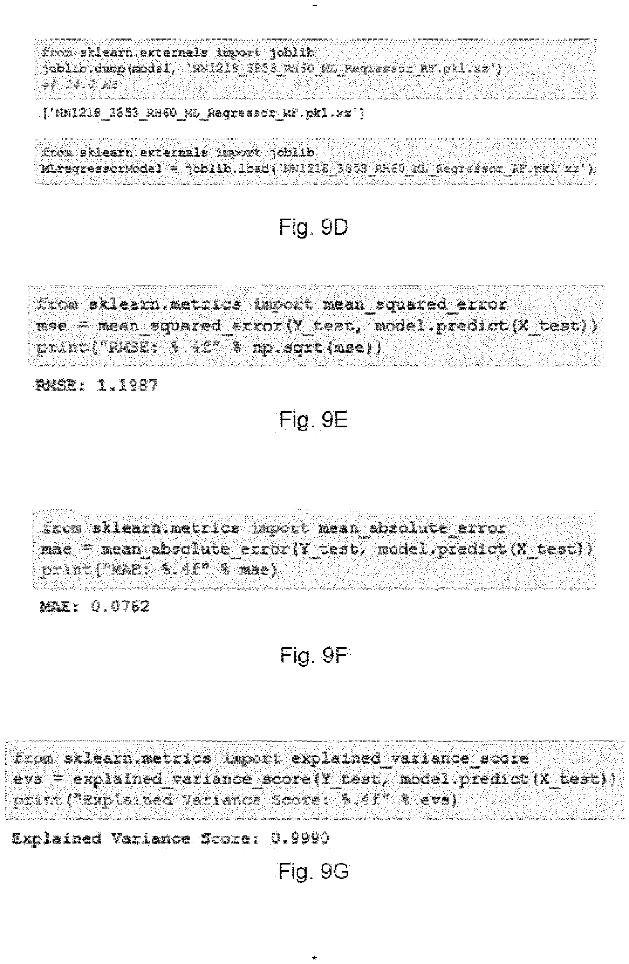

Referring to figs. 9A, 9B, 9C, 9D, 9E, 9F, and 9G, the data can be used to train a random forest regressor model. Note that setting the random_state variable to 42 is to enable reproducible models for research purposes (e.g. re-running this machine learning training step would produce a model with the same weights and topology each time). In this embodiment, 500 Decision Trees (n_estimators parameter) were selected. In other embodiments, the number of trees used for the random forest regressor may be selected from the set of 50, 100, 150, 175, 200, 250, 300, 400, and 1000 trees).

The random forest regressor model and the other models described herein, in some embodiments, require significant computing resources (e.g. ≥1MB, ≥5MB, ≥50MB, ≥500MB, ≥1GB, or ≥5GB or random access memory). In some embodiments, to facilitate processing of large amounts of data and training models, the processes described in this example may be performed on distributed, parallelized computing systems (e.g. using Hadoop, MapReduce, and Spark on Amazon Web Services and other such services) or on local servers.

After the random forest regressor has been trained, multiple performance metrics may be calculated. These metrics may include an "Accuracy Score" or r-squared metric for the training set, an "Accuracy Score" or r-squared metric, a Root Mean Square Error (RMSE) metric, and an Explained Variance Score (EVS) metric, among others.

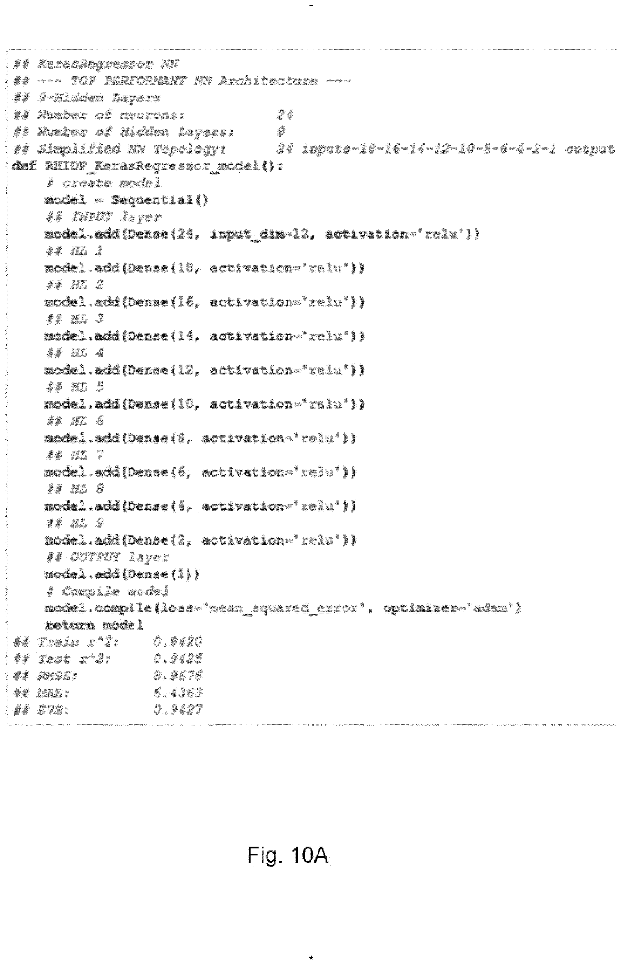

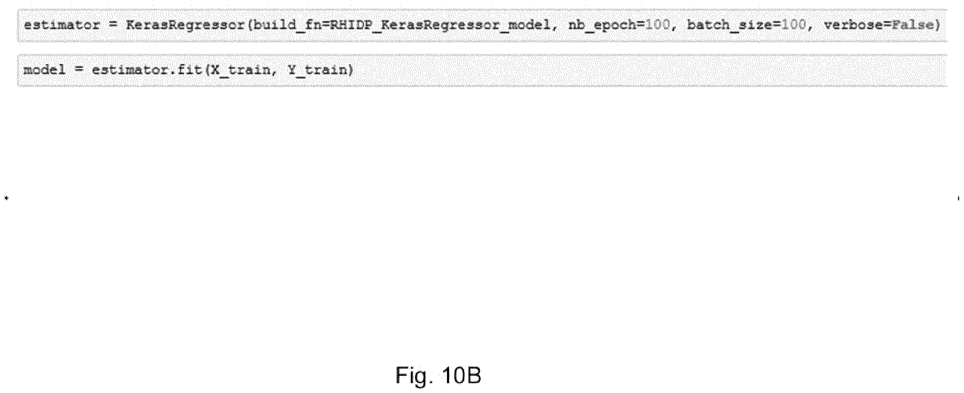

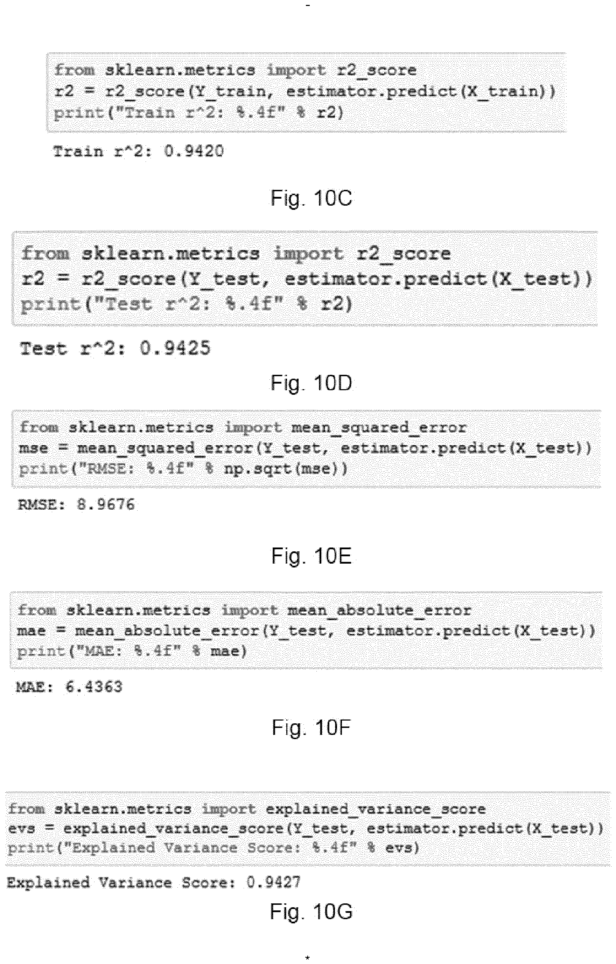

Referring to figs. 10A, 10B, 10C, 10D, 10E, 10F, and 10G, the same data can also be used to train a Keras artificial neural network regressor model. In this embodiment, the artificial neural network has nine hidden layers. In some embodiments, the artificial neural network may have up to 1 hidden layer, up to 2 hidden layers, up to 3 hidden layers, up to 4 hidden layers, up to 5 hidden layers, up to 10 hidden layers, up to 15 hidden layers, up to 20 hidden layers, up to 25 hidden layers, or up to 50 hidden layers.

After the Keras neural network regressor has been trained, multiple performance metrics may be calculated. These metrics may include an "Accuracy Score" or r-squared metric for the training set, an "Accuracy Score" or r-squared metric, a Root Mean Square Error (RMSE) metric, a Mean Absolute Error (MAE) metric, and an Explained Variance Score (EVS) metric, among others.

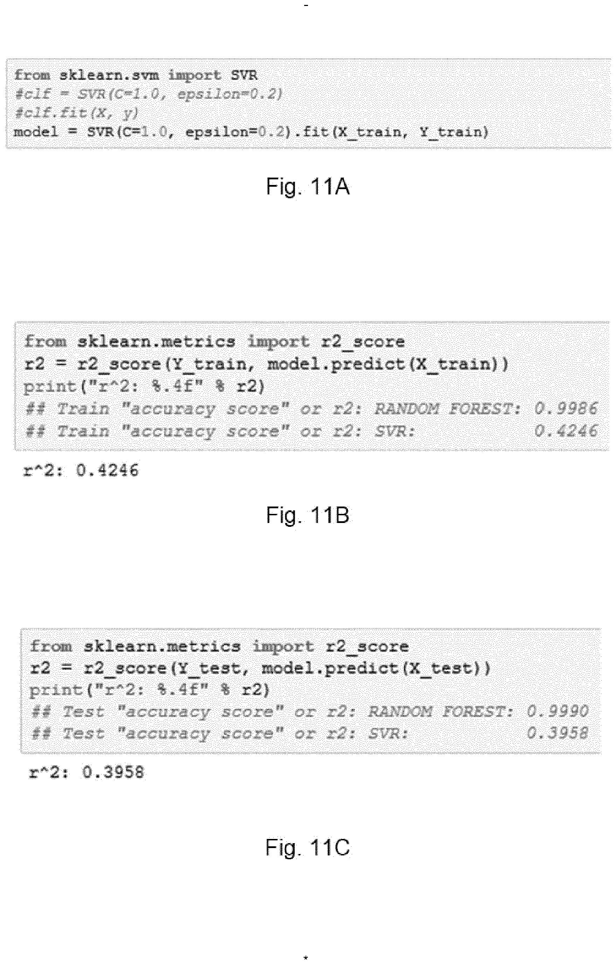

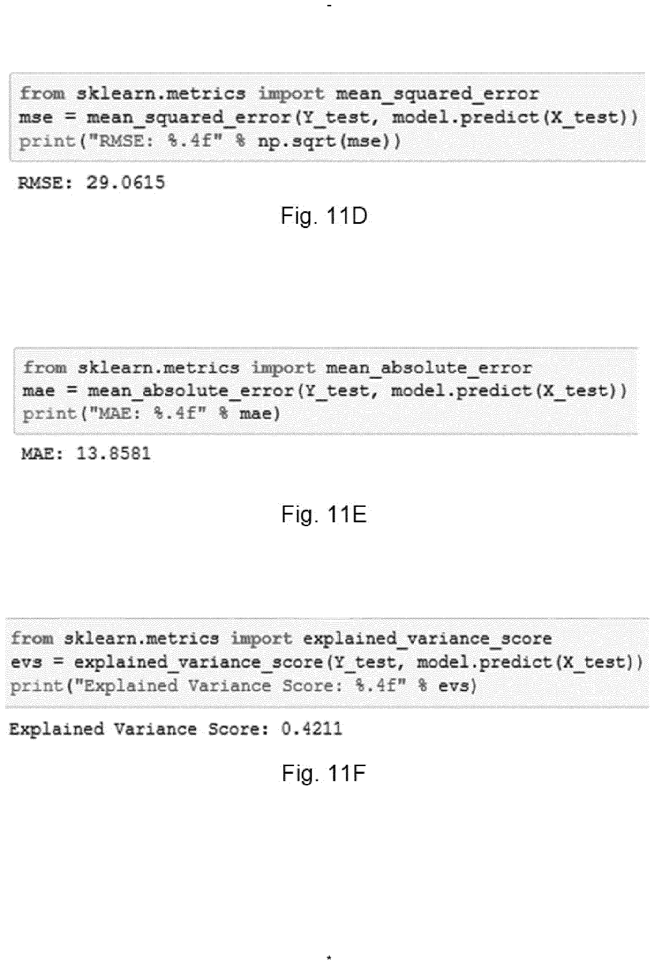

Referring to figs. 11A, 11B, 11C, 11D, 11E and 11F, the same data can also be used to train a support vector machine regressor model. After the support vector machine regressor has been trained, multiple performance metrics may be calculated. These metrics may include an "Accuracy Score" or r-squared metric for the training set, an "Accuracy Score" or r-squared metric, a Root Mean Square Error (RMSE) metric, and a Mean Absolute Error (MAE) metric, among others.

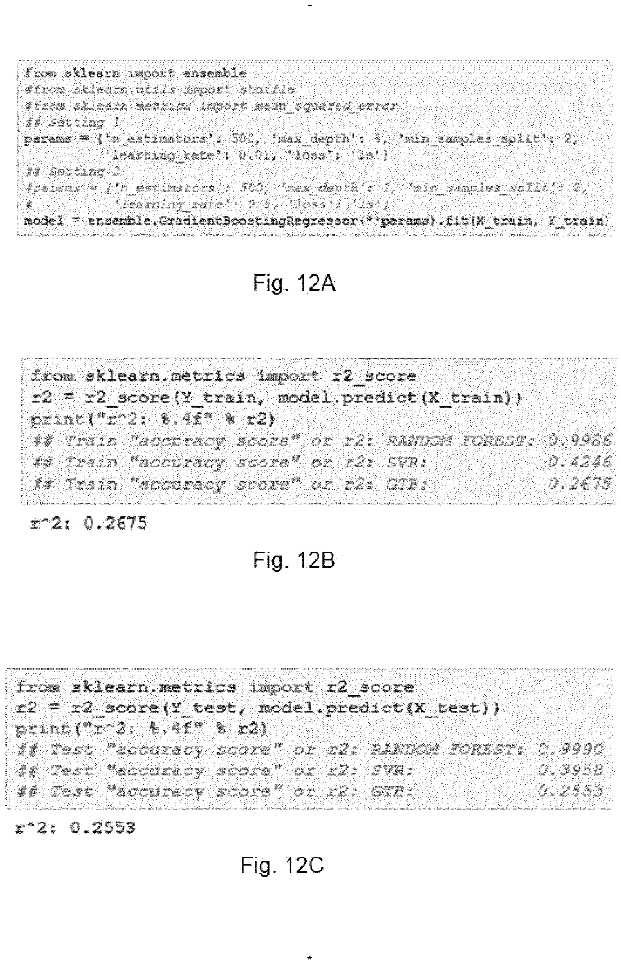

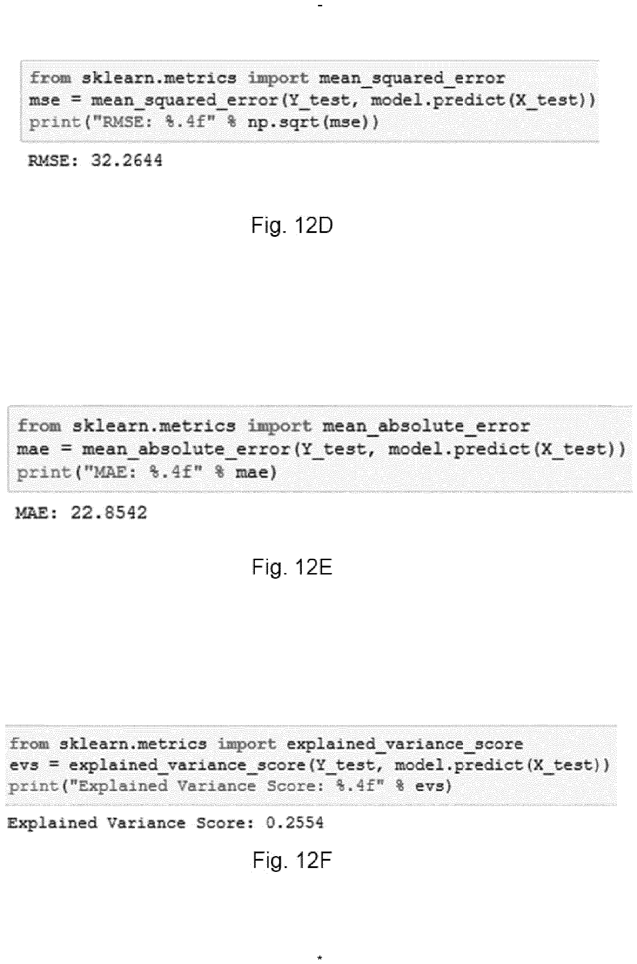

Referring to figs. 12A, 12B, 12C, 12D, 12E and 12F, the same data can also be used to train a gradient tree boosting regressor model. After the gradient tree boosting regressor has been trained, multiple performance metrics may be calculated. These metrics may include an "Accuracy Score" or r-squared metric for the training set, an "Accuracy Score" or r-squared metric, a Mean Absolute Error (MAE) metric, and an Explained Variance Score (EVS) metric, among others.

The accuracy scores and metrics of each model trained here are shown below in Table 1.

Table 1: Performance of trained regressor models.

| Model | TRAIN Accuracy | TEST Accuracy | Metric |
|---|---|---|---|
| REGRESSORS | | | |
| Random Forest (RF) 500 decision trees | 99.86% | 99.90% | RMSE = 1.19 IU |
| Keras ANN (Artificial Neural Net), 9 layers | 94.20% | 94.25% | RMSE = 8.97 IU |
| Support Vector Machine (SVM) | 42.46% | 39.58% | RMSE = 29.06 IU |
| Gradient Tree Boosting (GTB) | 26.75% | 26.75% | RMSE = 32.26 IU |

In conclusion, the models trained here achieved high accuracies and relatively small errors, particularly both the Random Forest (RF: 99.90% test accuracy performance at about 1 IU error) and the Neural Network (DCNN: 94.25% test accuracy performance at about 9 IU error) regressors.

The strength of the approach outlined here is that training multiple types of models provides the option of selecting the best performing model at any point in time. In other embodiments, other filtering functions may be incorporated to ensure that data is analyzed appropriately and that the insulin injection prediction falls within reasonable ranges (e.g. physiologically reasonable ranges).

**REFERENCES CITED AND ALTERNATIVE EMBODIMENTS**

All headings and sub-headings are used herein for convenience only and should not be construed as limiting the invention in any way.

The use of any and all examples, or exemplary language (e.g., "such as") provided herein, is intended merely to better illuminate the Z invention. The scope of the invention is defined by the appended claims. Z No language in the specification should be construed as indicating any non-claimed element as essential to the practice of the invention.

The citation of patent documents herein is done for convenience only and does not reflect any view of the validity, patentability, and/or enforceability of such patent documents.

The present invention can be implemented as a computer program product that comprises a computer program mechanism embedded in a non-transitory computer readable storage medium. For instance, the computer program product could contain the program modules shown in any combination of figs. 3A-3C and/or described in fig. 1. These program modules can be stored on a CD-ROM, DVD, magnetic disk storage product, USB key, or any other non-transitory computer readable data or program storage product.

Many modifications and variations of this invention can be made, as will be apparent to those skilled in the art. The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims.

## Claims

1. A computing system (48) for providing medication dose guidance recommendations for a query subject to treat diabetes mellitus, wherein the system comprises one or more processors and a memory, the memory comprising:

   - instructions that, when executed by the one or more processors, perform a method responsive to receiving a dose guidance request, the instructions comprising the steps of:

      a) obtaining a data structure (412) comprising query subject parameters,
      b) obtaining a first data set, comprising a plurality of glucose measurements (402) of the query subject taken over a time course and thereby establish a blood glucose history (BGH), each respective glucose measurement in the plurality of glucose measurements comprising:

         (i) a blood glucose (BG) level, and
         (ii) a corresponding blood glucose timestamp representing when in the time course the respective glucose measurement was made,

      c) obtaining a second data set, comprising an insulin injection history (404) of the query subject, wherein the injection history comprises a plurality of injections during all or a portion of the time course and, for each respective injection in the plurality of injections comprising:

         (i) a corresponding injection amount, and
         (ii) an injection timestamp representing when in the time course the respective injection occurred,

      d) obtaining a trained model for retrospective insulin dose prediction based on blood glucose response data sets having a given retrospective horizon (RH),
      e) performing data optimization (406) for the dataset of the query subject, the data optimization including inputting query subject data to the trained model and obtaining at least one reconstructed insulin dose amount (408) for the query subject, and
      f) providing a medication dose guidance recommendation (416), the recommendation being calculated based on data from the first data structure, the first data set, the second data set, and at least one reconstructed insulin dose amount for the query subject.

2. A computing system as in claim 1, wherein the step of performing data optimization comprises:

   - obtaining from the dataset of the query subject a plurality of query subject blood glucose measurements in a respective time course and at least one query subject insulin dose amount in a respective subset of time points, and
   - inputting the plurality of query subject blood glucose measurements into the trained model, thereby obtaining one or more query model output values for the query subject, wherein the one or more query output values for the query subject comprise at least one or more reconstructed insulin dose amounts for the query subject.

3. A computing system as in claim 1 or 2, wherein the method further comprises performing data preprocessing and data processing for the dataset of the query subject, wherein:

(i) the data preprocessing includes accessing the dataset of the query subject, selecting a predetermined type of insulin dose, selecting a predetermined type of blood glucose measurement, and reorganizing the plurality of query subject blood glucose measurements and the at least one subject insulin dose amounts into a plurality of preprocessed query subject blood glucose measurements and/or an at least one preprocessed query subject insulin dose amount, and

(ii) the data processing includes combining the preprocessed query subject blood glucose histories and the preprocessed query subject insulin dose histories into a query subject data table, and designating independent variables and dependent variables in the query subject data table, and

(iii) the data optimization includes applying the query subject data table to the trained model and obtaining at least one reconstructed insulin dose amount for the query subject.

4. A computing system as in any one of claims 1-3, wherein the step of obtaining a trained model comprises:

- obtaining a training set that comprises a plurality of reference entities, wherein the plurality of reference entities comprises a plurality of reference insulin dose histories and a plurality of reference blood glucose histories, wherein each reference insulin dose history is paired with a reference blood glucose history to form a reference entity in the plurality of reference entities,

- for each respective reference entity in the plurality of reference entities, obtaining (i) a blood glucose measurement for each time point in a respective time course from the corresponding blood glucose history and (ii) an insulin dose amount for each time point in a respective subset of time points in the respective time course from the corresponding reference insulin dose history, wherein:

- each respective reference entity in the plurality of reference entities provides a reference plurality of blood glucose measurements for the respective time course, wherein the reference plurality of blood glucose measurements is of a predetermined type of blood glucose measurements,

- each respective reference entity in the plurality of reference entities provides one or more reference insulin dose amounts in the respective subset of time points, wherein the one or more reference insulin dose amounts are of a predetermined type of insulin dose,

- obtaining a test set that comprises a plurality of test entities, wherein the plurality of test entities comprises a plurality of test insulin dose histories and a plurality of test blood glucose histories, wherein each test insulin dose history is paired with a test blood glucose history to form a test entity in the plurality of test entities, wherein each respective test blood glucose history provides a plurality of blood glucose measurements, and wherein each respective test insulin dose history provides one or more test insulin dose amounts, wherein the one or more test insulin dose amounts are of a predetermined type of insulin dose,

- training a model using the reference plurality of blood glucose measurements for the respective time course and the at least one reference insulin dose amount across the training set, thereby obtaining a trained model,

- inputting the test plurality of blood glucose histories into the trained model thereby obtaining one or more trained model output values for the test set, wherein the one or more trained output values comprises one or more reconstructed insulin dose amounts for the test set, and

- evaluating the trained model based on the trained model output values for the test set.

5. A computing system as in claim 4, wherein the training and test set data are resampled for injecting into a machine learning engine.

6. A computing system as in claim 4 or 5, wherein:

- the plurality of reference insulin dose histories and the plurality of reference blood glucose histories are from a plurality of training subjects, or

- the plurality of reference insulin dose histories and the plurality of reference blood glucose histories are from a single training subject.

7. A computing system as in any of claims 4-6, wherein:

- each time point in the subset of time points immediately precedes a predefined time period in the time course, and

- the predefined period in the time course is selected from the set of 20-40 minutes, 30-60 minutes, 40-60 minutes, 50-70 minutes, 60-80 minutes, 80-100 minutes, 100-120 minutes and 120-180 minutes.

8. A computing system as in claim 1, wherein the respective reference insulin dose history comprises a plurality of insulin doses administered to the corresponding reference entity during all or a portion of the time course and, for each respective insulin dose in the plurality of insulin doses, a corresponding insulin dose amount and an insulin dose timestamp representing when in the time course the respective insulin dose occurred, and wherein the insulin dose timestamp is in the subset of time points.

9. A computing system as in any of claims 4-7, wherein the method further comprises providing one or more model performance metrics, wherein the one or more model performance metrics includes a root mean square error or an accuracy score.

10. A computing system as in any of claims 1-9, wherein the data optimization further includes handling missing data, wherein one or more temporal gaps in the subject data table are interpolated by resampling the subject data table by a predefined time interval.

11. A computing system as in any one of claims 1-10, wherein the predetermined type of blood glucose measurements is (i) continuous glucose monitoring data, or (ii) self-monitoring of blood glucose data.

12. A computing system as in any of claims 1-11, wherein the predetermined type of insulin dose consists of (i) one or more bolus insulin dose events, or (ii) one or more basal insulin dose events.

13. A computing system as in any of claims 1-11 adapted for providing a long-acting or ultra-long-acting insulin adjustment day dose recommendation (ADDR) for the query subject, wherein the data structure (412) comprises: (i) a glucose upper target range level of the subject, (ii) a glucose lower target range level of the subject, and (iii) a current dose guidance baseline.

14. A computing system as in any of claims 1-11 adapted for providing a short-acting insulin adjustment dose recommendation for the query subject, wherein the data structure (412) comprises meal data and/or exercise data.

**Patentansprüche**

1. Computersystem (48) zur Bereitstellung von Empfehlungen zu einer Medikamentendosisanleitung für ein Abfrageobjekt zur Behandlung von Diabetes mellitus, wobei das System einen oder mehrere Prozessoren und einen Speicher umfasst, der Speicher umfassend:

- Anweisungen, die bei Ausführung durch den einen oder die mehreren Prozessoren ein Verfahren in Reaktion auf den Empfang einer Dosisanleitungsanforderung durchführen, die Anweisungen die folgenden Schritte umfassend:

a) Erhalten einer Datenstruktur (412) umfassend Parameter des Abfrageobjekts,
b) Erhalten eines ersten Datensatzes umfassend eine Vielzahl von über einen Zeitverlauf aufgenommenen Glukosemessungen (402) des Abfrageobjekts und dadurch Erstellen einer Blutzuckerhistorie (BGH), wobei jede jeweilige Glukosemessung in der Vielzahl von Glukosemessungen umfasst:

(i) einen Blutzuckerspiegel (BG-Spiegel), und
(ii) einen entsprechenden Blutzucker-Zeitstempel, der angibt, wann im Zeitverlauf die jeweilige Glukosemessung vorgenommen wurde,

c) Erhalten eines zweiten Datensatzes umfassend eine Insulininjektionshistorie (404) des Abfrageobjekts, wobei die Injektionshistorie eine Vielzahl von Injektionen während des gesamten oder eines Teils des Zeitverlaufs umfasst und für jede entsprechende Injektion in der Vielzahl von Injektionen umfasst:

(i) eine entsprechende Injektionsmenge, und
(ii) einen Zeitstempel der Injektion, der angibt, wann im Zeitverlauf die jeweilige Injektion erfolgte,

d) Erhalten eines trainierten Modells für die retrospektive Insulindosis-Vorhersage basierend auf Blutzuckerantwort-Datensätzen mit einem bestimmten retrospektiven Horizont (RH),
e) Durchführen einer Datenoptimierung (406) für den Datensatz des Abfrageobjekts, wobei die Datenoptimierung das Eingeben von Daten des Abfrageobjekts in das trainierte Modell und das Erhalten zumindest einer rekonstruierten Insulindosismenge (408) für das Abfrageobjekt umfasst, und

f) Bereitstellen einer Empfehlung für die Medikamentendosisanleitung (416), wobei die Empfehlung basierend auf Daten aus der ersten Datenstruktur, dem ersten Datensatz, dem zweiten Datensatz und zumindest einer rekonstruierten Insulindosismenge für das Abfrageobjekt berechnet wird.

2. Computersystem nach Anspruch 1, wobei der Schritt der Durchführung der Datenoptimierung umfasst:

- Erhalten einer Vielzahl von Blutzuckermessungen des Abfrageobjekts in einem jeweiligen Zeitverlauf und zumindest einer Insulindosismenge des Abfrageobjekts in einer jeweiligen Untergruppe von Zeitpunkten aus dem Datensatz des Abfrageobjekts, und

- Eingeben der Vielzahl von Blutzuckermessungen des Abfrageobjekts in das trainierte Modell, wodurch ein oder mehrere Abfragemodell-Ausgabewerte für das Abfrageobjekt erhalten werden, wobei der eine oder die mehreren Abfrage-Ausgabewerte für das Abfrageobjekt zumindest eine oder mehrere rekonstruierte Insulindosismengen für das Abfrageobjekt umfassen.

3. Computersystem nach Anspruch 1 oder 2, wobei das Verfahren ferner die Durchführung von Datenvorverarbeitung und Datenverarbeitung für den Datensatz des Abfrageobjekts umfasst, wobei:

(i) die Datenvorverarbeitung den Zugriff auf den Datensatz des Abfrageobjekts, die Auswahl einer vorbestimmten Art der Insulindosis, die Auswahl einer vorbestimmten Art der Blutzuckermessung und die Reorganisation der Vielzahl von Blutzuckermessungen des Abfrageobjekts und der zumindest einen Insulindosismenge des Abfrageobjekts in eine Vielzahl von vorverarbeiteten Blutzuckermessungen des Abfrageobjekts und/oder zumindest eine vorverarbeitete Insulindosismenge des Abfrageobjekts beinhaltet, und

(ii) die Datenverarbeitung das Kombinieren der vorverarbeiteten Blutzuckerhistorien des Abfrageobjekts und der vorverarbeiteten Insulindosishistorien des Abfrageobjekts in einer Datentabelle des Abfrageobjekts und das Bestimmen von unabhängigen Variablen und abhängigen Variablen in der Datentabelle des Abfrageobjekts beinhaltet, und

(iii) die Datenoptimierung die Anwendung der Datentabelle des Abfrageobjekts auf das trainierte Modell und den Erhalt zumindest einer rekonstruierten Insulindosismenge für das Abfrageobjekt beinhaltet.

4. Computersystem nach einem der Ansprüche 1 bis 3, wobei der Schritt des Erhaltens eines trainierten Modells umfasst:

- Erhalten eines Trainingssatzes, der eine Vielzahl von Referenzeinheiten umfasst, wobei die Vielzahl von Referenzeinheiten eine Vielzahl von Referenzinsulindosishistorien und eine Vielzahl von Referenzblutglukosehistorien umfasst, wobei jede Referenzinsulindosishistorie mit einer Referenzblutglukosehistorie gepaart ist, um eine Referenzeinheit in der Vielzahl von Referenzeinheiten zu bilden,

- für jede jeweilige Referenzeinheit in der Vielzahl von Referenzeinheiten das Erhalten (i) einer Blutzuckermessung für jeden Zeitpunkt in einem jeweiligen Zeitverlauf aus der entsprechenden Blutzuckerhistorie und (ii) einer Insulindosismenge für jeden Zeitpunkt in einer jeweiligen Untergruppe von Zeitpunkten in dem jeweiligen Zeitverlauf aus der entsprechenden Referenzinsulindosishistorie, wobei:

- jede entsprechende Referenzeinheit in der Vielzahl von Referenzeinheiten eine Referenzvielzahl von Blutzuckermessungen für den entsprechenden Zeitverlauf bereitstellt, wobei die Referenzvielzahl von Blutzuckermessungen von einer vorbestimmten Art von Blutzuckermessungen ist,

- jede entsprechende Referenzeinheit in der Vielzahl von Referenzeinheiten peine oder mehrere Referenzinsulindosismengen in der entsprechenden Untergruppe von Zeitpunkten bereitstellt, wobei die eine oder mehreren Referenzinsulindosismengen von einer vorbestimmten Art von Insulindosis sind,

- Erhalten eines Testsatzes, der eine Vielzahl von Testeinheiten umfasst, wobei die Vielzahl von Testeinheiten eine Vielzahl von Testinsulindosishistorien und eine Vielzahl von Testblutzuckerhistorien umfasst, wobei jede Testinsulindosishistorie mit einer Testblutzuckerhistorie gepaart ist, um eine Testeinheit in der Vielzahl von Testeinheiten zu bilden, wobei jede jeweilige Testblutzuckerhistorie eine Vielzahl von Blutzuckermessungen bereitstellt, und wobei jede jeweilige Testinsulindosishistorie eine oder mehrere Testinsulindosismengen bereitstellt, wobei die eine oder mehreren Testinsulindosismengen von einer vorbestimmten Art von Insulindosis sind,

- Trainieren eines Modells unter Verwendung der Vielzahl von Referenzblutzuckermessungen für den jeweiligen Zeitverlauf und der zumindest einen Referenzinsulindosismenge in dem Trainingssatz, wodurch ein trainiertes Modell erhalten wird,
- Eingabe der Testvielzahl von Blutzuckerhistorien in das trainierte Modell, wodurch ein oder mehrere trainierte Modellausgabewerte für den Testsatz erhalten werden, wobei der eine oder die mehreren trainierten Ausgabewerte eine oder mehrere rekonstruierte Insulindosismengen für den Testsatz umfassen, und
- Evaluieren des trainierten Modells basierend auf den Ausgabewerten des trainierten Modells für den Testsatz.

5. Computersystem nach Anspruch 4, wobei die Daten des Trainings- und des Testsatzes für die Injektion erneut in eine Maschinenlernmaschine eingelesen werden.

6. Computersystem nach Anspruch 4 oder 5, wobei:

   - die Vielzahl von Referenzinsulindosishistorien und die Vielzahl von Referenzblutzuckerhistorien von einer Vielzahl von Trainingspersonen stammen, oder
   - die Vielzahl der Referenzinsulindosishistorien und die Vielzahl der Referenzblutzuckerhistorien von einer einzigen Trainingsperson stammen.

7. Computersystem nach einem der Ansprüche 4 bis 6, wobei:

   - jeder Zeitpunkt in der Untergruppe der Zeitpunkte unmittelbar vor einem vordefinierten Zeitraum im Zeitverlauf liegt, und
   - der vordefinierte Zeitraum in dem Zeitverlauf ausgewählt ist aus der Gruppe von 20-40 Minuten, 30-60 Minuten, 40-60 Minuten, 50-70 Minuten, 60-80 Minuten, 80-100 Minuten, 100-120 Minuten und 120-180 Minuten.

8. Computersystem nach Anspruch 1, wobei die jeweilige Referenzinsulindosishistorie eine Vielzahl von Insulindosen umfasst, die der entsprechenden Referenzeinheit während des gesamten oder eines Teils des Zeitverlaufs verabreicht wurden, und für jede jeweilige Insulindosis in der Vielzahl von Insulindosen eine entsprechende Insulindosismenge und einen Insulindosis-Zeitstempel, der angibt, wann in dem Zeitverlauf die jeweilige Insulindosis aufgetreten ist, und wobei der Insulindosis-Zeitstempel in der Untergruppe der Zeitpunkte liegt.

9. Computersystem nach einem der Ansprüche 4 bis 7, wobei das Verfahren ferner das Bereitstellen einer oder mehrerer Modellleistungsmetriken umfasst, wobei die eine oder die mehreren Modellleistungsmetriken einen mittleren quadratischen Fehler oder eine Genauigkeitsbewertung umfassen.

10. Computersystem nach einem der Ansprüche 1 bis 9, wobei die Datenoptimierung ferner die Behandlung fehlender Daten beinhaltet, wobei eine oder mehrere zeitliche Lücken in der Datentabelle des Objekts durch das erneute Einlesen der Datentabelle des Objekts mit einem vordefinierten Zeitintervall interpoliert werden.

11. Computersystem nach einem der Ansprüche 1 bis 10, wobei die vorbestimmte Art der Blutzuckermessungen (i) Daten zur kontinuierlichen Glukoseüberwachung oder (ii) Daten zur Selbstüberwachung des Blutzuckers ist.

12. Computersystem nach einem der Ansprüche 1 bis 11, wobei die vorbestimmte Art der Insulindosis aus (i) einem oder mehreren Bolusinsulindosisereignissen oder (ii) einem oder mehreren Basalinsulindosisereignissen besteht.

13. Computersystem nach einem der Ansprüche 1 bis 11, ausgelegt für die Bereitstellung einer Tagesdosisempfehlung für langwirkendes oder ultralangwirkendes Insulin (ADDR) für das Abfrageobjekt, wobei die Datenstruktur (412) umfasst: (i) einen oberen Glukose-Zielbereichsspiegel des Objekts, (ii) einen unteren Glukose-Zielbereichsspiegel des Objekts und (iii) eine aktuelle Dosisanleitungs-Basislinie.

14. Computersystem nach einem der Ansprüche 1 bis 11, ausgelegt für die Bereitstellung einer Empfehlung zur Anpassung der Dosis für kurzwirksames Insulin für das Abfrageobjekt, wobei die Datenstruktur (412) Mahlzeitendaten und/oder Bewegungsdaten umfasst.

**Revendications**

1. Système informatisé (48) permettant de fournir des recommandations indicatives de dose de médicament relatives à un sujet de requête en vue de traiter le diabète sucré, ledit système comprenant un ou plusieurs processeurs et une mémoire, la mémoire comprenant :

- des instructions qui, lorsqu'elles sont exécutées par les un ou plusieurs processeurs, réalisent un procédé en réponse à la réception d'une demande d'indication de dose, les instructions comprenant les étapes consistant à :

a) obtenir une structure de données (412) comprenant des paramètres du sujet de la requête,
b) obtenir un premier ensemble de données, comprenant une pluralité de mesures de glucose (402) du sujet de la requête prises sur un créneau temporel, et ainsi établir un historique de glycémie (HDG), chaque mesure de glucose respective de la pluralité de mesures de glucose comprenant :

(i) un taux de glycémie, et
(ii) une estampille temporelle de glycémie correspondante représentant le moment auquel, dans le créneau temporel, la mesure de glucose respective a été effectuée,

c) obtenir un deuxième ensemble de données, comprenant un historique d'injections d'insuline (404) du sujet de la requête, ledit historique d'injections comprenant une pluralité d'injections effectuées pendant la totalité ou une partie du créneau temporel et comprenant, pour chaque injection respective de la pluralité d'injections :

(i) une quantité d'injection correspondante, et
(ii) une estampille temporelle d'injection représentant le moment auquel, dans le créneau temporel, l'injection respective s'est produite,

d) obtenir un modèle entraîné pour une prédiction de dose d'insuline rétrospective fondée sur des ensembles de données de réponse glycémique ayant un horizon rétrospectif (HR) donné,
e) réaliser une optimisation de données (406) relativement à l'ensemble de données du sujet de la requête, l'optimisation des données comprenant la fourniture en entrée de données du sujet de la requête au modèle entraîné et l'obtention d'au moins une quantité de dose d'insuline reconstruite (408) propre au sujet de la requête, et
f) fournir une recommandation indicative de dose de médicament (416), la recommandation étant calculée compte tenu de données provenant de la première structure de données, du premier ensemble de données, du deuxième ensemble de données, et d'au moins une quantité de dose d'insuline reconstruite propre au sujet de la requête.

2. Système informatisé selon la revendication 1, dans lequel l'étape consistant à réaliser une optimisation de données comprend :

- l'obtention, à partir de l'ensemble de données du sujet de la requête, d'une pluralité de mesures de glycémie du sujet de la requête dans un créneau temporel respectif, et d'au moins une quantité de dose d'insuline du sujet de la requête dans un sous-ensemble respectif de points temporels, et
- la fourniture en entrée de la pluralité de mesures de glycémie du sujet de la requête dans le modèle entraîné, pour ainsi obtenir une ou plusieurs valeurs de sortie de modèle de requête propres au sujet de la requête, lesdites une ou plusieurs valeurs de sortie de requête propres au sujet de la requête comprenant au moins une ou plusieurs quantités de dose d'insuline reconstruites propres au sujet de la requête.

3. Système informatisé selon la revendication 1 ou 2, dans lequel le procédé comprend en outre la réalisation d'un prétraitement de données et d'un traitement de données relativement à l'ensemble de données du sujet de la requête, étant entendu que :

(i) le prétraitement des données comprend l'accès à l'ensemble de données du sujet de la requête, la sélection d'un type prédéterminé de dose d'insuline, la sélection d'un type prédéterminé de mesure de glycémie, et la réorganisation de la pluralité de mesures de glycémie du sujet de la requête et de l'au moins une quantité de dose d'insuline du sujet en une pluralité de mesures de glycémie du sujet de la requête prétraitées et/ou au moins une quantité de dose d'insuline du sujet de la requête prétraitée, et
(ii) le traitement des données comprend la combinaison des historiques de glycémie du sujet de la requête prétraités et des historiques de doses d'insuline du sujet de la requête prétraités en une table de données du sujet de la requête, et la désignation de variables indépendantes et de variables dépendantes dans la table de données du sujet de la requête, et

(iii) l'optimisation des données comprend l'application de la table de données du sujet de la requête au modèle entraîné et l'obtention d'au moins une quantité de dose d'insuline reconstruite propre au sujet de la requête.

4. Système informatisé selon l'une quelconque des revendications 1 à 3, dans lequel l'étape consistant à obtenir un modèle entraîné comprend :

- l'obtention d'un ensemble d'entraînement qui comprend une pluralité d'entités de référence, ladite pluralité d'entités de référence comprenant une pluralité d'historiques de doses d'insuline de référence et une pluralité d'historiques de glycémie de référence, chaque historique de doses d'insuline de référence étant apparié à un historique de glycémie de référence pour former une entité de référence de la pluralité d'entités de référence,
- pour chaque entité de référence respective de la pluralité d'entités de référence, l'obtention (i) d'une mesure de glycémie pour chaque point temporel dans un créneau temporel respectif à partir de l'historique de glycémie correspondant et (ii) d'une quantité de dose d'insuline pour chaque point temporel d'un sous-ensemble respectif de points temporels dans le créneau temporel respectif à partir de l'historique de doses d'insuline de référence correspondant, étant entendu que :

- chaque entité de référence respective de la pluralité d'entités de référence fournit une pluralité de mesures de glycémie de référence pour le créneau temporel respectif, ladite pluralité de mesures de glycémie de référence étant d'un type prédéterminé de mesures de glycémie,
- chaque entité de référence respective de la pluralité d'entités de référence fournit une ou plusieurs quantités de dose d'insuline de référence dans le sous-ensemble respectif de points temporels, lesdites une ou plusieurs quantités de dose d'insuline de référence étant d'un type prédéterminé de dose d'insuline,

- l'obtention d'un ensemble d'essai qui comprend une pluralité d'entités d'essai, ladite pluralité d'entités d'essai comprenant une pluralité d'historiques de doses d'insuline d'essai et une pluralité d'historiques de glycémie d'essai, chaque historique de doses d'insuline d'essai étant apparié à un historique de glycémie d'essai pour former une entité d'essai de la pluralité d'entités d'essai, chaque historique de glycémie d'essai respectif fournissant une pluralité de mesures de glycémie, et chaque historique de doses d'insuline d'essai respectif fournissant une ou plusieurs quantités de dose d'insuline d'essai, lesdites une ou plusieurs quantités de dose d'insuline d'essai étant d'un type prédéterminé de dose d'insuline,
- l'entraînement d'un modèle à l'aide de la pluralité de mesures de glycémie de référence pour le créneau temporel respectif et de l'au moins une quantité de dose d'insuline de référence sur tout l'ensemble d'entraînement, pour ainsi obtenir un modèle entraîné,
- la fourniture en entrée de la pluralité d'historiques de glycémie d'essai dans le modèle entraîné, pour ainsi obtenir une ou plusieurs valeurs de sortie de modèle entraîné relatives à l'ensemble d'essai, lesdites une ou plusieurs valeurs de sortie entraînées comprenant une ou plusieurs quantités de dose d'insuline reconstruites relatives à l'ensemble d'essai, et
- l'évaluation du modèle entraîné, compte tenu des valeurs de sortie de modèle entraîné relatives à l'ensemble d'essai.

5. Système informatisé selon la revendication 4, dans lequel les données d'entraînement et d'ensemble d'essai sont rééchantillonnées pour être injectées dans un moteur d'apprentissage automatique.

6. Système informatisé selon la revendication 4 ou 5, dans lequel :

- la pluralité d'historiques de doses d'insuline de référence et la pluralité d'historiques de glycémie de référence proviennent d'une pluralité de sujets d'entraînement, ou
- la pluralité d'historiques de doses d'insuline de référence et la pluralité d'historiques de glycémie de référence proviennent d'un seul sujet d'entraînement.

7. Système informatisé selon l'une quelconque des revendications 4 à 6, dans lequel :

- chaque point temporel du sous-ensemble de points temporels précède immédiatement une période prédéfinie dans le créneau temporel, et
- la période prédéfinie dans le créneau temporel est sélectionnée dans le groupe comprenant 20 à 40 minutes, 30 à 60 minutes, 40 à 60 minutes, 50 à 70 minutes, 60 à 80 minutes, 80 à 100 minutes, 100 à 120 minutes et 120 à 180 minutes.

**8.** Système informatisé selon la revendication 1, dans lequel l'historique de doses d'insuline de référence respectif comprend une pluralité de doses d'insuline administrées à l'entité de référence correspondante pendant la totalité ou une partie du créneau temporel et, pour chaque dose d'insuline respective de la pluralité de doses d'insuline, une quantité de dose d'insuline correspondante et une estampille temporelle de dose d'insuline représentant le moment auquel, dans le créneau temporel respectif, la dose d'insuline respective s'est produite, ladite estampille temporelle de dose d'insuline se trouvant dans le sous-ensemble de points temporels.

**9.** Système informatisé selon l'une quelconque des revendications 4 à 7, dans lequel le procédé comprend en outre la fourniture d'une ou plusieurs mesures de performance de modèle, lesdites une ou plusieurs mesures de performance de modèle comprenant une erreur quadratique moyenne ou un score d'exactitude.

**10.** Système informatisé selon l'une quelconque des revendications 1 à 9, dans lequel l'optimisation des données comprend en outre la manipulation de données manquantes, un ou plusieurs battements temporels dans la table de données du sujet étant interpolés d'un intervalle de temps prédéfini par rééchantillonnage de la table de données du sujet.

**11.** Système informatisé selon l'une quelconque des revendications 1 à 10, dans lequel le type prédéterminé de mesures de glycémie est celui de (i) données de surveillance continue de la glycémie, ou (ii) données d'autosurveillance de la glycémie.

**12.** Système informatisé selon l'une quelconque des revendications 1 à 11, dans lequel le type prédéterminé de dose d'insuline consiste en (i) un ou plusieurs événements de dose d'insuline en bolus, ou (ii) un ou plusieurs événements de dose d'insuline basale.

**13.** Système informatisé selon l'une quelconque des revendications 1 à 11, adapté à fournir une recommandation de dose journalière d'ajustement (RDJA) d'insuline à action longue ou ultra-longue relativement au sujet de la requête, et dans lequel la structure de données (412) comprend : (i) un niveau de plage cible supérieur de glucose du sujet, (ii) un niveau de plage cible inférieur de glucose du sujet, et (iii) une ligne de base indicative de dose actuelle.

**14.** Système informatisé selon l'une quelconque des revendications 1 à 11, adapté à fournir une recommandation de dose d'ajustement d'insuline à action brève relativement au sujet de la requête, et dans lequel la structure de données (412) comprend des données de repas et/ou des données d'activité physique.

# Fig. 1

# Fig. 2A

```
┌──────────────┐                    ╭───╮
│    Input     │                    │ A │
└──────┬───────┘                    ╰─▲─╯
       │                              │
       ▼                              │
┌──────────────┐              ┌───────┴──────┐
│     1.1      │              │     1.8      │
└──────┬───────┘              └───────▲──────┘
       │                              │
       ▼                              │
┌──────────────┐              ┌───────┴──────┐
│     1.2      │              │     1.7      │
└──────┬───────┘              └───────▲──────┘
       │                              │
       ▼                              │
┌──────────────┐              ┌───────┴──────┐
│     1.3      │              │     1.6      │
└──────┬───────┘              └───────▲──────┘
       │                              │
       ▼                              │
┌──────────────┐              ┌───────┴──────┐
│     1.4      │─────────────▶│     1.5      │
└──────────────┘              └──────────────┘
```

*

# Fig. 2B

# Fig. 2C

# Fig. 2D

## Fig. 3A

# Fig. 3B

250

Persistent memory 220

Controller 218

CPU 202

Network interface 210

Internet/ Network 106

214

212

222

224

226

User interface

Display

Input/ Keyboard

| Operating System | 204 |
| Training Set 1 | 230-1 |
| Reference Entity 1 | 240-1 |
| Blood Glucose History 1 | 242-1 |
| Glucose measurement 1-1 | 244-1-1 |
| Glucose measurement timestamp 1-1 | 246-1-1 |
| ⋮ | |
| Glucose measurement 1-A | 244-1-A |
| Glucose measurement timestamp 1-A | 246-1-A |
| Insulin Injection History 1 | 260-1 |
| Insulin dose amount 1-1 | 262-1-1 |
| Insulin dose event timestamp 1-1 | 264-1-1 |
| ⋮ | |
| Insulin dose amount 1-B | 262-1-B |
| Insulin dose event timestamp 1-B | 264-1-B |
| Reference Identity C | 240-C |
| Test Set 1 | 270-1 |
| Test Entity 1 | 272-1 |
| Blood Glucose History D | 242-D |
| Glucose measurement D-1 | 244-D-1 |
| Glucose measurement timestamp D-1 | 246-D-1 |
| ⋮ | |
| Glucose measurement D-Z | 244-D-Z |
| Glucose measurement timestamp D-Z | 246-D-Z |
| Insulin Injection History E | 260-E |
| Insulin dose amount E-1 | 262-E-1 |
| Insulin dose event timestamp E-1 | 264-E-1 |
| ⋮ | |
| Insulin dose amount E-Y | 262-E-Y |
| Insulin dose event timestamp E-Y | 264-E-Y |
| Test Entity X | 272-X |
| Models | 282 |
| Trained Model 1 | 284-1 |
| ⋮ | |
| Trained Model F | 284-F |
| Ⓐ | |
| ⋮ | |

# Fig. 3C

| | |
|---|---|
| Subject Query Entity 1 | 290-1 |
| Blood Glucose History G | 242-G |
| Glucose measurement G-1 | 244-G-1 |
| Glucose measurement timestamp G-1 | 246-G-1 |
| ⋮ | |
| Glucose measurement G-W | 244-G-W |
| Glucose measurement timestamp G-W | 246-G-W |
| Insulin Injection History H | 260-H |
| Insulin dose amount H-1 | 262-H-1 |
| Insulin dose event timestamp H-1 | 264-H-1 |
| ⋮ | |
| Insulin dose amount H-V | 262-H-V |
| Insulin dose event timestamp H-V | 264-H-V |
| ⋮ | |
| Subject Query Entity J | 290-J |

214

-

(302) A computing system for inferentially performing data quality control of a dataset from a subject, where the computer system comprises one or more processors and a memory, the memory comprising instructions that, when executed by the one or more processors, perform a method

(304) Obtain a training set that comprises a plurality of reference entities, where the plurality of reference entities comprises a plurality of reference insulin dose histories and a plurality of reference blood glucose histories, where each reference insulin dose history is paired with a reference blood glucose history to form a reference entity in the plurality of reference entities

(306) The plurality of reference insulin dose histories and the plurality of reference blood glucose histories are from a plurality of training subjects

(308) The plurality of reference insulin dose histories and the plurality of reference blood glucose histories are from a single training subject

(310) For each respective reference entity in the plurality of reference entities, obtain (i) a blood glucose measurement for each time point in a respective time course from the corresponding blood glucose history and (ii) an insulin dose amount for each time point in a respective subset of time points in the respective time course from the corresponding reference insulin dose history, where each respective reference entity in the plurality of reference entities provides a reference plurality of blood glucose measurements for the respective time course, where the reference plurality of blood glucose measurements are of a predetermined type of blood glucose measurements, and each respective reference entity in the plurality of reference entities provides one or more reference insulin dose amounts in the respective subset of time points, where the one or more reference insulin dose amounts are of a predetermined type of insulin dose

(312) The predetermined type of blood glucose measurements is continuous glucose monitoring data

(314) The predetermined type of blood glucose measurements is self-monitoring of blood glucose data

(316) The predetermined type of insulin dose consists of one or more bolus insulin dose events

(318) The predetermined type of insulin dose consists of one or more basal insulin dose events

# Fig. 4A

(A)

# Fig. 4B

(A)

(320) Obtain a test set that comprises a plurality of test entities, where the plurality of test entities comprises a plurality of test insulin dose histories and a plurality of test blood glucose histories, where each test insulin dose history is paired with a test blood glucose history to form a test entity in the plurality of test entities, where each respective test blood glucose history provides a plurality of blood glucose measurements, and where each respective test insulin dose history provides one or more test insulin dose amounts, where the one or more test insulin dose amounts are of a predetermined type of insulin dose

(322) Train a model using the reference plurality of blood glucose measurements for the respective time course and the at least one reference insulin dose amount across the training set, thereby obtaining a trained model

(324) The model is a regression model selected from the group consisting of a linear regression model, a nonlinear regression model, a support vector machine, a random forest, a Keras artificial neural network, and a gradient tree boosting model

(326) Input the test plurality of blood glucose histories into the trained model thereby obtaining one or more trained model output values for the test set, where the one or more trained output values comprise one or more reconstructed insulin dose amounts for the test set

(328) The model also provides one or more model performance metrics, wherein the one or more model performance metrics includes a root mean square error or an accuracy score

(330) Obtain from the dataset of a query subject a plurality of query subject blood glucose measurements in a respective time course and at least one query subject insulin dose amount in a respective subset of time points

(332) Input the plurality of query subject blood glucose measurements into the trained model, thereby obtaining one or more query model output values for the query subject, wherein the one or more query output values for the query subject comprise at least one or more reconstructed insulin dose amounts for the query subject

*

```
import numpy as np

## 13 CGM points at 13 timestamps
inputArrayCGM = [140,130,110,90,80,75,85,120,135,142,157,188,134]
inputArrayTimestamp = ['2014-02-27 21:53:46',  ## 60 minutes back. 140 mg/dL.
                       '2014-02-27 21:58:46',  ## 55 minutes back. 130 mg/dL.    -10 mg/dL slope difference.
                       '2014-02-27 22:03:46',  ## 50 minutes back. 110 mg/dL.    -20 mg/dL slope difference.
                       '2014-02-27 22:08:46',  ## 45 minutes back. 90 mg/dL.     -20 mg/dL slope difference.
                       '2014-02-27 22:13:46',  ## 40 minutes back. 80 mg/dL.     -10 mg/dL slope difference.
                       '2014-02-27 22:18:46',  ## 35 minutes back. 75 mg/dL.     -5  mg/dL slope difference.
                       '2014-02-27 22:23:46',  ## 30 minutes back. 85 mg/dL.     +10 mg/dL slope difference.
                       '2014-02-27 22:28:46',  ## 25 minutes back. 120 mg/dL.    +35 mg/dL slope difference.
                       '2014-02-27 22:33:46',  ## 20 minutes back. 135 mg/dL.    +15 mg/dL slope difference.
                       '2014-02-27 22:38:46',  ## 15 minutes back. 142 mg/dL.    +7  mg/dL slope difference.
                       '2014-02-27 22:43:46',  ## 10 minutes back. 157 mg/dL.    +15 mg/dL slope difference.
                       '2014-02-27 22:48:46',  ## 5 minutes back.  188 mg/dL.    +31 mg/dL slope difference.
                       '2014-02-27 22:53:46',] ## 0 minutes back.  134 mg/dL.    -54 mg/dL slope difference.

## 12 Differences or slopes between the array elements.
inputArrayRHIDP = np.diff(inputArrayCGM)
print(inputArrayRHIDP)
```

[-10 -20 -20 -10  -5  10  35  15   7  15  31 -54]

Fig. 5

```
## CGM Response Injected Dose (CGM_RID)
## Response Horizon (RH)
## INSULIN DOSE
csvDoses = 'Doses.csv'
#CGM_RID_RH60(csvPath)
```

Fig. 6A

```
## Importing libraries
import pandas as pd
import numpy as np
from sklearn.externals import joblib
```

Fig. 6B

*

-

```
## DATA INPUT
dataInsulin = pd.read_csv(csvDoses)
dataInsulin
```

|    | Trial | Subject | Visit | DateTime | Amount | Unit | Type | Treatment |
|----|-------|---------|-------|----------|--------|------|------|-----------|
| 0 | NN1218-3853 | 102004 | 1000 | 2014-02-27 21:17:00 | 41 | U/IU | Basal | Faster aspart |
| 1 | NN1218-3853 | 102004 | 1000 | 2014-02-28 22:00:00 | 41 | U/IU | Basal | Faster aspart |
| 2 | NN1218-3853 | 102004 | 1000 | 2014-03-01 21:00:00 | 41 | U/IU | Basal | Faster aspart |
| 3 | NN1218-3853 | 102004 | 1000 | 2014-03-02 21:08:00 | 41 | U/IU | Basal | Faster aspart |
| 4 | NN1218-3853 | 102004 | 1000 | 2014-03-03 21:00:00 | 41 | U/IU | Basal | Faster aspart |
| 5 | NN1218-3853 | 102004 | 1000 | 2014-03-04 21:00:00 | 41 | U/IU | Basal | Faster aspart |
| 6 | NN1218-3853 | 102004 | 1000 | 2014-03-05 20:33:00 | 41 | U/IU | Basal | Faster aspart |
| 7 | NN1218-3853 | 102004 | 1000 | 2014-03-06 21:15:00 | 43 | U/IU | Basal | Faster aspart |
| 8 | NN1218-3853 | 102004 | 1000 | 2014-03-07 21:15:00 | 43 | U/IU | Basal | Faster aspart |
| 9 | NN1218-3853 | 102004 | 1000 | 2014-03-08 21:35:00 | 43 | U/IU | Basal | Faster aspart |
| 10 | NN1218-3853 | 102004 | 1000 | 2014-03-09 21:15:00 | 43 | U/IU | Basal | Faster aspart |
| 11 | NN1218-3853 | 102004 | 1000 | 2014-03-10 21:05:00 | 43 | U/IU | Basal | Faster aspart |
| 12 | NN1218-3853 | 102004 | 1000 | 2014-03-12 21:15:00 | 43 | U/IU | Basal | Faster aspart |
| 13 | NN1218-3853 | 102004 | 3600 | 2014-08-27 12:45:00 | 6 | U/IU | Bolus | Faster aspart |
| 14 | NN1218-3853 | 102004 | 3600 | 2014-08-27 18:10:00 | 28 | U/IU | Bolus | Faster aspart |
| 15 | NN1218-3853 | 102004 | 3600 | 2014-08-27 21:00:00 | 16 | U/IU | Basal | Faster aspart |
| 16 | NN1218-3853 | 102004 | 3600 | 2014-08-28 07:15:00 | 6 | U/IU | Bolus | Faster aspart |
| 17 | NN1218-3853 | 102004 | 3600 | 2014-08-28 11:15:00 | 6 | U/IU | Bolus | Faster aspart |
| 18 | NN1218-3853 | 102004 | 3600 | 2014-08-28 19:00:00 | 28 | U/IU | Bolus | Faster aspart |
| 19 | NN1218-3853 | 102004 | 3600 | 2014-08-28 21:00:00 | 16 | U/IU | Basal | Faster aspart |
| 20 | NN1218-3853 | 102004 | 3600 | 2014-08-29 09:20:00 | 6 | U/IU | Bolus | Faster aspart |
| 21 | NN1218-3853 | 102004 | 3600 | 2014-08-29 12:15:00 | 6 | U/IU | Bolus | Faster aspart |
| 22 | NN1218-3853 | 102004 | 3600 | 2014-08-29 20:15:00 | 28 | U/IU | Bolus | Faster aspart |
| 23 | NN1218-3853 | 102004 | 3600 | 2014-08-29 21:00:00 | 16 | U/IU | Basal | Faster aspart |

Fig. 6C

*

```
## DATA PREPROCESSING
## Implementation: AUTOMATED
def CGMRID_Insulin_preprocess(dfRAW):
    ## ONLY Basal
    dfPROC = dfRAW[dfRAW.Type == 'Basal']
    ## Drop Duplicates
    dfPROC = dfPROC.drop_duplicates()
    ## Sort values by Subject and Date Time
    dfPROC = dfPROC.sort_values(['Subject', 'DateTime'], ascending=[True, True])
    ## New Columns
    dfPROC = dfPROC.assign(insulinDose = dfPROC.Amount,
                           timestamp = pd.to_datetime(dfPROC.DateTime),
                           day = pd.to_datetime(dfPROC.DateTime, errors='coerce').dt.strftime('%Y-%m-%d'),
                           time = pd.to_datetime(dfPROC.DateTime, errors='coerce').dt.strftime('%H-%M-%S'))
    dfPROC = dfPROC.drop(dfPROC.columns[[3,4,5]], axis=1)
    dfPROC = dfPROC[['Trial', 'Subject', 'Visit', 'Treatment', 'Type', 'insulinDose', 'timestamp', 'day', 'time']]
    dfPROC


    return dfPROC
```

Fig. 6D

-

```
subjectIDs = dataInsulin.Subject.unique().tolist()
dfInsulin = CGMRID_Insulin_preprocess(dataInsulin)
dfInsulin
```

| | Trial | Subject | Visit | Treatment | Type | insulinDose | timestamp | day | time |
|---|---|---|---|---|---|---|---|---|---|
| 0 | NN1218-3853 | 102004 | 1000 | Faster aspart | Basal | 41 | 2014-02-27 21:17:00 | 2014-02-27 | 21-17-00 |
| 1 | NN1218-3853 | 102004 | 1000 | Faster aspart | Basal | 41 | 2014-02-28 22:00:00 | 2014-02-28 | 22-00-00 |
| 2 | NN1218-3853 | 102004 | 1000 | Faster aspart | Basal | 41 | 2014-03-01 21:00:00 | 2014-03-01 | 21-00-00 |
| 3 | NN1218-3853 | 102004 | 1000 | Faster aspart | Basal | 41 | 2014-03-02 21:08:00 | 2014-03-02 | 21-08-00 |
| 4 | NN1218-3853 | 102004 | 1000 | Faster aspart | Basal | 41 | 2014-03-03 21:00:00 | 2014-03-03 | 21-00-00 |
| 5 | NN1218-3853 | 102004 | 1000 | Faster aspart | Basal | 41 | 2014-03-04 21:00:00 | 2014-03-04 | 21-00-00 |
| 6 | NN1218-3853 | 102004 | 1000 | Faster aspart | Basal | 41 | 2014-03-05 20:33:00 | 2014-03-05 | 20-33-00 |
| 7 | NN1218-3853 | 102004 | 1000 | Faster aspart | Basal | 43 | 2014-03-06 21:15:00 | 2014-03-06 | 21-15-00 |
| 8 | NN1218-3853 | 102004 | 1000 | Faster aspart | Basal | 43 | 2014-03-07 21:15:00 | 2014-03-07 | 21-15-00 |
| 9 | NN1218-3853 | 102004 | 1000 | Faster aspart | Basal | 43 | 2014-03-08 21:35:00 | 2014-03-08 | 21-35-00 |
| 10 | NN1218-3853 | 102004 | 1000 | Faster aspart | Basal | 43 | 2014-03-09 21:15:00 | 2014-03-09 | 21-15-00 |
| 11 | NN1218-3853 | 102004 | 1000 | Faster aspart | Basal | 43 | 2014-03-10 21:05:00 | 2014-03-10 | 21-05-00 |
| 12 | NN1218-3853 | 102004 | 1000 | Faster aspart | Basal | 43 | 2014-03-12 21:15:00 | 2014-03-12 | 21-15-00 |
| 15 | NN1218-3853 | 102004 | 3600 | Faster aspart | Basal | 16 | 2014-08-27 21:00:00 | 2014-08-27 | 21-00-00 |
| 19 | NN1218-3853 | 102004 | 3600 | Faster aspart | Basal | 16 | 2014-08-28 21:00:00 | 2014-08-28 | 21-00-00 |
| 23 | NN1218-3853 | 102004 | 3600 | Faster aspart | Basal | 16 | 2014-08-29 21:00:00 | 2014-08-29 | 21-00-00 |
| 26 | NN1218-3853 | 102004 | 3600 | Faster aspart | Basal | 16 | 2014-08-30 21:08:00 | 2014-08-30 | 21-08-00 |
| 29 | NN1218-3853 | 102004 | 3600 | Faster aspart | Basal | 16 | 2014-08-31 21:00:00 | 2014-08-31 | 21-00-00 |
| 32 | NN1218-3853 | 102004 | 3600 | Faster aspart | Basal | 16 | 2014-09-01 20:40:00 | 2014-09-01 | 20-40-00 |
| 34 | NN1218-3853 | 102004 | 3600 | Faster aspart | Basal | 16 | 2014-09-02 20:55:00 | 2014-09-02 | 20-55-00 |
| 37 | NN1218-3853 | 102004 | 3600 | Faster aspart | Basal | 16 | 2014-09-03 20:45:00 | 2014-09-03 | 20-45-00 |
| 41 | NN1218-3853 | 102004 | 3600 | Faster aspart | Basal | 16 | 2014-09-04 20:55:00 | 2014-09-04 | 20-55-00 |
| 45 | NN1218-3853 | 102004 | 3600 | Faster aspart | Basal | 16 | 2014-09-05 21:15:00 | 2014-09-05 | 21-15-00 |

## Fig. 6E

```
dfInsulin.to_csv('NN1218_3853_INSULIN_PROC_20180329.csv', sep=',', index=False)
```

## Fig. 6F

*

```
## CGM Response Injected Dose (CGM_RID)
## Response Horizon (RH)
## GlUCOSE CONCENTRATION
csvCGM = 'CGM.csv'
#CGM_RID_RH60(csvPath)
```

Fig. 6G

```
## Importing libraries
import pandas as pd
import numpy as np
from sklearn.externals import joblib
```

Fig. 6H

*

-

```
## DATA INPUT
#from pandas import read_csv
dataCGM = pd.read_csv(csvCGM)
dataCGM
```

| | Trial | Subject | Visit | DateTime | Value | Unit | Type | Treatment |
|---|---|---|---|---|---|---|---|---|
| 0 | NN1218-3853 | 102004 | 1000 | 2014-02-27 13:48:26 | 8.712542 | mmol/L | SMPG | Faster aspart |
| 1 | NN1218-3853 | 102004 | 1000 | 2014-02-27 14:31:04 | 8.546060 | mmol/L | SMPG | Faster aspart |
| 2 | NN1218-3853 | 102004 | 1000 | 2014-02-27 14:33:47 | 9.100999 | mmol/L | CGM | Faster aspart |
| 3 | NN1218-3853 | 102004 | 1000 | 2014-02-27 14:38:47 | 9.156493 | mmol/L | CGM | Faster aspart |
| 4 | NN1218-3853 | 102004 | 1000 | 2014-02-27 14:43:47 | 8.990011 | mmol/L | CGM | Faster aspart |
| 5 | NN1218-3853 | 102004 | 1000 | 2014-02-27 14:48:47 | 9.211987 | mmol/L | CGM | Faster aspart |
| 6 | NN1218-3853 | 102004 | 1000 | 2014-02-27 14:53:47 | 9.267481 | mmol/L | CGM | Faster aspart |
| 7 | NN1218-3853 | 102004 | 1000 | 2014-02-27 14:58:47 | 9.211987 | mmol/L | CGM | Faster aspart |
| 8 | NN1218-3853 | 102004 | 1000 | 2014-02-27 15:03:47 | 9.156493 | mmol/L | CGM | Faster aspart |
| 9 | NN1218-3853 | 102004 | 1000 | 2014-02-27 15:08:47 | 9.211987 | mmol/L | CGM | Faster aspart |
| 10 | NN1218-3853 | 102004 | 1000 | 2014-02-27 15:13:47 | 9.267481 | mmol/L | CGM | Faster aspart |
| 11 | NN1218-3853 | 102004 | 1000 | 2014-02-27 15:18:47 | 9.100999 | mmol/L | CGM | Faster aspart |
| 12 | NN1218-3853 | 102004 | 1000 | 2014-02-27 15:23:47 | 8.879023 | mmol/L | CGM | Faster aspart |
| 13 | NN1218-3853 | 102004 | 1000 | 2014-02-27 15:28:47 | 8.657048 | mmol/L | CGM | Faster aspart |
| 14 | NN1218-3853 | 102004 | 1000 | 2014-02-27 15:33:47 | 8.102109 | mmol/L | CGM | Faster aspart |
| 15 | NN1218-3853 | 102004 | 1000 | 2014-02-27 15:38:47 | 8.046615 | mmol/L | CGM | Faster aspart |
| 16 | NN1218-3853 | 102004 | 1000 | 2014-02-27 15:43:46 | 7.880133 | mmol/L | CGM | Faster aspart |
| 17 | NN1218-3853 | 102004 | 1000 | 2014-02-27 15:48:46 | 7.547170 | mmol/L | CGM | Faster aspart |
| 18 | NN1218-3853 | 102004 | 1000 | 2014-02-27 15:53:46 | 7.824639 | mmol/L | CGM | Faster aspart |
| 19 | NN1218-3853 | 102004 | 1000 | 2014-02-27 15:58:46 | 8.102109 | mmol/L | CGM | Faster aspart |
| 20 | NN1218-3853 | 102004 | 1000 | 2014-02-27 16:03:46 | 8.102109 | mmol/L | CGM | Faster aspart |
| 21 | NN1218-3853 | 102004 | 1000 | 2014-02-27 16:08:46 | 7.991121 | mmol/L | CGM | Faster aspart |
| 22 | NN1218-3853 | 102004 | 1000 | 2014-02-27 16:13:46 | 7.824639 | mmol/L | CGM | Faster aspart |
| 23 | NN1218-3853 | 102004 | 1000 | 2014-02-27 16:18:46 | 7.658158 | mmol/L | CGM | Faster aspart |

Fig. 6I

*

```python
## DATA PREPROCESSING
## Implementation: AUTOMATED
def CGMRID_CGM_preprocess(dfRAW):
    dfRAW = cgmData
    ## One patient
    dfPROC = dfRAW[dfRAW.Type != 'SMPG']
    ## Drop Duplicates
    dfPROC = dfPROC.drop_duplicates()
    ## Sort values by Subject and Date Time
    #df.sort_values(['a', 'b'], ascending=[True, False])
    dfPROC = dfPROC.sort_values(['Subject', 'DateTime'], ascending=[True, True])
    ## New Columns
    dfPROC = dfPROC.assign(glucoseConc = dfPROC.Value * 18,
                           timestamp = pd.to_datetime(dfPROC.DateTime),
                           day = pd.to_datetime(dfPROC.DateTime, errors='coerce').dt.strftime('%Y-%m-%d'),
                           time = pd.to_datetime(dfPROC.DateTime, errors='coerce').dt.strftime('%H-%M-%S'))
    dfPROC = dfPROC.drop(dfPROC.columns[[3,4,5,6]], axis=1)
    dfPROC = dfPROC[['Trial', 'Subject', 'Visit', 'Treatment', 'glucoseConc', 'timestamp', 'day', 'time']]

    return dfPROC
```

Fig. 6J

-

```
subjectIDs = dataCGM.Subject.unique().tolist()
dfCGM = CGMRID_CGM_preprocess(dataCGM)
dfCGM
```

| | Trial | Subject | Visit | Treatment | glucoseConc | timestamp | day | time |
|---|---|---|---|---|---|---|---|---|
| 2 | NN1218-3853 | 102004 | 1000 | Faster aspart | 163.817980 | 2014-02-27 14:33:47 | 2014-02-27 | 14-33-47 |
| 3 | NN1218-3853 | 102004 | 1000 | Faster aspart | 164.816870 | 2014-02-27 14:38:47 | 2014-02-27 | 14-38-47 |
| 4 | NN1218-3853 | 102004 | 1000 | Faster aspart | 161.820200 | 2014-02-27 14:43:47 | 2014-02-27 | 14-43-47 |
| 5 | NN1218-3853 | 102004 | 1000 | Faster aspart | 165.815760 | 2014-02-27 14:48:47 | 2014-02-27 | 14-48-47 |
| 6 | NN1218-3853 | 102004 | 1000 | Faster aspart | 166.814650 | 2014-02-27 14:53:47 | 2014-02-27 | 14-53-47 |
| 7 | NN1218-3853 | 102004 | 1000 | Faster aspart | 165.815760 | 2014-02-27 14:58:47 | 2014-02-27 | 14-58-47 |
| 8 | NN1218-3853 | 102004 | 1000 | Faster aspart | 164.816870 | 2014-02-27 15:03:47 | 2014-02-27 | 15-03-47 |
| 9 | NN1218-3853 | 102004 | 1000 | Faster aspart | 165.815760 | 2014-02-27 15:08:47 | 2014-02-27 | 15-08-47 |
| 10 | NN1218-3853 | 102004 | 1000 | Faster aspart | 166.814650 | 2014-02-27 15:13:47 | 2014-02-27 | 15-13-47 |
| 11 | NN1218-3853 | 102004 | 1000 | Faster aspart | 163.817980 | 2014-02-27 15:18:47 | 2014-02-27 | 15-18-47 |
| 12 | NN1218-3853 | 102004 | 1000 | Faster aspart | 159.822420 | 2014-02-27 15:23:47 | 2014-02-27 | 15-23-47 |
| 13 | NN1218-3853 | 102004 | 1000 | Faster aspart | 155.826859 | 2014-02-27 15:28:47 | 2014-02-27 | 15-28-47 |
| 14 | NN1218-3853 | 102004 | 1000 | Faster aspart | 145.837958 | 2014-02-27 15:33:47 | 2014-02-27 | 15-33-47 |
| 15 | NN1218-3853 | 102004 | 1000 | Faster aspart | 144.839068 | 2014-02-27 15:38:47 | 2014-02-27 | 15-38-47 |
| 16 | NN1218-3853 | 102004 | 1000 | Faster aspart | 141.842397 | 2014-02-27 15:43:46 | 2014-02-27 | 15-43-46 |
| 17 | NN1218-3853 | 102004 | 1000 | Faster aspart | 135.849057 | 2014-02-27 15:48:46 | 2014-02-27 | 15-48-46 |
| 18 | NN1218-3853 | 102004 | 1000 | Faster aspart | 140.843507 | 2014-02-27 15:53:46 | 2014-02-27 | 15-53-46 |
| 19 | NN1218-3853 | 102004 | 1000 | Faster aspart | 145.837958 | 2014-02-27 15:58:46 | 2014-02-27 | 15-58-46 |
| 20 | NN1218-3853 | 102004 | 1000 | Faster aspart | 145.837958 | 2014-02-27 16:03:46 | 2014-02-27 | 16-03-46 |
| 21 | NN1218-3853 | 102004 | 1000 | Faster aspart | 143.840178 | 2014-02-27 16:08:46 | 2014-02-27 | 16-08-46 |
| 22 | NN1218-3853 | 102004 | 1000 | Faster aspart | 140.843507 | 2014-02-27 16:13:46 | 2014-02-27 | 16-13-46 |
| 23 | NN1218-3853 | 102004 | 1000 | Faster aspart | 137.846837 | 2014-02-27 16:18:46 | 2014-02-27 | 16-18-46 |
| 24 | NN1218-3853 | 102004 | 1000 | Faster aspart | 134.850166 | 2014-02-27 16:23:46 | 2014-02-27 | 16-23-46 |
| 25 | NN1218-3853 | 102004 | 1000 | Faster aspart | 131.853496 | 2014-02-27 16:28:46 | 2014-02-27 | 16-28-46 |
| 26 | NN1218-3853 | 102004 | 1000 | Faster aspart | 129.855716 | 2014-02-27 16:33:46 | 2014-02-27 | 16-33-46 |
| 27 | NN1218-3853 | 102004 | 1000 | Faster aspart | 133.851276 | 2014-02-27 16:38:46 | 2014-02-27 | 16-38-46 |

Fig. 6K

```
dfCGM.to_csv('NN1218_3853_CGM_PROC_20180329.csv', sep=',', index=False)
```

Fig. 6L

\*

```
## CGM Response Injected Dose (CGM_RID)
## Response Horizon (RH)
## BOTH TOGETHER
## GlUCOSE CONCENTRATION
## +
## INSULIN DOSE
csvCGM = 'NN1218_3853_CGM_PROC_20180329.csv'
csvInsulin = 'NN1218_3853_INSULIN_PROC_20180329.csv'
## Note: Insulin is BASAL only for now
```

Fig. 7A

```
## Importing libraries
import pandas as pd
import numpy as np
from sklearn.externals import joblib
```

Fig. 7B

```
## CGM DATA INPUT
dataCGM = pd.read_csv(csvCGM)
```

```
## INSULIN DATA INPUT
dataInsulin = pd.read_csv(csvInsulin)
```

Fig. 7C

*

```python
## Xs, independent varible
## Modular Function
from pandas.tseries.offsets import *
import pandas as pd

def RHIDP_processing_generate_Xs(dataCGM, dataInsulin):
    X = []
    Xs = []
    for subject in dataInsulin.Subject:

        dataCGM_subject = dataCGM[dataCGM.Subject==subject]
        dataInsulin_subject = dataInsulin[dataInsulin.Subject==subject]

        for doseTimestamp in dataInsulin_subject.timestamp:
            ## Response Horizon (RH) = 60 minutes, 1 hour
            dt0 = pd.to_datetime(doseTimestamp)
            dtRH60 = dt0 + DateOffset(hours=1)
            dataCGM_RH60 = dataCGM_subject[(dataCGM_subject.timestamp>=str(dt0)) & (dataCGM_subject.timestamp<=str(dtRH60))]
            if dataCGM_RH60.empty:
                continue
            else:
                ## Processing
                dataCGM_RH60_S = dataCGM_RH60.drop(dataCGM_RH60.columns[[0, 1, 2, 3, 6, 7]], axis=1)
                tsData = dataCGM_RH60_S.set_index('timestamp')
                tsData.index.name = None
                ## Convert the index to Datetime for resampling
                tsData.index = pd.to_datetime(tsData.index)
                ## Reindexing
                date_index2 = pd.date_range(str(dt0), str(dtRH60),freq='5T')
                dfReindexed = tsData.reindex(date_index2, method='nearest',tolerance='3min')
                ## Interpolation
                dfInterpolated = dfReindexed.interpolate(method='linear', axis=0).bfill()
                ## Differences or slopes between the interpolated data
                dfDiffs = dfInterpolated.diff()
                ## Feed these diffs or slope differences found in the interpolated data as the X, independent variable
                ## For one timestamp
                X = dfDiffs[1:].glucoseConc.tolist()
                ## For all timestamps
                Xs.append(X)
                X = []

    return Xs

Xs = RHIDP_processing_generate_Xs(dataCGM, dataInsulin)
```

Fig. 7D

```
print(Xs)
#len(Xs)
#32907
```

```
[[0.0, 0.0, 0.0, 0.0, 0.0, 0.0, 0.0, 10.987791335999958, -40.954495013999974, 10.987791353999967, -24.97225305599997, 0.0
], [0.0, 0.9988901279999709, 0.0, 8.990011098000053, 0.9988901099999907, -8.990011098000025, -6.9922308419999695, -5.9933
40732000007, -1.997780256000027, -1.9977802397999653, -1.9977802452000049, -0.9988901208000129], [0.0, -7.991120987999977
, -6.992230842000026, -5.99334073199995, 0.0, 0.0, -0.9988901280000277, 1.9977802380000185, 0.9988901279999993, 1.9977802
3799999, 0.9988901279999993, 0.9988901279999709], [15.982241954399996, 15.982241952600049, 10.987791343199945, 8.99001109
9800029, 9.9889012278, 10.987791335999987, 9.988901225999967, 5.993340732000007, 0.9988901100000191, 3.995560494000017, 0
.9988901279999709, 0.0], [0.0, 17.98002219600002, 0.0, 0.0, 0.0, 0.0, 0.0, 0.0, 0.0, 0.0, 0.0, 0.0], [0.0, -2.99667036600
0018, 0.0, 0.0, -0.9988901280000277, 0.0, -1.99778023799999, -3.9955604939999887, -4.994450604000008, 0.9988901099999907,
1.9977802559999702, 1.997780238000047], [0.0, 0.0, 0.0, 0.0, 0.9988901208000129, 3.99556048499997, 7.991120988000034, 9.9
88901207999987, 8.990011097999997, 9.988901226000024, 7.991120969999997, 0.0], [0.0, 13.984461720000013, 11.9866814639999
57, -0.9988901279999709, -3.995560494000017, 6.99223085999995, 8.990011098000082, 14.983351830000004, 17.980022195999993,
10.987791353999967, 0.0, -9.988901225999996], [0.0, -1.9977802559999418, -4.994450604000065, 0.9988901279999993, -0.99889
01279999993, 0.0, -3.9955604939999603, -1.99778023799999, 2.9966703659999894, 9.988901225999967, -0.9988901279999709, -4.
994450604000036], [0.0, -5.993340732000036, -5.993340749999987, -1.99778023799999, 0.0, 0.9988901279999709, 1.99778023799
999, 0.0, 5.993340732000007, 7.991120988000006, 6.992230842000026, 3.995560494000017], [0.0, 2.9966703659999894, 59.93340
7319999986, -11.986681463999957, 0.9988901280000277, -13.984461720000013, -10.987791336000015, -5.99334073199995, -2.9966
703660000746, 2.9966703660000746, 4.994450603999923, -5.99334073199995], [0.0, 4.994450609400019, -0.9988901207999845, -0
.9988901226000166, -3.4961154272999977, -3.4961154272999977, -4.994450611199994, -1.9977802434000012, 0.9988901225999882,
1.9977802434000012, -0.9988901225999882, 0.0], [0.0, 4.9944506093999905, 4.994450612999998, 6.9922308600000065, 7.9911209
```

Fig. 7E

```
#inputXs = 'NN1218_3853_RH60_Xs.csv'
#dataXs = pd.read_csv(inputXs)
from sklearn.externals import joblib
joblib.dump(Xs, 'NN1218_3853_RH60_Xs.pkl.xz')
## 70 KB
```

['NN1218_3853_RH60_Xs.pkl.xz']

Fig. 7F

```
## Ys, dependent varible
## Modular Function
from pandas.tseries.offsets import *
import pandas as pd

def RHIDP_processing_generate_Ys(dataCGM, dataInsulin):
    Y = []
    Ys = []
    ## For every subject in Insulin dataset
    for subject in dataInsulin.Subject:
        ## Align the current subject ID of the Insulin dataset to the same current subject ID of the CGM dataset

        ## Subset from the CGM dataset a smaller table of just the aligned and current subject ID of the Insulin dataset
        dataCGM_subject = dataCGM[dataCGM.Subject==subject]
        ## Subset from the Insulin dataset a smaller table of just the aligned and current subject ID of the Insulin dataset
        dataInsulin_subject = dataInsulin[dataInsulin.Subject==subject]

        for doseTimestamp in dataInsulin_subject.timestamp:
            ## Response Horizon (RH) = 60 minutes, 1 hour
            dt0 = pd.to_datetime(doseTimestamp)
            dtRH60 = dt0 + DateOffset(hours=1)
            dataCGM_RH60 = dataCGM_subject[(dataCGM_subject.timestamp>=str(dt0)) & (dataCGM_subject.timestamp<=str(dtRH60))]

            if dataCGM_RH60.empty:
                ## If there is a subject ID for the Insulin timestamp that has no corresponding CGM glucose profile
                continue
            else:
                dtX = dataInsulin_subject[dataInsulin_subject.timestamp == doseTimestamp]
                Y = dtX.insulinDose.tolist()
                Ys.extend(Y)

    return Ys


Ys = RHIDP_processing_generate_Ys(dataCGM, dataInsulin)
```

Fig. 7G

```
print(Ys)
#len(Ys)
#32907
```

```
[41, 41, 41, 41, 41, 41, 41, 43, 43, 43, 43, 43, 43, 16, 16, 16, 16, 16, 16, 16, 16, 16, 16, 16, 16, 16, 16, 41, 41, 41, 41,
41, 41, 41, 43, 43, 43, 43, 43, 43, 16, 16, 16, 16, 16, 16, 16, 16, 16, 16, 16, 16, 16, 41, 41, 41, 41, 41, 41, 41, 43, 4
3, 43, 43, 43, 43, 16, 16, 16, 16, 16, 16, 16, 16, 16, 16, 16, 16, 16, 41, 41, 41, 41, 41, 41, 41, 43, 43, 43, 43, 43, 43
, 16, 16, 16, 16, 16, 16, 16, 16, 16, 16, 16, 16, 16, 41, 41, 41, 41, 41, 41, 41, 43, 43, 43, 43, 43, 43, 16, 16, 16, 16,
16, 16, 16, 16, 16, 16, 16, 41, 41, 41, 41, 41, 41, 41, 43, 43, 43, 43, 43, 43, 16, 16, 16, 16, 16, 16, 16, 16, 1
6, 16, 16, 16, 16, 41, 41, 41, 41, 41, 41, 41, 43, 43, 43, 43, 43, 43, 16, 16, 16, 16, 16, 16, 16, 16, 16, 16, 16, 16, 16
, 41, 41, 41, 41, 41, 41, 41, 43, 43, 43, 43, 43, 43, 16, 16, 16, 16, 16, 16, 16, 16, 16, 16, 16, 16, 16, 41, 41, 41, 41,
41, 41, 41, 43, 43, 43, 43, 43, 43, 16, 16, 16, 16, 16, 16, 16, 16, 16, 16, 16, 16, 16, 41, 41, 41, 41, 41, 41, 41, 43, 4
```

Fig. 7H

```
from sklearn.externals import joblib
joblib.dump(Ys, 'NN1218_3853_RH60_Ys.pkl.xz')
## 70 KB
```

```
['NN1218_3853_RH60_Ys.pkl.xz']
```

Fig. 7I

EP 3 844 782 B1

# Train and Test datasets from Xs and Ys

```python
import pandas as pd
from sklearn.model_selection import train_test_split
# fix random seed for reproducibility
seed = 42
np.random.seed(seed)
```

```python
# Train = 70%
# Test =  30%
X_train, X_test, Y_train, Y_test = train_test_split(Xs, Ys, test_size=0.30, random_state=seed)
```

```python
from sklearn.externals import joblib
joblib.dump(X_train, 'NN1218_3853_RH60_X_train.pkl.xz')
## 287 KB
joblib.dump(X_test, 'NN1218_3853_RH60_X_test.pkl.xz')
## 132 KB
joblib.dump(Y_train, 'NN1218_3853_RH60_Y_train.pkl.xz')
## 20 KB
joblib.dump(Y_test, 'NN1218_3853_RH60_Y_test.pkl.xz')
## 10 KB
```

```
['NN1218_3853_RH60_Y_test.pkl.xz']
```

Fig. 7J

EP 3 844 782 B1

```
## Importing libraries
import pandas as pd
import numpy as np
from sklearn.externals import joblib
```

Fig. 8A

```
## Load the Train and Test datasets of Xs and Ys
X_train = joblib.load('NN1218_3853_RH60_X_train.pkl.xz')
X_test = joblib.load('NN1218_3853_RH60_X_test.pkl.xz')
Y_train = joblib.load('NN1218_3853_RH60_Y_train.pkl.xz')
Y_test = joblib.load('NN1218_3853_RH60_Y_test.pkl.xz')
```

Fig. 8B

*

-

```
## RF
from sklearn.ensemble import RandomForestRegressor
## Answer to life is 42, so the random seed would be set to 42
model = RandomForestRegressor(n_estimators=500, random_state=42).fit(X_train, Y_train)
```

Fig. 9A

```
from sklearn.metrics import r2_score
r2 = r2_score(Y_train, model.predict(X_train))
print("r^2: %.4f" % r2)
## Train "accuracy score" or r2: 0.9986
```

r^2: 0.9986

Fig. 9B

```
from sklearn.metrics import r2_score
r2 = r2_score(Y_test, model.predict(X_test))
print("r^2: %.4f" % r2)
## Test "accuracy score" or r2: 0.9990
```

r^2: 0.9990

Fig. 9C

*

-

```
from sklearn.externals import joblib
joblib.dump(model, 'NN1218_3853_RH60_ML_Regressor_RF.pkl.xz')
## 14.0 MB
```

```
['NN1218_3853_RH60_ML_Regressor_RF.pkl.xz']
```

```
from sklearn.externals import joblib
MLregressorModel = joblib.load('NN1218_3853_RH60_ML_Regressor_RF.pkl.xz')
```

Fig. 9D

```
from sklearn.metrics import mean_squared_error
mse = mean_squared_error(Y_test, model.predict(X_test))
print("RMSE: %.4f" % np.sqrt(mse))
```

RMSE: 1.1987

Fig. 9E

```
from sklearn.metrics import mean_absolute_error
mae = mean_absolute_error(Y_test, model.predict(X_test))
print("MAE: %.4f" % mae)
```

MAE: 0.0762

Fig. 9F

```
from sklearn.metrics import explained_variance_score
evs = explained_variance_score(Y_test, model.predict(X_test))
print("Explained Variance Score: %.4f" % evs)
```

Explained Variance Score: 0.9990

Fig. 9G

*

EP 3 844 782 B1

-

```
## KerasRegressor NN
## ~~~ TOP PERFORMANT NN Architecture ~~~
## 9-Hidden Layers
## Number of neurons:          24
## Number of Hidden Layers:     9
## Simplified NN Topology:     24 inputs-18-16-14-12-10-8-6-4-2-1 output
def RHIDP_KerasRegressor_model():
    # create model
    model = Sequential()
    ## INPUT layer
    model.add(Dense(24, input_dim=12, activation='relu'))
    ## HL 1
    model.add(Dense(18, activation='relu'))
    ## HL 2
    model.add(Dense(16, activation='relu'))
    ## HL 3
    model.add(Dense(14, activation='relu'))
    ## HL 4
    model.add(Dense(12, activation='relu'))
    ## HL 5
    model.add(Dense(10, activation='relu'))
    ## HL 6
    model.add(Dense(8, activation='relu'))
    ## HL 7
    model.add(Dense(6, activation='relu'))
    ## HL 8
    model.add(Dense(4, activation='relu'))
    ## HL 9
    model.add(Dense(2, activation='relu'))
    ## OUTPUT layer
    model.add(Dense(1))
    # Compile model
    model.compile(loss='mean_squared_error', optimizer='adam')
    return model
## Train r^2:     0.9420
## Test r^2:      0.9425
## RMSE:          8.9676
## MAE:           6.4363
## EVS:           0.9427
```

Fig. 10A

*

```
estimator = KerasRegressor(build_fn=RHIDP_KerasRegressor_model, nb_epoch=100, batch_size=100, verbose=False)

model = estimator.fit(X_train, Y_train)
```

Fig. 10B

-

```
from sklearn.metrics import r2_score
r2 = r2_score(Y_train, estimator.predict(X_train))
print("Train r^2: %.4f" % r2)
```

Train r^2: 0.9420

Fig. 10C

```
from sklearn.metrics import r2_score
r2 = r2_score(Y_test, estimator.predict(X_test))
print("Test r^2: %.4f" % r2)
```

Test r^2: 0.9425

Fig. 10D

```
from sklearn.metrics import mean_squared_error
mse = mean_squared_error(Y_test, estimator.predict(X_test))
print("RMSE: %.4f" % np.sqrt(mse))
```

RMSE: 8.9676

Fig. 10E

```
from sklearn.metrics import mean_absolute_error
mae = mean_absolute_error(Y_test, estimator.predict(X_test))
print("MAE: %.4f" % mae)
```

MAE: 6.4363

Fig. 10F

```
from sklearn.metrics import explained_variance_score
evs = explained_variance_score(Y_test, estimator.predict(X_test))
print("Explained Variance Score: %.4f" % evs)
```

Explained Variance Score: 0.9427

Fig. 10G

*

-

```
from sklearn.svm import SVR
#clf = SVR(C=1.0, epsilon=0.2)
#clf.fit(X, y)
model = SVR(C=1.0, epsilon=0.2).fit(X_train, Y_train)
```

Fig. 11A

```
from sklearn.metrics import r2_score
r2 = r2_score(Y_train, model.predict(X_train))
print("r^2: %.4f" % r2)
## Train "accuracy score" or r2: RANDOM FOREST: 0.9986
## Train "accuracy score" or r2: SVR:           0.4246
```

r^2: 0.4246

Fig. 11B

```
from sklearn.metrics import r2_score
r2 = r2_score(Y_test, model.predict(X_test))
print("r^2: %.4f" % r2)
## Test "accuracy score" or r2: RANDOM FOREST: 0.9990
## Test "accuracy score" or r2: SVR:           0.3958
```

r^2: 0.3958

Fig. 11C

*

-

```
from sklearn.metrics import mean_squared_error
mse = mean_squared_error(Y_test, model.predict(X_test))
print("RMSE: %.4f" % np.sqrt(mse))
```

RMSE: 29.0615

Fig. 11D

```
from sklearn.metrics import mean_absolute_error
mae = mean_absolute_error(Y_test, model.predict(X_test))
print("MAE: %.4f" % mae)
```

MAE: 13.8581

Fig. 11E

```
from sklearn.metrics import explained_variance_score
evs = explained_variance_score(Y_test, model.predict(X_test))
print("Explained Variance Score: %.4f" % evs)
```

Explained Variance Score: 0.4211

Fig. 11F

*

-

```
from sklearn import ensemble
#from sklearn.utils import shuffle
#from sklearn.metrics import mean_squared_error
## Setting 1
params = {'n_estimators': 500, 'max_depth': 4, 'min_samples_split': 2,
          'learning_rate': 0.01, 'loss': 'ls'}
## Setting 2
#params = {'n_estimators': 500, 'max_depth': 1, 'min_samples_split': 2,
#          'learning_rate': 0.5, 'loss': 'ls'}
model = ensemble.GradientBoostingRegressor(**params).fit(X_train, Y_train)
```

Fig. 12A

```
from sklearn.metrics import r2_score
r2 = r2_score(Y_train, model.predict(X_train))
print("r^2: %.4f" % r2)
## Train "accuracy score" or r2: RANDOM FOREST: 0.9986
## Train "accuracy score" or r2: SVR:           0.4246
## Train "accuracy score" or r2: GTB:           0.2675
```
r^2: 0.2675

Fig. 12B

```
from sklearn.metrics import r2_score
r2 = r2_score(Y_test, model.predict(X_test))
print("r^2: %.4f" % r2)
## Test "accuracy score" or r2: RANDOM FOREST: 0.9990
## Test "accuracy score" or r2: SVR:           0.3958
## Test "accuracy score" or r2: GTB:           0.2553
```
r^2: 0.2553

Fig. 12C

*

-

```
from sklearn.metrics import mean_squared_error
mse = mean_squared_error(Y_test, model.predict(X_test))
print("RMSE: %.4f" % np.sqrt(mse))
```

RMSE: 32.2644

Fig. 12D

```
from sklearn.metrics import mean_absolute_error
mae = mean_absolute_error(Y_test, model.predict(X_test))
print("MAE: %.4f" % mae)
```

MAE: 22.8542

Fig. 12E

```
from sklearn.metrics import explained_variance_score
evs = explained_variance_score(Y_test, model.predict(X_test))
print("Explained Variance Score: %.4f" % evs)
```

Explained Variance Score: 0.2554

Fig. 12F

*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017053101 A
- US 20190035500 A

**Non-patent literature cited in the description**

- **BERGENSTAL et al.** Can a Tool that Automates Insulin Titration be a Key to Diabetes Management?. *Diabetes Tech. and Thera,* 2012, vol. 14 (8), 675-682
- **HOLMAN et al.** 10-year follow-up of intensive glucose control in type 2 diabetes. *N. Engl. J. Med.,* 2008, vol. 359, 1577-1589
- **KHUNTI et al.** Self-titration of insulin in the management of people with type 2 diabetes: a practical solution to improve management in primary care. *Diabetes, Obes., and Metabol.,* 2012, vol. 15 (8), 690-700
- **NORRIS et al.** Self-management education for adults with type 2 diabetes: a meta-analysis on the effect of glycemic control. *Diabetes Care,* 2002, vol. 25, 1159-71
- **KULZER et al.** Effects of self-management training in type 2 diabetes: a randomized, prospective trial. *Diabet. Med.,* 2007, vol. 24, 415-23
- **ANDERSON et al.** Patient empowerment: results of a randomized controlled trial. *Diabetes Care,* 1995, vol. 18, 943-9
- **FRIEDRICHS et al.** Dosing Accuracy and Injection Force of Different Insulin Glargine Pens. *J Diabetes Sci Technol,* 2013, vol. 7 (5), 1346-1353

- **ABDEL-TWAB et al.** Dosing Accuracy of Two Disposable Insulin Pens According to New Iso 11680-1: 2012 Requirements. *J Diabetes Sci Technol.,* 2015, vol. 10 (1), 157-161
- **DASKALAKI et al.** Real-Time Adaptive Models for the personalized prediction of Glycemic Profile in Type 1 Diabetes Patients. *Diabetes Technol. Thera.,* 2012, vol. 14 (2), 168-174
- **PAPPADA et al.** Neural Network-Based Real-Time Prediction of Glucose in Patients with Insulin-Dependence Diabetes. *Diabetes Technol. Thera.,* 2011, vol. 13 (2), 135-141
- **SCHMID.** New options in insulin therapy. *J Pediatria (Rio J),* 2007, vol. 83 (5), S146-S155
- **PLANK et al.** A double-blind, randomized, dose-response study investigating the pharmacodynamic and pharmacokinetic properties of the long-acting insulin analog detemir. *Diabetes Care,* 2005, vol. 28, 1107-1112
- **DUNN et al.** An Updated Review of its Use in the Management of Diabetes Mellitus. *Drugs,* 2003, vol. 63, 1743